# EUROPEAN PATENT APPLICATION

(11) **EP 4 818 191 A1**
(43) Date of publication of application: **07.10.2026**
(21) Application number: 24897725.8
(22) Date of filing: 27.11.2024
(51) Int. Cl.: C07K 7/08, A61K 38/10, A61K 47/54, A61K 47/62, A61K 49/00, A61K 51/08, A61P 35/00, A61P 43/00, C07K 1/107, C07K 7/52, C07K 7/54, C12Q 1/02, C12Q 1/527

(54) **PEPTIDE AND CONJUGATE CONTAINING SAID PEPTIDE**

(30) Priority: 27.11.2023 JP 2023200246; 04.04.2024 JP 2024060915
(71) Applicant: PeptiDream Inc., Kawasaki-Shi, Kanagawa 210-0821 (JP)
(72) Inventor: AOKI, Takanori, Kawasaki-shi, Kanagawa 210-0821 (JP); INABA, Hiroko, Kawasaki-shi, Kanagawa 210-0821 (JP); KITAMURA, Hidetomo, Kawasaki-shi, Kanagawa 210-0821 (JP); KOTAKE, Tatsuro, Kawasaki-shi, Kanagawa 210-0821 (JP); SHIMIZU, Hikaru, Kawasaki-shi, Kanagawa 210-0821 (JP); TSUCHIDA, Shota, Kawasaki-shi, Kanagawa 210-0821 (JP); MIZUKOSHI, Yoshihide, Kawasaki-shi, Kanagawa 210-0821 (JP); MURAKAMI, Masato, Kawasaki-shi, Kanagawa 210-0821 (JP); YANAGIDA, Hayato, Kawasaki-shi, Kanagawa 210-0821 (JP); TAKUWA, Masatoshi, Kawasaki-shi, Kanagawa 210-0821 (JP)
(74) Representative: Mewburn Ellis LLP
(86) International application number: PCT/JP2024/080211
(87) International publication number: WO 2025/116046

(57) **Abstract**

The present invention relates to a peptide, a conjugate containing said peptide, and a composition containing the peptide and/or the conjugate, etc. The peptide of the present invention comprises the following amino acid sequence: da-Tbg-3Py6CON-Hgl-Meda-Ahp-PeG-S-3Py6NH2-HseMe-dk-Y or an amino acid sequence having a substitution, addition, deletion, or insertion in 1 to 10 amino acid residues selected from the group consisting of amino acid residues at positions 1, 2, 3, 4, 5, 7, 8, 9, 10, and 11 in the above amino acid sequence.

## Description

### TECHNICAL FIELD

The present invention relates to a peptide, a conjugate containing said peptide and a payload (also sometimes called a "peptide conjugate", a "peptide complex", or simply a "conjugate"), and use of the peptide or conjugate.

### BACKGROUND ART

CA9 (Carbonic anhydrase IX, carbonic anhydrase-9) is one of the 15 carbonic anhydrases and is a homodimeric transmembrane protein localized on the cell membrane (NPL 1). Here, CA9 has three domains: a catalytic domain, a proteoglycan-like domain, and a transmembrane domain and a cytoplasmic tail. As one of its functions, the catalytic domain has zinc-requiring activity to convert water molecules and carbon dioxide into hydrogen and bicarbonate ions, so that the intracellular and extracellular pH can be adjusted to conditions suitable for cell growth (NPL 2). In fact, it has been confirmed *in vitro* and in studies using tumor-bearing mice that tumor cell growth is slower in tumor cells in which CA9 is knocked out than in the wild type, proving experimentally that CA9 is involved in tumor cell growth (NPL 3). In patients with various tumors, CA9 expression levels are found to be significantly associated with overall survival, disease-free survival, local control, disease-specific survival, metastasis-free survival, and progression-free survival. This suggests that patients with tumors that express high levels of CA9 have a high risk of local recurrence, disease progression, and metastasis, regardless of tumor type or location (NPL 4). Based on these reports, CA9 is expected to be a promising target for the detection of tumors with poor prognosis.

The activation of the hypoxia-inducible factor (HIF) 1α pathway has been reported as the mechanism by which CA9 expression is induced (NPL 5), and it has been reported that the number of patients with high CA9 expression levels is particularly high in clear cell renal cell carcinoma and colon cancer (NPL 6) (NPL 7). In colon cancer, CA9 is highly expressed in tumor tissues of about 70% of patients due to activation of the HIF1α pathway associated with hypoxia (NPL 7). On the other hand, in patients with clear cell renal cell carcinoma, more than half of them have somatic mutations in the VHL (Von Hippel-Lindau) gene, a tumor suppressor gene, or excessive methylation of the promoter region (NPL 8), and the HIF1α pathway is chronically activated even under normal oxygen conditions (NPL 5), and CA9 is reported to be highly expressed in tumor tissues of about 90% of patients (NPL 6). Metastatic colon cancer and metastatic renal cell carcinoma have a very poor prognosis with a 5-year survival rate of 20% or less, so there is an urgent need to establish diagnostic and therapeutic methods for these diseases.

Until now, cancer therapeutic agents targeting CA9 have been developed using antibodies such as Girentuximab, but they have failed to show efficacy (NPL 9). For this reason, antibody-RI complexes have been developed in which Girentuximab is conjugated with a chelator and attached to a nuclide for PET and therapy. PET imaging with an antibody-RI complex can detect tumors with high sensitivity. However, depending on the length of half-life of the antibody in blood, it is necessary to use a high-energy nuclide, so that it takes time to detect tumors, as tumors become detectable 5 days after administration (NPL 10). Furthermore, in therapeutic use, the blood half-life is similarly prolonged; therefore, thrombocytopenia induced by the RI persists for an extended period. As a result, repeated administration is difficult, and in fact, in the Phase II clinical trial, thrombocytopenia did not recover, making it impossible to administer three repeated doses (NPL 11).

Accordingly, there remains a need for the development of novel anticancer therapeutic agents and diagnostic agents that target CA9 and contain, as an active ingredient, a compound other than an antibody, for example, an imaging agent for PET.

### CITATION LIST

### NON PATENT LITERATURE

NPL 1: Mayank Aggarwal, et al. J. Enzyme Inhib. Med. Chem. 2013 Apr; 28(2): 267-77.
NPL 2: Pawel Swietach, et al. J. Biol Chem. 2009 Jul 24; 284(30): 20299-310.
NPL 3: Scott K Parks, et al. Oncotarget 2017 Feb 7; 8(6): 10225-10237
NPL 4: Simon J A van Kuijk, et al. Front Oncol. 2016 Mar 29: 6: 69.
NPL 5: C C Wykoff, N J Beasley et al. Cancer Res. 2000 Dec 15; 60(24): 7075-83.
NPL 6: Matthew H T Bui, et al. Clin. Cancer Res. 2003 Feb; 9(2): 802-11.
NPL 7: Emese Sarolta Bádon, et al. Int. J. Mol. Sci. 2023 Jan 30; 24(3): 2581.
NPL 8: Yusuke Sato, et al. Nat. Genet. 2013 Aug; 45(8): 860-7.
NPL 9: Karim Chamie, et al. JAMA Oncol. 2017 Jul 1; 3(7): 913-920.
NPL 10: Brian M. Shuch, et al. Results from phase 3 study of 89Zr-DFO-girentuximab for PET/CT imaging of clear cell renal cell carcinoma (ZIRCON). 2023 ASCO Genitourinary Cancers Symposium Meeting Abstract
NPL 11: Constantijn H J Muselaers, et al. Eur. Urol. 2016 May; 69(5): 767-70.

### SUMMARY OF INVENTION

### TECHNICAL PROBLEM

The present inventors have conducted intensive research to create a compound with CA9 binding activity, and as a result, have conceived the present invention. The present inventors have also found a conjugate of a compound having CA9 binding activity and a payload such as a radioactive or fluorescent substance. The present invention relates to a novel peptide having CA9 binding activity, a conjugate of the peptide with a payload, a composition containing the peptide or conjugate (e.g., a pharmaceutical composition, diagnostic composition, research composition), and use of the peptide or conjugate.

### SOLUTION TO PROBLEM

The present application includes, but not limited thereto, the following items of the invention.
[1] A peptide comprising the following amino acid sequence:
   da-Tbg-3Py6CON-Hgl-Meda-Ahp-PeG-S-3Py6NH2-HseMe-dk-Y (SEQ ID No. 1) or
   an amino acid sequence having a substitution, addition, deletion, or insertion in 1 to 10 amino acid residues selected from the group consisting of amino acid residues at positions 1, 2, 3, 4, 5, 7, 8, 9, 10, and 11 in the above amino acid sequence.
[2] The peptide according to [1], wherein (1) MeC, C, or MeCt is attached, as an amino acid residue at position 13, to the amino acid sequence set forth in SEQ ID No. 1 or
   (2) MeA, MeS, MeN, Hpr, G, or P is attached, as an amino acid residue at position 13, to the amino acid sequence of SEQ ID No. 1 and MeG, G, da, or Meda is further attached as an amino acid residue at position 14.
[3] The peptide according to [1] or [2], comprising an amino acid sequence having a substitution, addition, deletion, or insertion in 1 to 7 amino acid residues selected from the group consisting of amino acid residues at positions 1, 4, 7, 8, 9, 10, and 11 in the amino acid sequence set forth in SEQ ID No. 1.
[4] The peptide according to any one of [1] to [3], wherein the following one or two or more requirements are satisfied:
   the amino acid residue at position 1 is da, dq, de, dhgl, or dk;
   the amino acid residue at position 2 is I or Tbg;
   the amino acid residue at position 3 is 3Py6NH2 or 3Py6CON;
   the amino acid residue at position 4 is S, Hgl, Hgl(PEG8Me), alT, Hgn, SMe, KCOpipzaa, or DabAc;
   the amino acid residue at position 5 is PeG, MeG, Meda, or MedkCOpipzaa;
   the amino acid residue at position 7 is PeG, pMeOPeG, MsMeapG, 3OMePeG, pHPeG, PpG, pFPeG, mCPeG, pCPeG, 4HPpG, 4OMePpG, 4PypG, 4COOPeG, 3COOPeG, or 3FPeG;
   the amino acid residue at position 8 is H, S, P, D, KCOpipzaa, Q(PEG8Me), P4Sh, Hse, SMe, Qmm, or Qdm;
   the amino acid residue at position 9 is 3Py6NH2, 5Inda, Y or F4OMe;
   the amino acid residue at position 10 is Ahp, HseMe, or E; and
   the amino acid residue at position 11 is dk, G, da, dq, dkCOpipzaa, dk(t4amCh), de, Acb, or dkAc.
[5] The peptide according to any one of [1] to [4], wherein the following one or two or more requirements are satisfied:
   the amino acid residue at position 2 is Tbg;
   the amino acid residue at position 3 is 3Py6NH2;
   the amino acid residue at position 5 is Meda;
   the amino acid residue at position 7 is PeG or pHPeg;
   the amino acid residue at position 9 is 3Py6NH2 or 5Inda; and
   the amino acid residue at position 10 is HseMe.
[6] The peptide according to any one of [1] to [4], wherein
   the amino acid residue at position 2 is Tbg;
   the amino acid residue at position 3 is 3Py6NH2;
   the amino acid residue at position 5 is Meda;
   the amino acid residue at position 7 is PeG or pHPeg;
   the amino acid residue at position 9 is 3Py6NH2 or 5Inda; and
   the amino acid residue at position 10 is HseMe.
[7] The peptide according to any one of [1] to [6], wherein the peptide comprises or consists of an amino acid sequence set forth in any one of SEQ ID Nos. 2-54.
[8] The peptide according to any one of [1] to [7], further comprising an additional amino acid residue.
[9] The peptide according to any one of [1] to [8], wherein the peptide is a cyclic peptide.
[10] The peptide according to any one of [1] to [9], which has a cyclic structure in which a chloroacetylated amino acid residue at position 1 in the amino acid sequence set forth in SEQ ID No. 1 is bonded to a cysteine residue included in the peptide.
[11] The peptide according to any one of [1] to [9], which has a cyclic structure in which an amino group of an amino acid residue at position 1 in the amino acid sequence set forth in SEQ ID No. 1 included in the peptide is bonded to a carboxy group of an amino acid residue at position 14.
[12] The peptide according to any one of [1] to [11], wherein
   (a) a linker and/or a payload can bind to a C-terminus of the peptide; or
   (b) the amino acid residue at position 1, 4, 8, 11, or 13 in the amino acid sequence set forth in SEQ ID No. 1 is an amino acid residue to which a linker and/or a payload can bind.
[13] A conjugate comprising the peptide according to any one of [1] to [12] and a payload, wherein
   (i) the amino acid residue at position 13 in SEQ ID No. 1 is attached, with or without a linker, to any payload; or
   (ii) the amino acid residue at position 1, 4, 8, or 11 in SEQ ID No. 1 is attached to a payload as shown in the following formula (I): 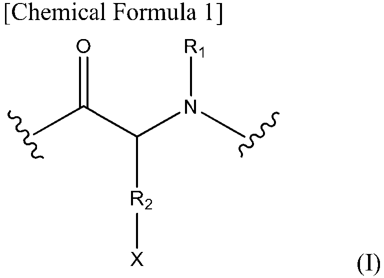
      wherein in the formula (I),
      R₁ is H or a C₁₋₃ alkyl group;
      R₂ is C₁₋₆ alkyl-NH-, C₁₋₆ alkyl-C₆ aryl-O-C₁₋₃ alkyl-NH-, C₁₋₆ alkyl-NH(=O)-CH(C₁₋₆ alkylphenyl)-NH-, C₁₋₆ alkyl-NH(=O)-CH(C₁₋₆ alkyl)-NH-, or C₁₋₆ alkyl-NH(=O)-C₃₋₈ cycloalkyl-C₁₋₃ alkyl-NH-; and
      X is any payload.
[14] The conjugate according to [13], wherein R₂ is C₁₋₆ alkyl-NH-.
[15] The conjugate according to [13] or [14], wherein X is a chelator.
[16] The conjugate according to [15], wherein the chelator is attached to a radioactive substance.
[17] The conjugate according to [15] or [16], wherein the chelator is DOTA, DOTAGA, or NODAGA.
[18] A pharmaceutical composition comprising the peptide according to any one of [1] to [12].
[19] A pharmaceutical composition for preventing or treating a CA9-associated disease, comprising the peptide according to any one of [1] to [12].
[20] A pharmaceutical composition comprising the conjugate according to any one of [13] to [17].
[21] A pharmaceutical composition for preventing or treating a CA9-associated disease, comprising the conjugate according to any one of [13] to [17].
[22] A diagnostic or research composition comprising the peptide according to any one of [1] to [12].
[23] A diagnostic or research composition for diagnosing a CA9-associated disease, comprising the peptide according to any one of [1] to [12].
[24] A diagnostic or research composition comprising the conjugate according to any one of [13] to [17].
[25] A diagnostic or research composition for diagnosing a CA9-associated disease, comprising the conjugate according to any one of [13] to [17].
[26] An imaging agent comprising the conjugate according to any one of [13] to [17].
[27] An imaging agent comprising the conjugate according to any one of [13] to [17], wherein the imaging agent is used for diagnosis of a tumor.
[28] A radioligand imaging agent used for positron emission tomography, comprising the conjugate according to any one of [13] to [17].
[29] A peptide testing method comprising testing a peptide in terms of at least one of:
   a) solubility in a solvent;
   b) CA9 binding activity;
   c) cell and/or tissue toxicity; or
   d) toxicity to an experimental animal, wherein
   the peptide is the peptide according to any one of [1] to [12].
[30] A conjugate testing method comprising testing a conjugate in terms of at least one of:
   a) solubility in a solvent;
   b) CA9 binding activity;
   c) cell and/or tissue toxicity; or
   d) toxicity to an experimental animal, wherein
   the conjugate is the conjugate according to any one of [13] to [17].
[31] The peptide according to any one of [1] to [3], wherein the following one or two or more requirements are satisfied:
   the amino acid residue at position 1 is de(PEG8Me), dk, dyae, or dkCOpipzaa;
   the amino acid residue at position 2 is I or Tbg;
   the amino acid residue at position 3 is 3Py6NH2 or 3Py6CON;
   the amino acid residue at position 4 is K, KCOpipzaa, Hgn(Qglucamine-NH2), Hgn(KCOpipzaa-NH2), or Qglucamine;
   the amino acid residue at position 5 is PeG, MeG, Meda, or MedkCOpipzaa;
   the amino acid residue at position 7 is PeG, pMeOPeG, MsMeapG, 3OMePeG, pHPeG, PpG, pFPeG, mCPeG, pCPeG, 4HPpG, 4OMePpG, 4PypG, 4COOPeG, 3COOPeG, or 3FPeG;
   the amino acid residue at position 8 is K;
   the amino acid residue at position 9 is 3Py6NH2, 5Inda, Y, or F4OMe;
   the amino acid residue at position 10 is Ahp, HseMe, or E; and
   the amino acid residue at position 11 is dk(F) or dk(PEG8c).
[32] The peptide according to any one of [1] to [6], wherein the peptide comprises or consists of an amino acid sequence set forth in any one of SEQ ID Nos. 55-103.
[33] A conjugate comprising the peptide according to [32] and a payload, wherein any payload is attached, with or without a linker, to the amino acid residue at position 1, 4, 8, 11, or 13.
[34] A conjugate described in any one of Conjugate Nos. 1-81 listed in Table 8 or Table 18.

### ADVANTAGEOUS EFFECTS OF INVENTION

The peptide or conjugate of the present invention has CA9 binding activity, and is thus applicable as a pharmaceutical composition for preventing or treating a CA9-associated symptom, a diagnostic composition, and a research composition, etc.

### BRIEF DESCRIPTION OF DRAWINGS

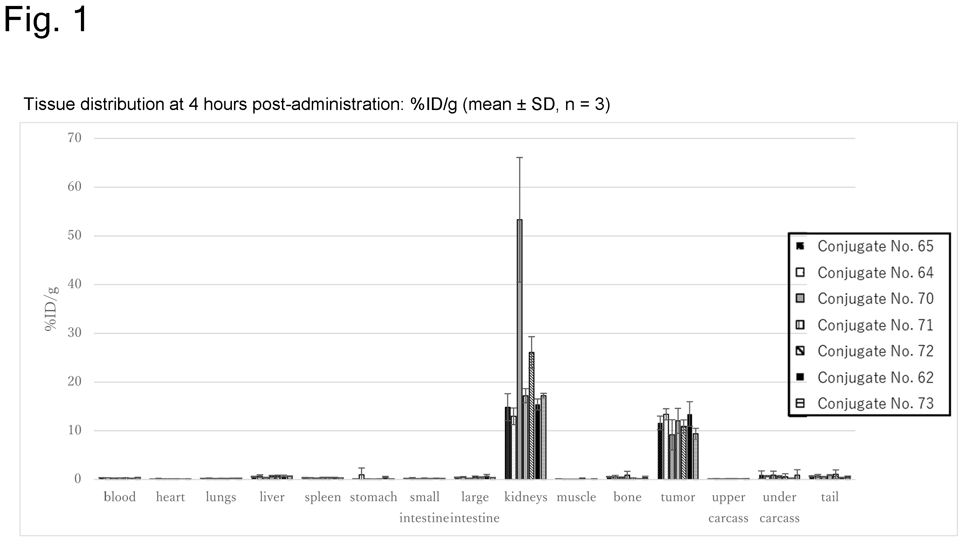

[Fig. 1] Fig. 1 is a graph showing the results of *in vivo* distribution of each conjugate comprising each peptide of the present invention with DOTA-⁶⁷Ga as a payload, at 4 hours after the administration, in a female BALB/cSlc-nu/nu tumor implantation model mouse. Note that the horizontal axis indicates each tissue, and the vertical axis indicates the radioactivity concentration (%ID/g) in each tissue.

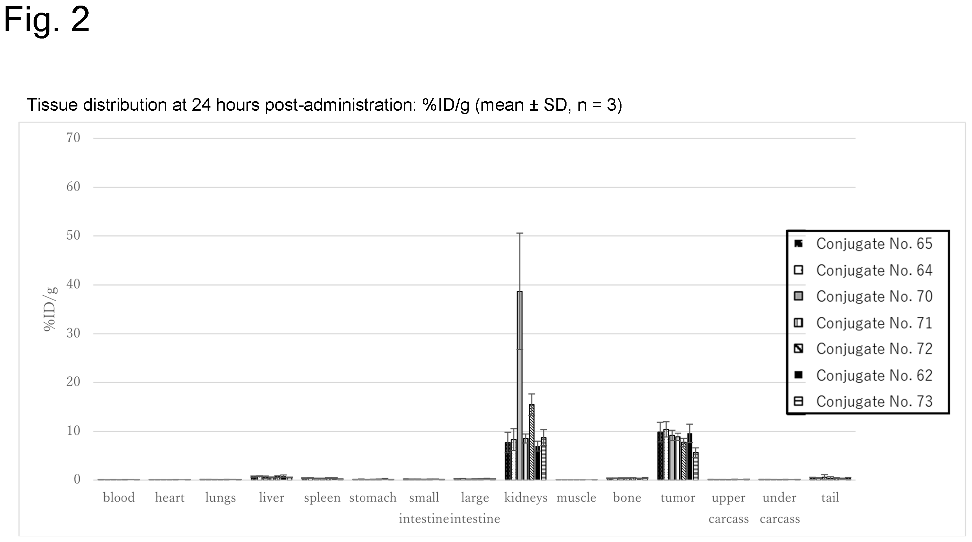

[Fig. 2] Fig. 2 is a graph showing the results of *in vivo* distribution of each conjugate comprising each peptide of the present invention with DOTA-⁶⁷Ga as a payload, at 24 hours after the administration, in a female BALB/cSlc-nu/nu tumor implantation model mouse. Note that the horizontal axis indicates each tissue, and the vertical axis indicates the radioactivity concentration (%ID/g) in each tissue.

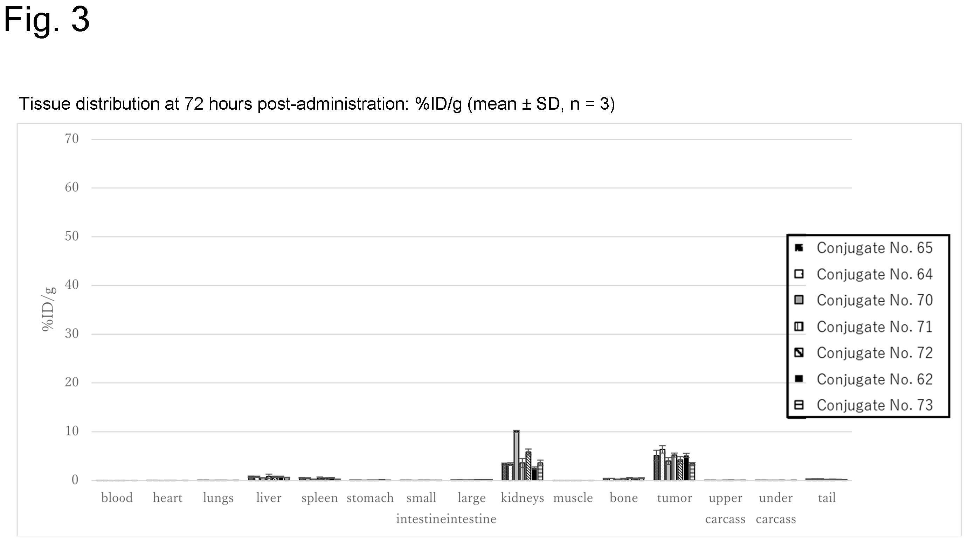

[Fig. 3] Fig. 3 is a graph showing the results of *in vivo* distribution of each conjugate comprising each peptide of the present invention with DOTA-⁶⁷Ga as a payload, at 72 hours after the administration, in a female BALB/cSlc-nu/nu tumor implantation model mouse. Note that the horizontal axis indicates each tissue, and the vertical axis indicates the radioactivity concentration (%ID/g) in each tissue.

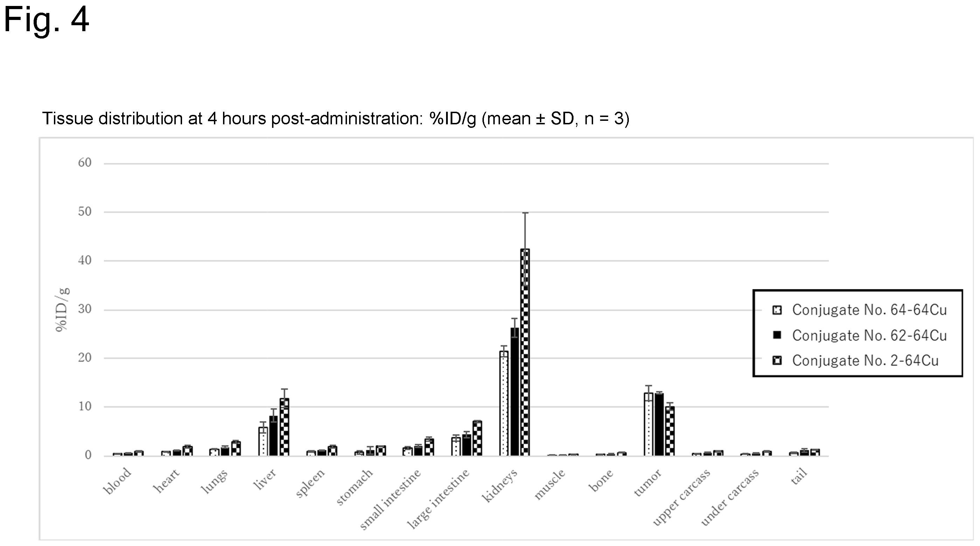

[Fig. 4] Fig. 4 is a graph showing the results of *in vivo* distribution of each conjugate comprising each peptide of the present invention with DOTA-⁶⁴Cu as a payload, at 4 hours after the administration, in a female BALB/cSlc-nu/nu tumor implantation model mouse. Note that the horizontal axis indicates each tissue, and the vertical axis indicates the radioactivity concentration (%ID/g) in each tissue.

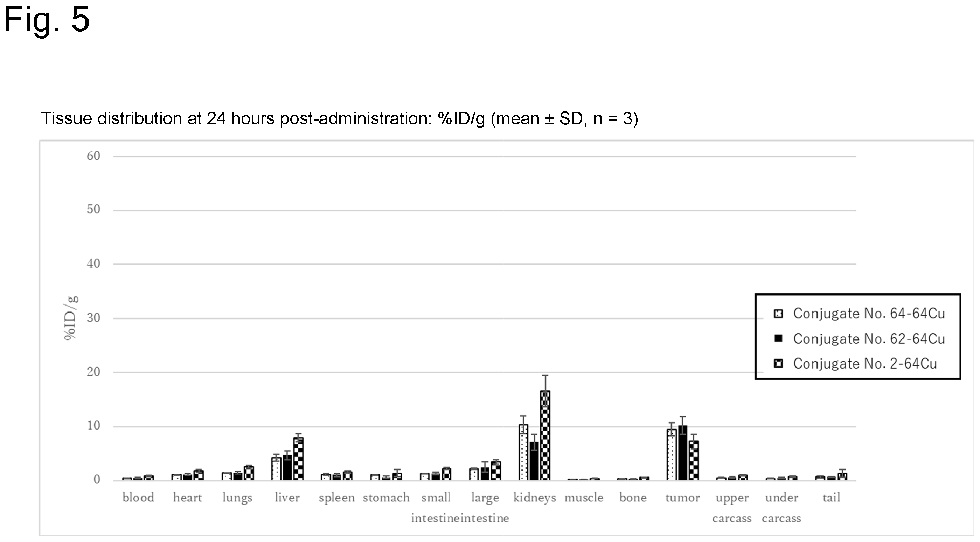

[Fig. 5] Fig. 5 is a graph showing the results of *in vivo* distribution of each conjugate comprising each peptide of the present invention with DOTA-⁶⁴Cu as a payload, at 24 hours after the administration, in a female BALB/cSlc-nu/nu tumor implantation model mouse. Note that the horizontal axis indicates each tissue, and the vertical axis indicates the radioactivity concentration (%ID/g) in each tissue.

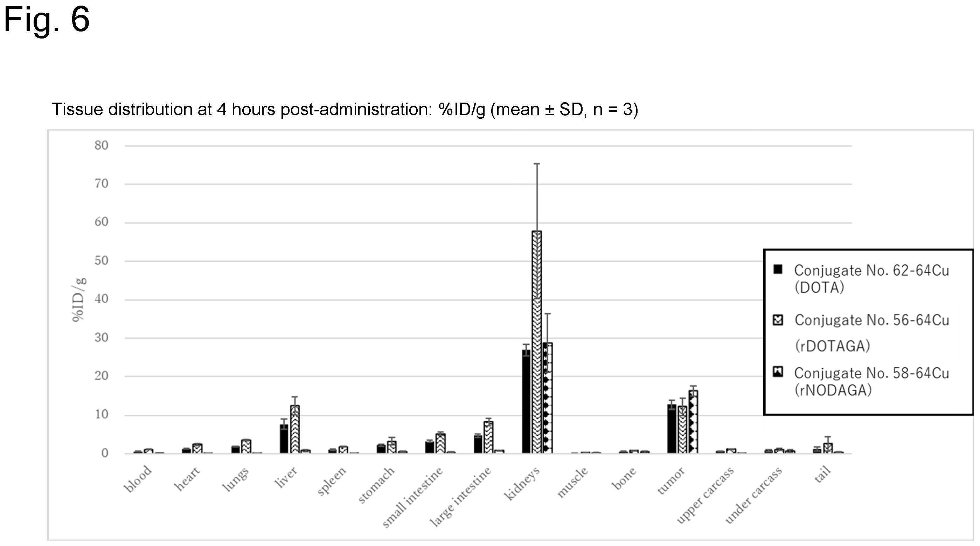

[Fig. 6] Fig. 6 is a graph showing the results of *in vivo* distribution of each conjugate comprising each peptide of the present invention with DOTA-⁶⁴Cu, DOTAGA-⁶⁴Cu, or NODAGA-⁶⁴Cu as a payload, at 4 hours after the administration, in a female BALB/cSlc-nu/nu tumor implantation model mouse. Note that the horizontal axis indicates each tissue, and the vertical axis indicates the radioactivity concentration (%ID/g) in each tissue.

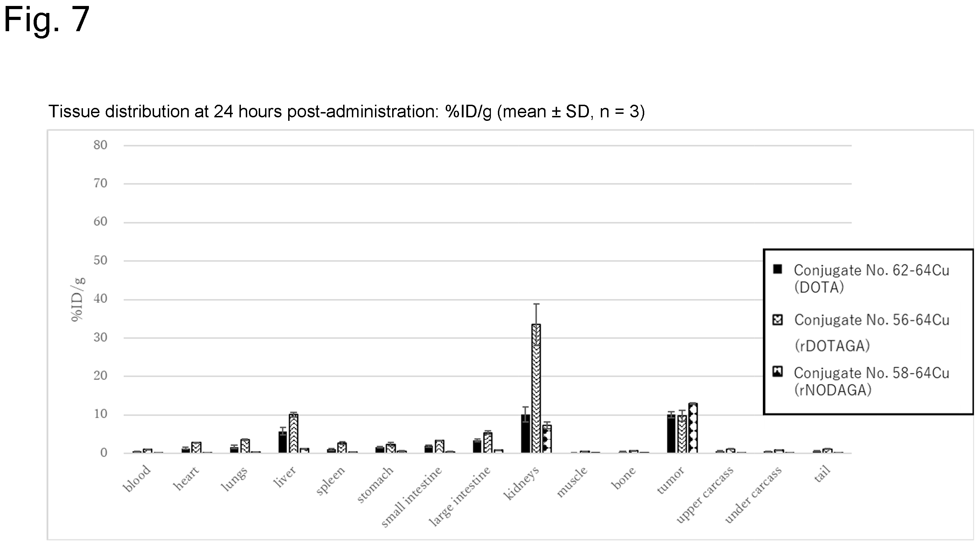

[Fig. 7] Fig. 7 is a graph showing the results of *in vivo* distribution of each conjugate comprising each peptide of the present invention with DOTA-⁶⁴Cu, DOTAGA-⁶⁴Cu, or NODAGA-⁶⁴Cu as a payload, at 24 hours after the administration, in a female BALB/cSlc-nu/nu tumor implantation model mouse. Note that the horizontal axis indicates each tissue, and the vertical axis indicates the radioactivity concentration (%ID/g) in each tissue.

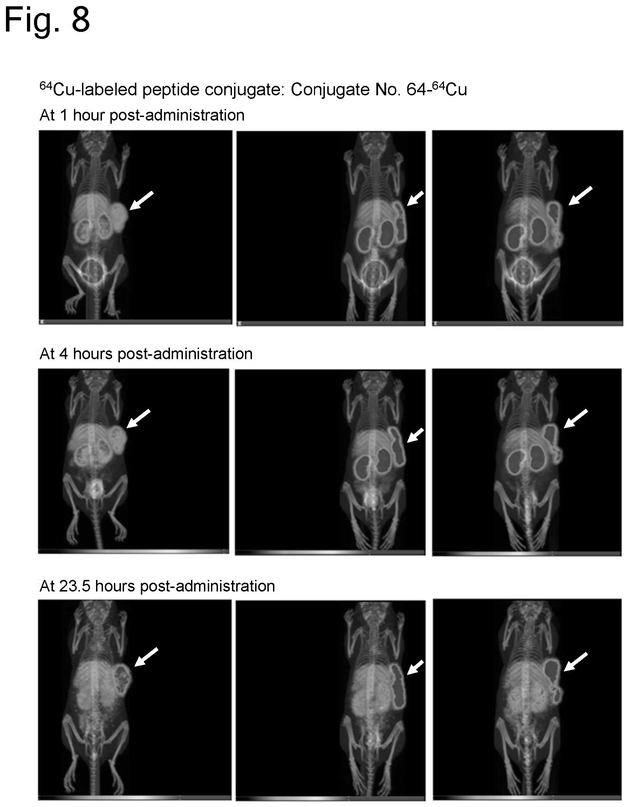

[Fig. 8] Fig. 8 is representative PET/CT images of a female BALB/cSlc-nu/nu tumor implantation model mouse at 1, 4, and 23.5 hours after the administration of Conjugate No. 64-⁶⁴Cu, namely a conjugate comprising a peptide of the present invention with DOTA-⁶⁴Cu as a payload. White arrows in the figure indicate the localization of the implanted tumor.

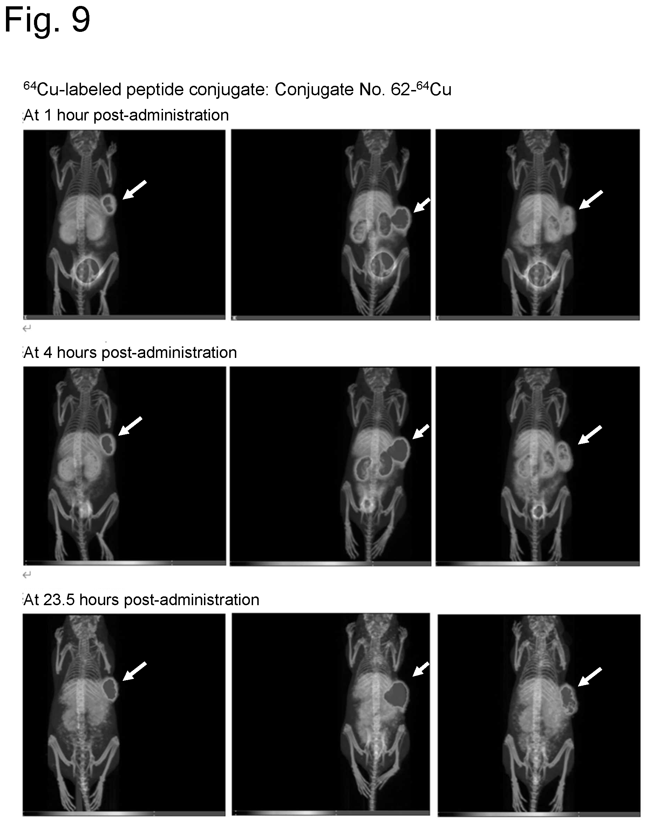

[Fig. 9] Fig. 9 is representative PET/CT images of a female BALB/cSlc-nu/nu tumor implantation model mouse at 1, 4, and 23.5 hours after the administration of Conjugate No. 62-⁶⁴Cu, namely a conjugate comprising a peptide of the present invention with DOTA-⁶⁴Cu as a payload. White arrows in the figure indicate the localization of the implanted tumor.

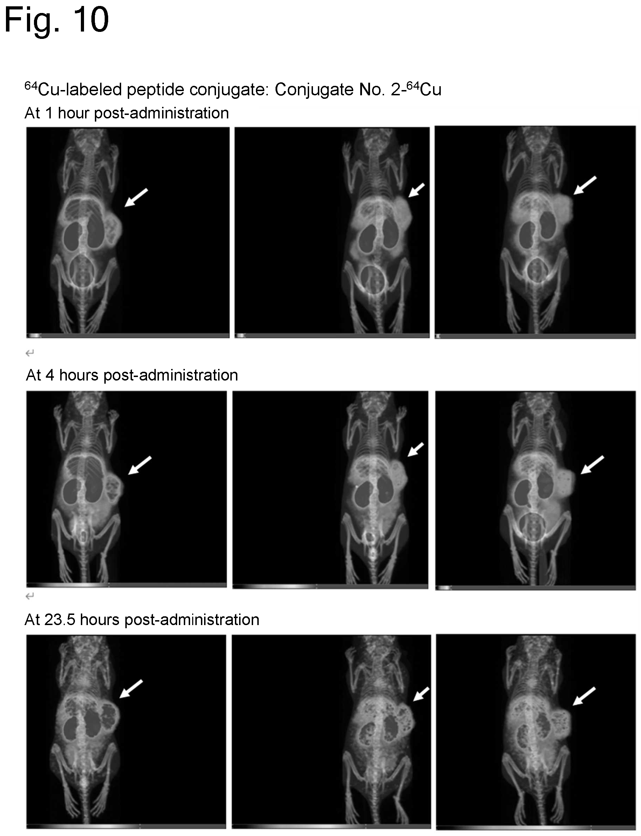

[Fig. 10] Fig. 10 is representative PET/CT images of a female BALB/cSlc-nu/nu tumor implantation model mouse at 1, 4, and 23.5 hours after the administration of Conjugate No. 2-⁶⁴Cu, namely a conjugate comprising a peptide of the present invention with DOTA-⁶⁴Cu as a payload. White arrows in the figure indicate the localization of the implanted tumor.

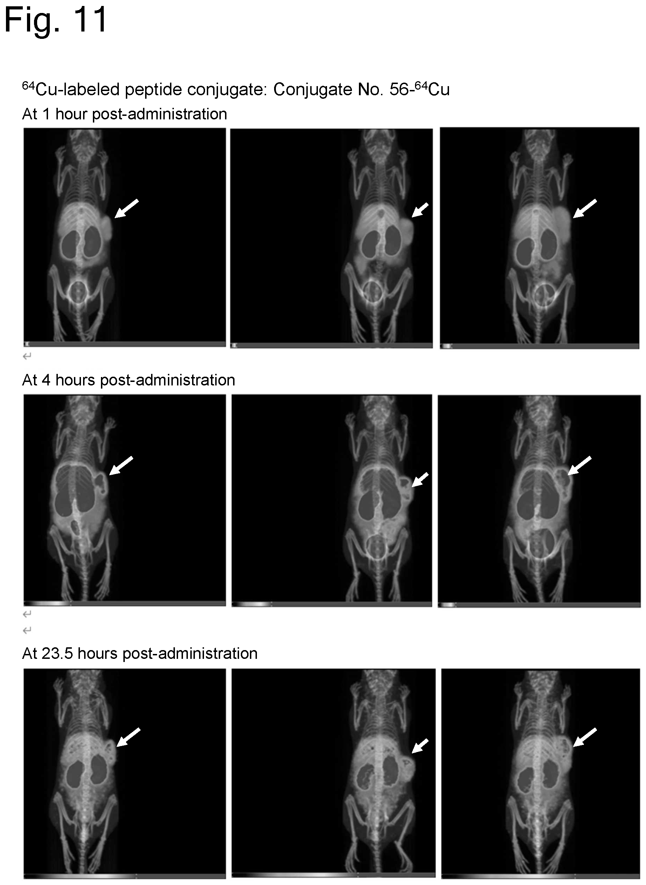

[Fig. 11] Fig. 11 is representative PET/CT images of a female BALB/cSlc-nu/nu tumor implantation model mouse at 1, 4, and 23.5 hours after the administration of Conjugate No. 56-⁶⁴Cu, namely a conjugate comprising a peptide of the present invention with rDOTAGA-⁶⁴Cu as a payload. White arrows in the figure indicate the localization of the implanted tumor.

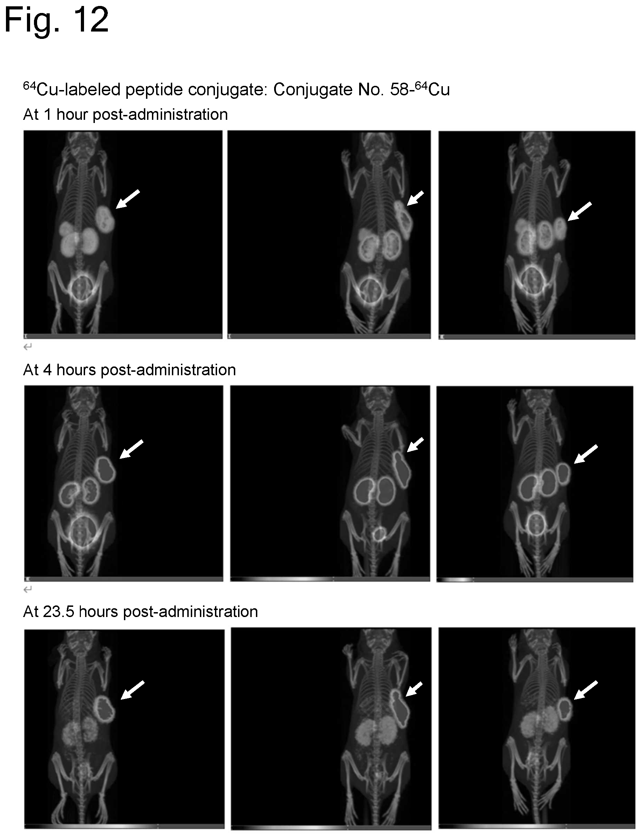

[Fig. 12] Fig. 12 is representative PET/CT images of a female BALB/cSlc-nu/nu tumor implantation model mouse at 1, 4, and 23.5 hours after the administration of Conjugate No. 58-⁶⁴Cu, namely a conjugate comprising a peptide of the present invention with rNODAGA-⁶⁴Cu as a payload. White arrows in the figure indicate the localization of the implanted tumor.

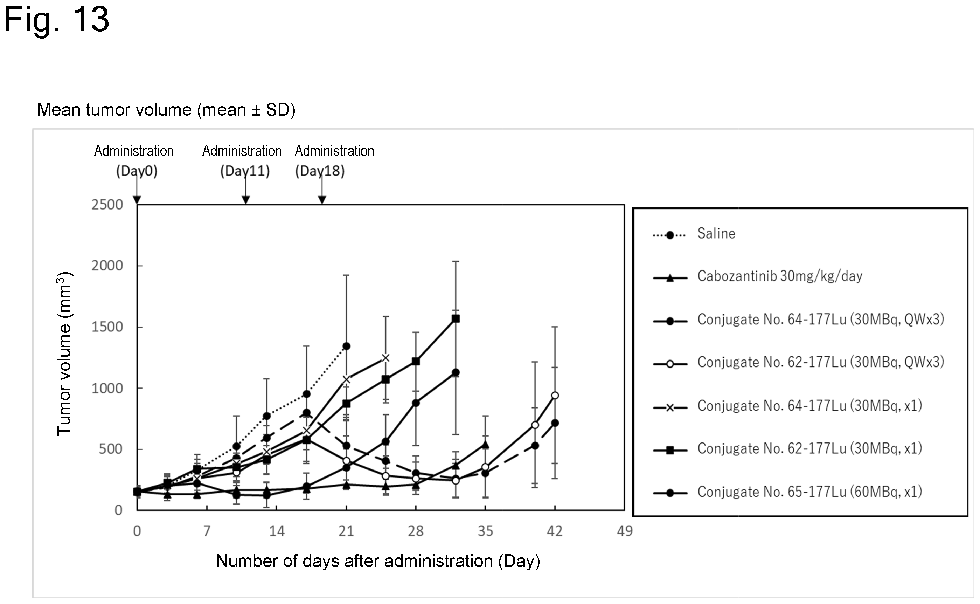

[Fig. 13] Fig. 13 is a graph showing the time-course of mean tumor volume in female BALB/cSlc-nu/nu tumor implantation model mice treated with each conjugate comprising each peptide of the present invention with DOTA-¹⁷⁷Lu as a payload, saline, or Cabozantinib. Note that the horizontal axis indicates the number of days after administration, and the vertical axis indicates the tumor volume (mm³). In addition, the black circle with a short dashed line represents saline; the black triangle with a solid line represents Cabozantinib; the black circle with a long dashed line represents Conjugate No. 64-¹⁷⁷Lu (three doses); the white circle with a solid line represents Conjugate No. 62-¹⁷⁷Lu (three doses); the "X" with a solid line represents Conjugate No. 64-¹⁷⁷Lu (single dose); the black square with a solid line represents Conjugate No. 62-¹⁷⁷Lu (single dose); and the black circle with a solid line represents Conjugate No. 65-¹⁷⁷Lu (60 MBq, single dose).

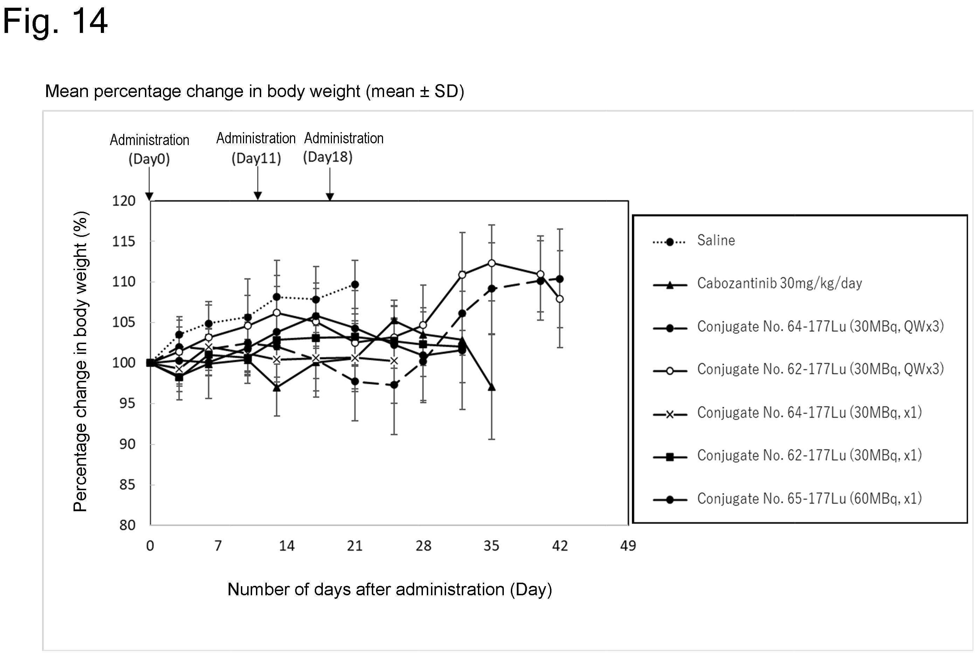

[Fig. 14] Fig. 14 is a graph showing the mean percentage change in body weight of female BALB/cSlc-nu/nu tumor implantation model mice treated with each conjugate comprising each peptide of the present invention with DOTA-¹⁷⁷Lu as a payload, saline, or Cabozantinib. Note that the horizontal axis indicates the number of days after administration, and the vertical axis indicates the percentage change in body weight (%). In addition, the black circle with a short dashed line represents saline; the black triangle with a solid line represents Cabozantinib; the black circle with a long dashed line represents Conjugate No. 64-¹⁷⁷Lu (three doses); the white circle with a solid line represents Conjugate No. 62-¹⁷⁷Lu (three doses); the "X" with a solid line represents Conjugate No. 64-¹⁷⁷Lu (single dose); the black square with a solid line represents Conjugate No. 62-¹⁷⁷Lu (single dose); and the black circle with a solid line represents Conjugate No. 65-¹⁷⁷Lu (60 MBq, single dose).

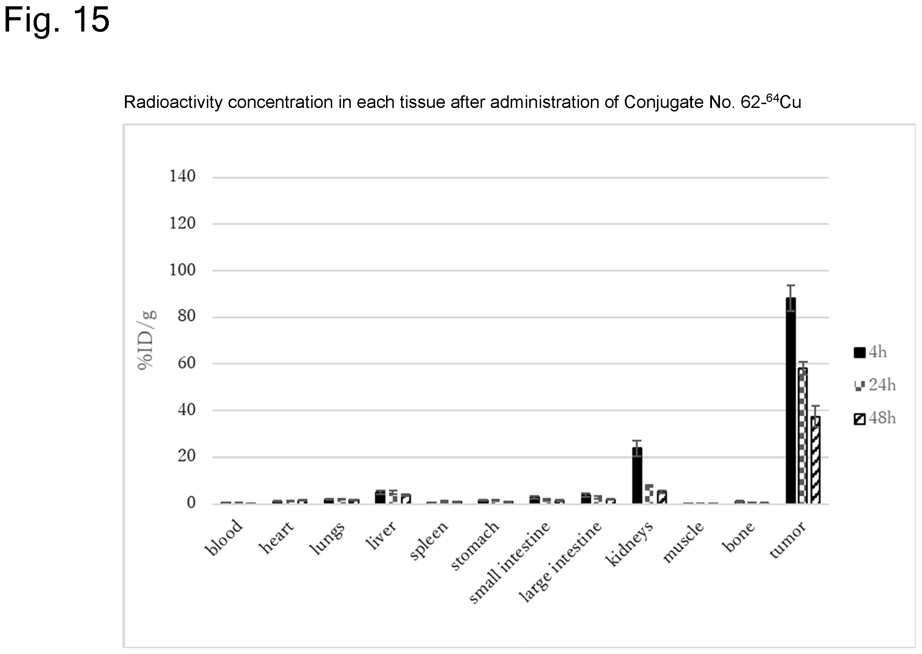

[Fig. 15] Fig. 15 is a graph showing the results of *in vivo* distribution of Conjugate No. 62-⁶⁴Cu comprising a peptide of the present invention with DOTA-⁶⁴Cu as a payload, at 4, 24, and 48 hours after the administration, in a VMRC-RCW tumor-bearing mouse. Note that the horizontal axis indicates each tissue, and the vertical axis indicates the radioactivity concentration (%ID/g) in each tissue.

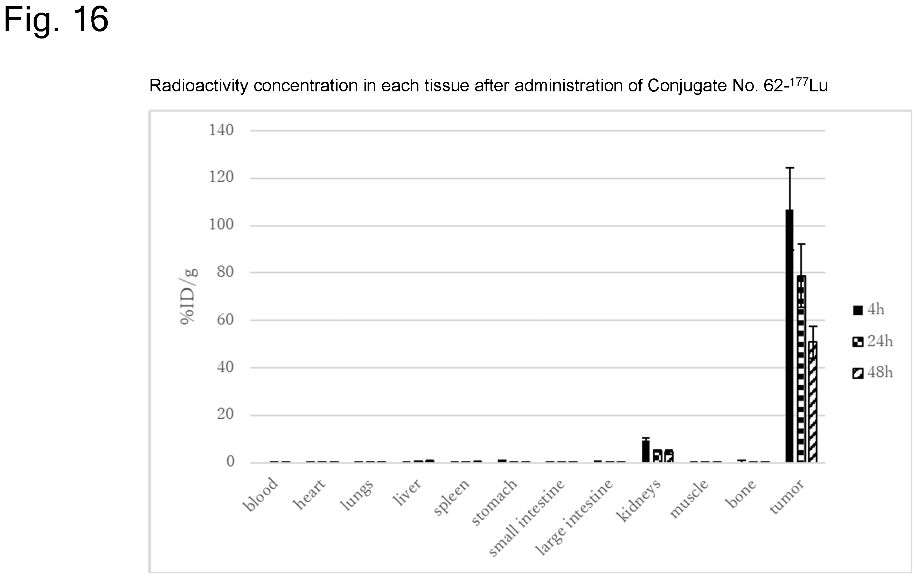

[Fig. 16] Fig. 16 is a graph showing the results of *in vivo* distribution of Conjugate No. 62-¹⁷⁷Lu comprising a peptide of the present invention with DOTA-¹⁷⁷Lu as a payload, at 1, 24, and 48 hours after the administration, in a VMRC-RCW tumor-bearing mouse. Note that the horizontal axis indicates each tissue, and the vertical axis indicates the radioactivity concentration (%ID/g) in each tissue.

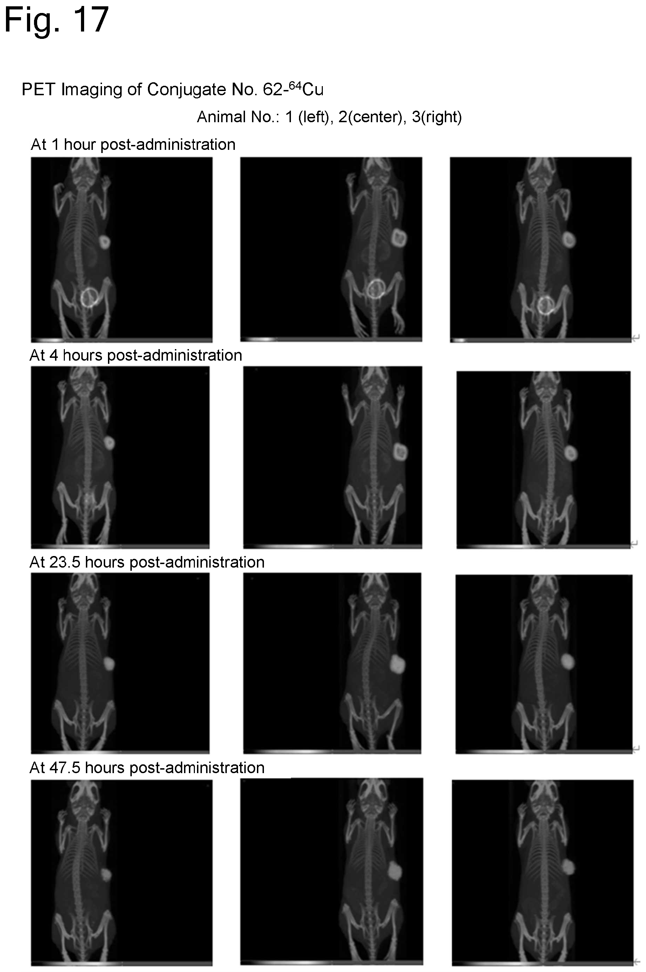

[Fig. 17] Fig. 17 is representative PET/CT images of a VMRC-RCW tumor-bearing mouse at 1, 4, 23.5, and 47.5 hours after the administration of Conjugate No. 62-⁶⁴Cu comprising a peptide of the present invention with DOTA-⁶⁴Cu as a payload.

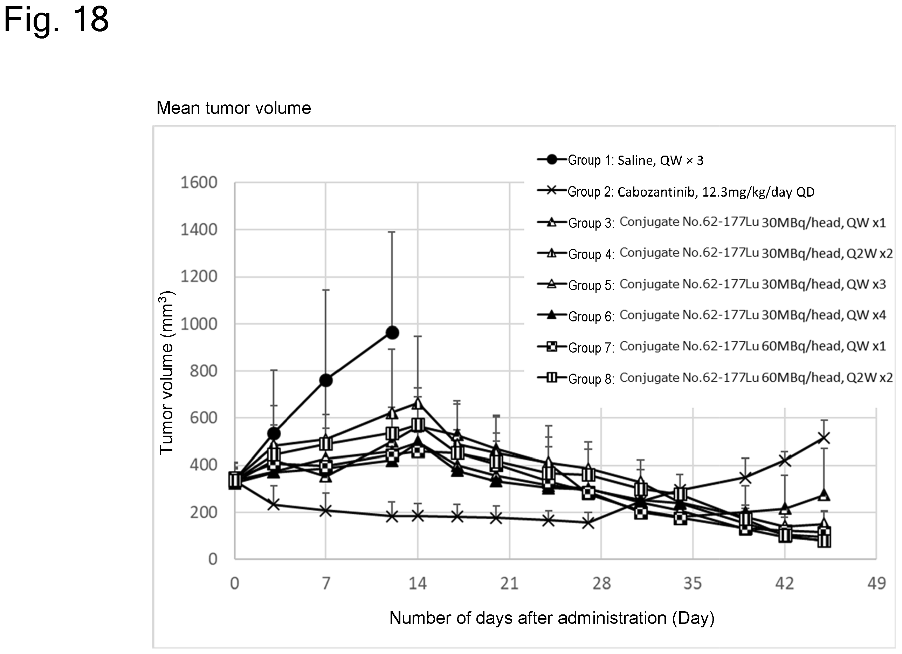

[Fig. 18] Fig. 18 is a graph showing the mean tumor volume in VMRC-RCW tumor-bearing mice treated with Conjugate No. 62-¹⁷⁷Lu comprising a peptide of the present invention with DOTA-¹⁷⁷Lu as a payload, saline, or Cabozantinib. Note that the horizontal axis indicates the number of days after administration, and the vertical axis indicates the tumor volume (mm³). In addition, the black circle represents Group 1: saline; "X" represents Group 2: Cabozantinib; the checkered triangle represents Group 3: Conjugate No. 62-¹⁷⁷Lu (30 MBq, single dose); the vertically striped triangle represents Group 4: Conjugate No. 62-¹⁷⁷Lu (30 MBq, two doses); the horizontally striped triangle represents Group 5: Conjugate No. 62-¹⁷⁷Lu (30 MBq, three doses); the black triangle represents Group 6: Conjugate No. 62-¹⁷⁷Lu (30 MBq, four doses); the checkered square represents Group 7: Conjugate No. 62-¹⁷⁷Lu (60 MBq, single dose); and the vertically striped square represents Group 8: Conjugate No. 62-¹⁷⁷Lu (60 MBq, two doses).

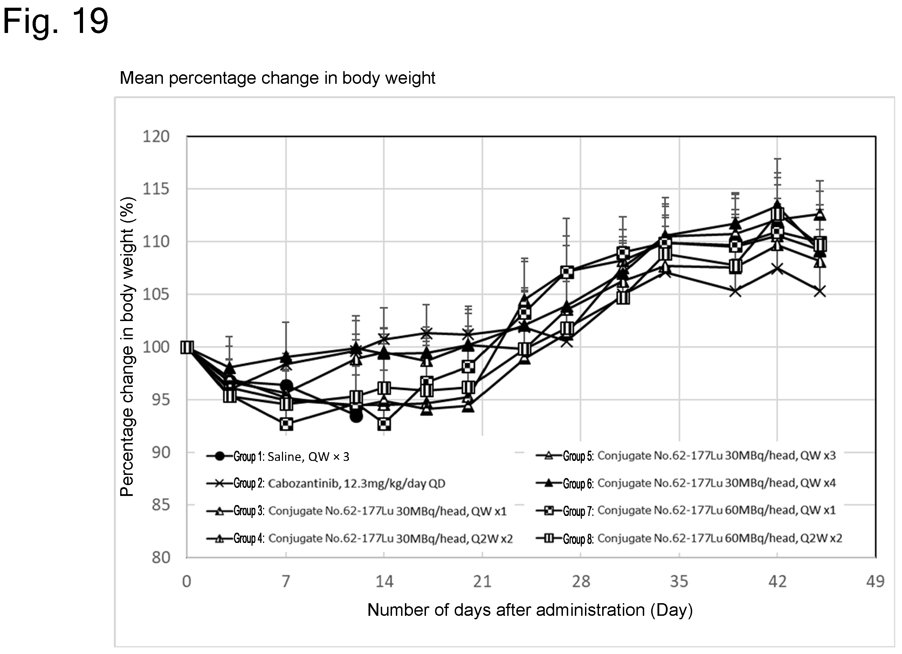

[Fig. 19] Fig. 19 is a graph showing the mean percentage change in body weight of VMRC-RCW tumor-bearing mice treated with Conjugate No. 62-¹⁷⁷Lu comprising a peptide of the present invention with DOTA-¹⁷⁷Lu as a payload, saline, or Cabozantinib. Note that the horizontal axis indicates the number of days after administration, and the vertical axis indicates the percentage change in body weight (%). In addition, the black circle represents Group 1: saline; "X" represents Group 2: Cabozantinib; the checkered triangle represents Group 3: Conjugate No. 62-¹⁷⁷Lu (30 MBq, single dose); the vertically striped triangle represents Group 4: Conjugate No. 62-¹⁷⁷Lu (30 MBq, two doses); the horizontally striped triangle represents Group 5: Conjugate No. 62-¹⁷⁷Lu (30 MBq, three doses); the black triangle represents Group 6: Conjugate No. 62-¹⁷⁷Lu (30 MBq, four doses); the checkered square represents Group 7: Conjugate No. 62-¹⁷⁷Lu (60 MBq, single dose); and the vertically striped square represents Group 8: Conjugate No. 62-¹⁷⁷Lu (60 MBq, two doses).

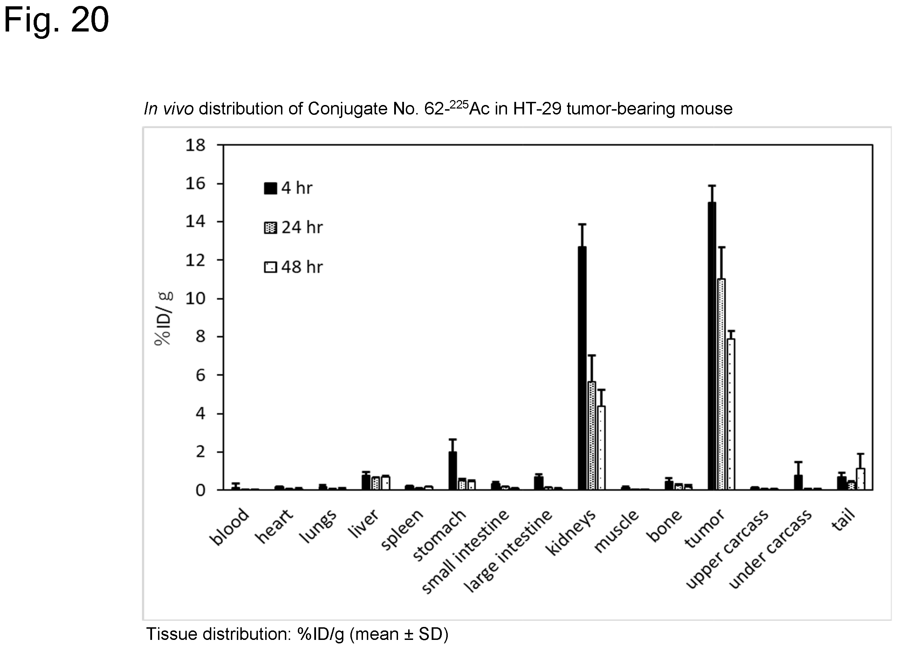

[Fig. 20] Fig. 20 is a graph showing the results of *in vivo* distribution of Conjugate No. 62-²²⁵Ac comprising a peptide of the present invention with DOTA-²²⁵Ac as a payload, at 4, 24, and 48 hours after the administration, in a HT-29 tumor-bearing mouse. Note that the horizontal axis indicates each tissue, and the vertical axis indicates the radioactivity concentration (%ID/g) in each tissue.

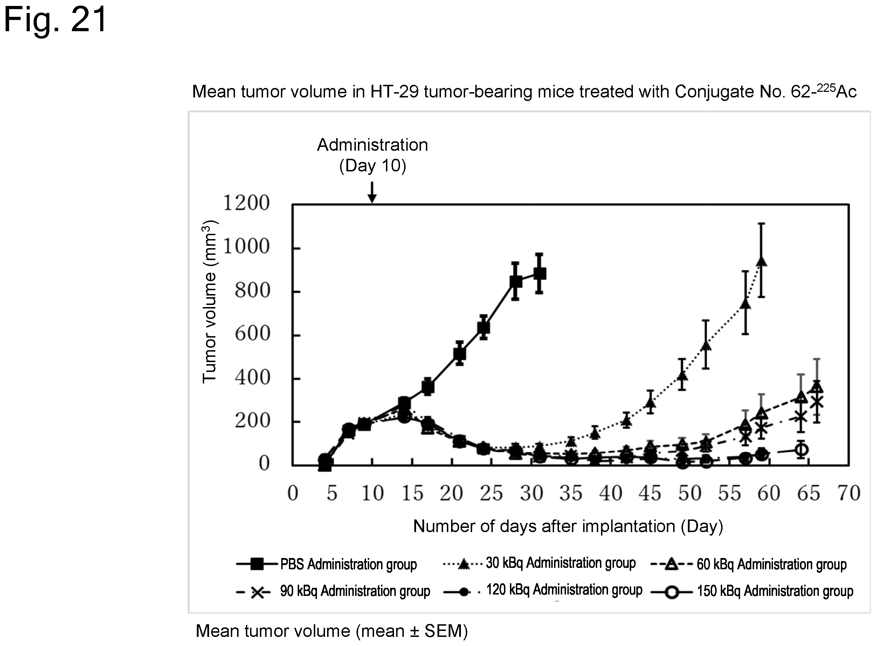

[Fig. 21] Fig. 21 is a graph showing the mean tumor volume in HT-29 tumor-bearing mice treated with Conjugate No. 62-²²⁵Ac comprising a peptide of the present invention with DOTA-²²⁵Ac as a payload or saline. Note that the horizontal axis indicates the number of days after administration, and the vertical axis indicates the tumor volume (mm³). In addition, the black square represents Group 1: saline; the black triangle represents Group 2: Conjugate No. 62-²²⁵Ac (30 MBq dose); the white triangle represents Group 3: Conjugate No. 62-²²⁵Ac (60 MBq dose); "X" represents Group 4: Conjugate No. 62-²²⁵Ac (90 MBq dose); the black circle represents Group 5: Conjugate No. 62-²²⁵Ac (120 MBq dose); and the white circle represents Group 6: Conjugate No. 62-²²⁵Ac (150 MBq dose).

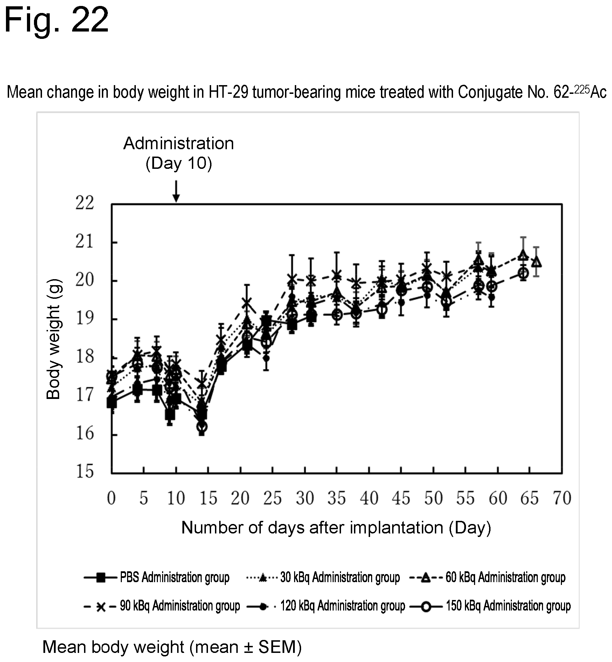

[Fig. 22] Fig. 22 is a graph showing the mean change in body weight of HT-29 tumor-bearing mice treated with Conjugate No. 62-²²⁵Ac comprising a peptide of the present invention with DOTA-²²⁵Ac as a payload or saline. Note that the horizontal axis indicates the number of days after implantation, and the vertical axis indicates the mean body weight (g). In addition, the black square represents Group 1: saline; the black triangle represents Group 2: Conjugate No. 62-²²⁵Ac (30 MBq dose); the white triangle represents Group 3: Conjugate No. 62-²²⁵Ac (60 MBq dose); "X" represents Group 4: Conjugate No. 62-²²⁵Ac (90 MBq dose); the black circle represents Group 5: Conjugate No. 62-²²⁵Ac (120 MBq dose); and the white circle represents Group 6: Conjugate No. 62-²²⁵Ac (150 MBq dose).

### DESCRIPTION OF EMBODIMENTS

The present invention includes, but is not limited thereto, the following embodiments. Unless otherwise indicated herein, technical and scientific terms used herein have the same meanings as those ordinarily understood by those skilled in the art. The substances, materials, and examples disclosed herein are merely illustrative and not intended to be limiting. Reference to "in one embodiment" herein means that it is not limited to the embodiment, i.e., it is not limited.

### 1. Abbreviations

As used herein, the following abbreviations are used with the following meanings unless otherwise indicated.

### Abbreviations (general)

Å: angstrom (unit);
ClAc: chloroacetyl;
Cy5SAlk: sulfo-Cy5-alkyne;
DCM: dichloromethane;
TIPS: triisopropylsilyl;
*tert*: tertiary;
DTT: dithiothreitol;
DMSO: dimethyl sulfoxide;
Trt: trityl;
Boc: tert-butoxycarbonyl;
DMF: N,N-dimethylformamide;
DIEA or DIPEA: N,N-diisopropylethylamine;
DIPCI or DIC: N,N'-diisopropylcarbodiimide;
Oxyma pure: ethyl cyano(hydroxyimino)acetate;
DODT: 3,6-dioxa-1,8-octanedithiol;
DOTA-NHS ester: 2,2',2"-(10-(2-((2,5-dioxopyrrolidin-1-yl)oxy)-2-oxoethyl)-1,4,7,10-tetraazacyclododecan-1,4,7-triyl)triacetic acid;
DOTA: 1,4,7,10-tetraazacyclododecan-1,4,7,10-tetraacetic acid;
Fmoc: 9-fluorenylmethyloxycarbonyl;
g: gram (unit);
H₂O: water;
HCl: hydrogen chloride;
HATU: O-(7-azabenzotriazol-1-yl)-N,N,N',N'-tetramethyluronium hexafluorophosphate;
HOSu: N-hydroxysuccinimide;
HPLC: high-performance liquid chromatography;
LC-MS or LC/MS: liquid chromatography mass spectrometer;
MeOH: methanol;
mL: milliliter (unit);
M: molar (unit);
µL: microliter (unit);
mM: millimolar (unit);
µM: micromolar (unit);
mmol: millimole (unit);
mg: milligram (unit);
MeCN or CH₃CN: acetonitrile;
min: minute (unit);
mm: millimeter (unit);
µm: micrometer (unit);
nm: nanometer (unit);
nM: nanomolar (unit);
NMP: N-methyl-2-pyrrolidone;
OSu: succinimide;
PEG: polyethylene glycol;
rpm: revolutions per min (unit);
tBu: *tert*-butyl;
TFA: trifluoroacetic acid;
TIS: triisopropylsilane;
Trt or Tr: trityl group;
H-PEG4Me: 2,5,8,11-tetraoxatridecan-13-amine;
H-PEG8Me: 2,5,8,11,14,17,20,23-octaoxapentacosan-25-amine;
AA: amino acid;
PyAOP: 7-((azabenzotriazol-1-yloxy)tripyrrolidinophosphonium hexafluorophosphate;
CSA: 10-camphorsulfonic acid;
Fmoc-OSu: N-(9-fluorenylmethoxycarbonyloxy)succinimide;
THF: tetrahydrofuran;
ClAcOSu: N-(chloroacetoxy)succinimide;
Pd₂(dba)₃·CHCl₃: tris(dibenzylideneacetone)dipalladium(0)-chloroform complex;
SPhos: 2-dicyclohexylphosphino-2',6'-dimethoxybiphenyl;
EDCI·HCl: 1-(3-dimethylaminopropyl)-3-ethylcarbodiimide hydrochloride;
Pd(PPh₃)₄: tetrakis(triphenylphosphine)palladium(0);
ClAcOH: chloroacetic acid;
conc: concentration;
HFIP: 1,1,1,3,3,3-hexafluoro-2-propanol;
Pbf: 2,2,4,6,7-pentamethyldihydrobenzofuran-5-sulfonyl;
Alloc: allyloxycarbonyl;
Allyl: allyl;
Fmoc-Qglucamine-NH2: (9H-fluoren-9-yl)methyl((S)-1-amino-1,5-dioxo-5-(((2S,3R,4R,5R)-2,3,4,5,6-pentahydroxyhexyl)amino)pentan-2-yl)carbamate;
H-Qglucamine-NH2: (S)-4-amino-N1-((2S,3R,4R,5R)-2,3,4,5,6-pentahydroxyhexyl)pentanediamide;
H-KCOpipzaa(Mpe)-NH2: 3-methylpentan-3-yl(S)-2-(4-((5,6-diamino-6-oxohexyl)carbamoyl)piperazin-1-yl);
TEA: triethylamine.

### Abbreviations (non-natural amino acids)

The abbreviations below for non-natural amino acids include those in which the amino group on the main chain is protected by a common protecting group such as a Boc or Fmoc group.

**[Table 1-1]**

| List of non-natural amino acids | |
|---|---|
| Abbreviation | Structure |
| da | 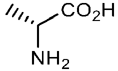 CAS:338-69-2 |
| de | 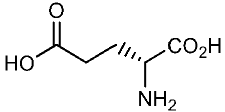 CAS:6893-26-1 |
| dhgl | 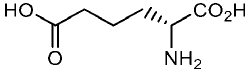 CAS:7620-28-2 |
| dq | 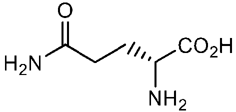 CAS:5959-95-5 |
| dk | 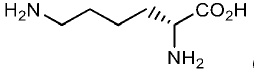 CAS:923-27-3 |
| dyae | 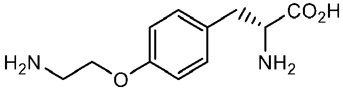 (2R)-2-Amino-3-[4-(2-aminoethoxy)phenyl]propanoic acid |
| df | 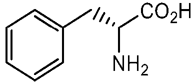 CAS:673-06-3 |
| dkCOpipzaa | 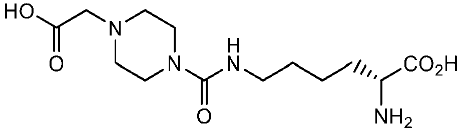 CAS:2973757-42-3 |
| Tbg | 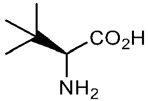 CAS:20859-02-3 |
| I | 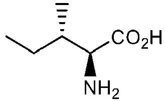 CAS:73-32-5 |

**[Table 1-2]**

| | |
|---|---|
| 3Py6CON | 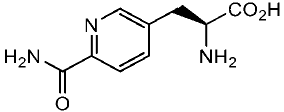 CAS:2973752-27-9 |
| 3Py6NH2 | 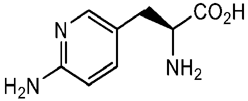 CAS: 1269968-61-7 |
| Hgl | 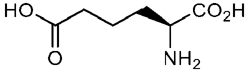 CAS:1118-90-7 |
| alT | 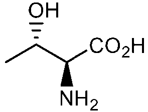 CAS:28954-12-3 |
| Hgn | 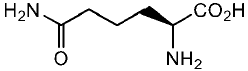 CAS:7433-32-1 |
| SMe | 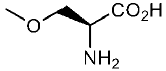 CAS:32620-11-4 |
| DabAc | 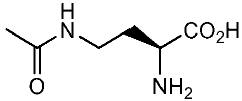 CAS:1190-46-1 |
| K | 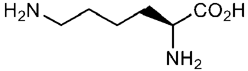 CAS:56-87-1 |
| KCOpipzaa | 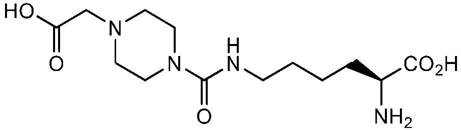 CAS:2913141-21-4 |
| Meda | 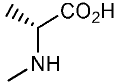 CAS:29475-64-7 |
| PeG | 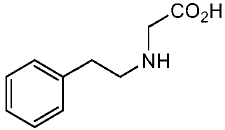 CAS:7738-38-7 |
| MeG | 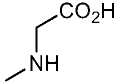 CAS:107-97-1 |

**[Table 1-3]**

| | |
|---|---|
| MedkCOpipzaa | 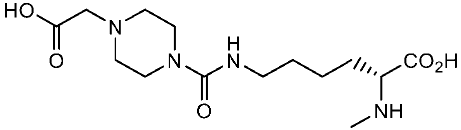 CAS:2973757-43-4 |
| Ahp | 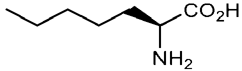 CAS:44902-02-5 |
| pHPeG | 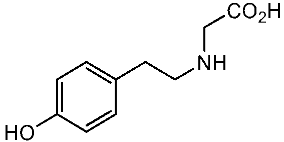 CAS:169836-45-7 |
| pMeOPeG | 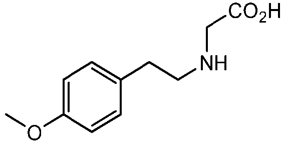 CAS:88720-74-5 |
| MsMeapG | 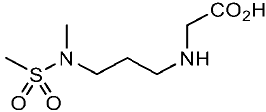 CAS:2030455-69-5 |
| 3OMePeG | 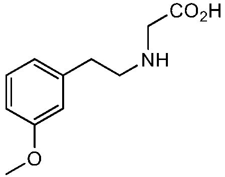 CAS:1267272-96-7 |
| PpG | 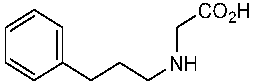 CAS:75822-20-7 |
| pFPeG | 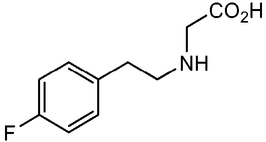 CAS:933736-89-1 |
| mCPeG | 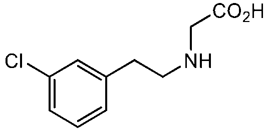 CAS:1267670-63-2 |

**[Table 1-4]**

| | |
|---|---|
| pCPeG | 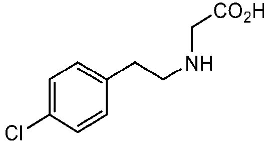 CAS:205124-63-6 |
| 4HPpG | 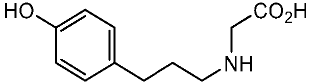 CAS:861566-97-4 |
| 4OMePpG | 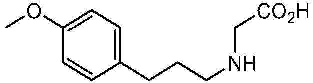 2-{[3-(4-Methoxyphenyl)propyl]amino}acetic acid |
| 4PypG | 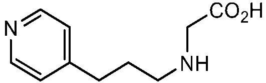 2-{[3-(Pyridin-4-yl)propyl]amino}acetic acid |
| 4COOPeG | 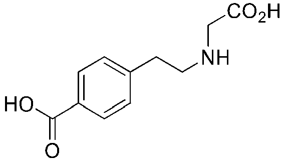 4-{2-[(Carboxymethyl)amino]ethyl}benzoic acid |
| 3COOPeG | 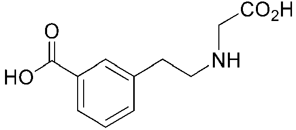 3-{2-[(Carboxymethyl)amino]ethyl}benzoic acid |
| 3FPeG | 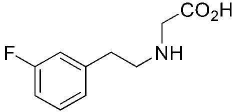 CAS:1155577-90-4 |
| S | 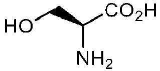 CAS:56-45-1 |
| H | 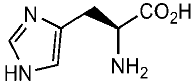 CAS:71-00-1 |
| P | 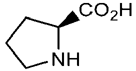 CAS:147-85-3 |
| D | 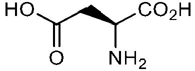 CAS:56-84-8 |

**[Table 1-5]**

| | |
|---|---|
| P4Sh | 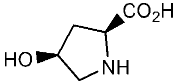 CAS:618-27-9 |
| Hse | 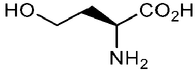 CAS:672-15-1 |
| Qmm | 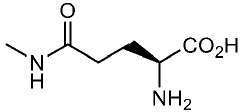 CAS:3081-62-7 |
| Qdm | 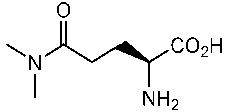 CAS:21432-98-4 |
| QPEG8Me | 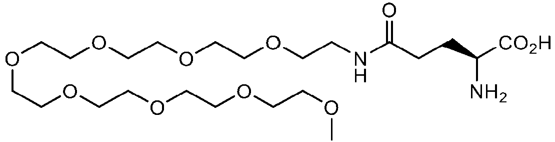 CAS:2913141-39-4 |
| Q | 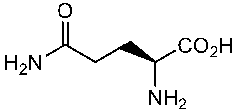 CAS:56-85-9 |
| 5Inda | 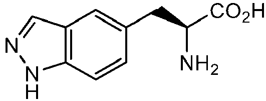 CAS:1335577-14-4 |
| F4OMe | 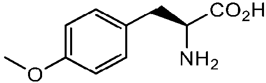 CAS:6230-11-1 |
| Y | 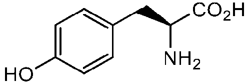 CAS:60-18-4 |
| HseMe | 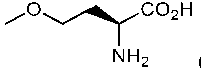 CAS:3311-01-1 |
| E | 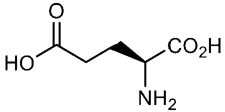 CAS:56-86-0 |
| G | 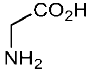 CAS:56-40-6 |

**[Table 1-6]**

| | |
|---|---|
| Acb | 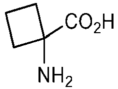 CAS:22264-50-2 |
| dkAc | 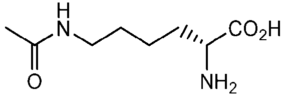 CAS:51621-57-9 |
| dnle | 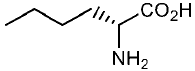 CAS:327-56-0 |
| t4amCh | 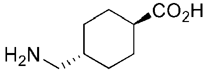 CAS:1197-18-8 |
| Nle | 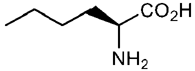 CAS:327-57-1 |
| F | 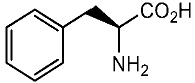 CAS:63-91-2 |
| MeC | 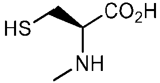 CAS:4026-48-6 |
| C | 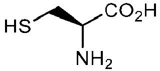 CAS:52-90-4 |
| MeA | 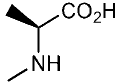 CAS:3913-67-5 |
| MeS | 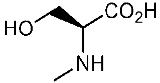 CAS:2480-26-4 |
| MeN | 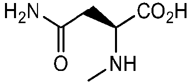 CAS:19026-58-5 |
| Hpr | 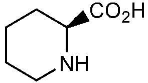 CAS:3105-95-1 |
| MeCt | 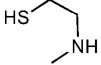 CAS:10061-40-2 |

**[Table 1-7]**

| | |
|---|---|
| DOTA | 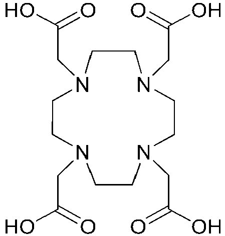 CAS:60239-18-1 |
| PEG8Me | 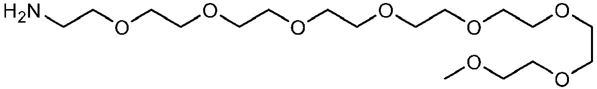 CAS:869718-81-0 |
| SulfoCy5 | 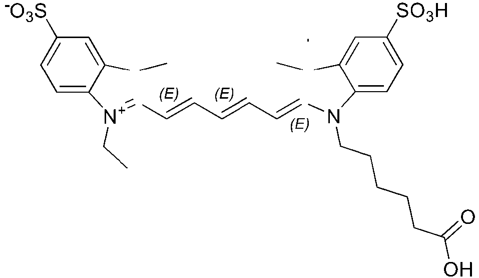 CAS:146368-11-8 |
| PEG8c | 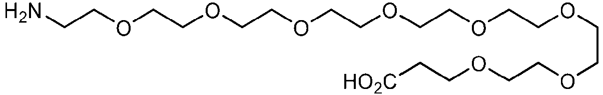 CAS:756526-04-2 |
| rDOTAGA (or DOTAGA) | 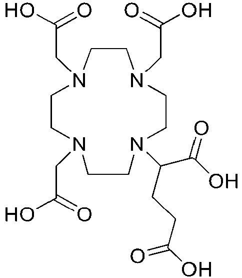 CAS:656242-49-8 |

**[Table 1-8]**

| | |
|---|---|
| rNODAGA (or NODAGA) | 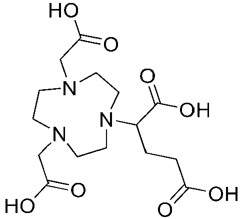 CAS:1092370-12-1 |
| PEG10c | 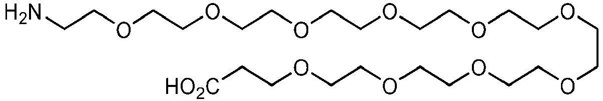 CAS:2170987-85-4 |
| Qglucamine | 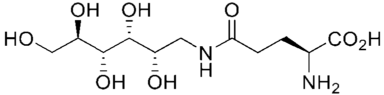 CAS:1956384-48-7 |

### 2. Peptide

The present invention relates to a peptide or a pharmaceutically acceptable salt thereof. References herein to "peptide" also include references to a pharmaceutically acceptable salt thereof, an isomer, or a solvate thereof, as long as there is no particular contradiction.

An embodiment of the present invention provides a peptide comprising the following amino acid sequence:
da-Tbg-3Py6CON-Hgl-Meda-Ahp-PeG-S-3Py6NH2-HseMe-dk-Y (SEQ ID No. 1) or
an amino acid sequence having a substitution, addition, deletion, or insertion in 1 to 10 amino acid residues selected from the group consisting of amino acid residues at positions 1, 2, 3, 4, 5, 7, 8, 9, 10, and 11 in the above amino acid sequence.

The peptide of the present invention may be a peptide comprising an amino acid sequence having a substitution, addition, deletion, or insertion in 1 to 9 amino acid residues selected from the group consisting of amino acid residues at positions 1, 2, 3, 4, 5, 7, 8, 9, and 11 in the amino acid sequence set forth in SEQ ID No. 1.

The 1 to 10 amino acid residues selected from the group consisting of amino acid residues at positions 1, 2, 3, 4, 5, 7, 8, 9, 10, and 11 in the amino acid sequence set forth in SEQ ID No. 1 may have a substitution, addition, deletion, or insertion. The number of amino acid substitutions, deletions, additions, and/or insertions should be 1 or more and 10 or less and the lower limit is 1. The upper limit may be 10, 9, 8, 7, 6, 5, 4, 3, or 2, and the minimum is 1.

Preferred is an amino acid sequence having a substitution, addition, deletion, or insertion in 1 to 7 amino acid residues selected from the group consisting of amino acid residues at positions 1, 4, 7, 8, 9, 10, and 11 in the amino acid sequence set forth in SEQ ID No. 1. The number of amino acid substitutions, deletions, additions, and/or insertions should be 1 or more and 7 or less and the lower limit is 1. The upper limit may be 7, 6, 5, 4, 3, or 2, and the minimum is 1. In addition, preferred may be an amino acid sequence having a substitution, addition, deletion, or insertion in 1 to 6 amino acid residues selected from the group consisting of amino acid residues at positions 1, 4, 7, 8, 9, and 11 in the amino acid sequence set forth in SEQ ID No. 1.

In one embodiment, an amino acid substitution is preferred. Such an amino acid substitution is preferably a conservative amino acid substitution, but is not limited thereto.

The term "conservative amino acid substitution" means a substitution with a functionally equivalent or similar amino acid. A conservative amino acid substitution in a peptide causes a static change in the amino acid sequence of the peptide. For example, one or two or more amino acids with similar polarity act in a functionally equivalent manner to produce a static change in the amino acid sequence of such a peptide. In general, a substitution within a group can be considered structurally and functionally conservative. However, as obvious to those skilled in the art, the role played by a particular amino acid residue can be determined by its significance in the three-dimensional structure of the molecule containing that amino acid. For example, a cysteine residue can take a less polar, oxidized (disulfide) form when compared to the reduced (thiol) form. The long aliphatic portion of an arginine side chain can constitute a structurally and functionally important feature. In addition, a side chain having an aromatic ring (of tryptophan, tyrosine, or phenylalanine) can contribute to an ion-aromatic or cation-pi interaction. In such cases, substitution of an amino acid having such a side chain with an amino acid belonging to an acidic or nonpolar group can be structurally and functionally conservative. A residue such as proline, glycine, or cysteine (disulfide form) can have a direct effect on the conformation of the main chain and often cannot be replaced without structural distortion.

The conservative amino acid substitution includes a specific substitution based on side chain similarity (e.g., L. Lehninger, Biochemistry, 2nd edition, pp73-75, Worth Publisher, New York (1975)) and a typical substitution as shown below. In addition, for example, the conservative amino acid substitution is preferably a substitution to an amino acid belonging to the same group as a group of amino acids as grouped on the basis of side chain properties common to those of the following natural amino acids.

Hydrophobic (also called nonpolar) amino acids: amino acids that are hydrophobic (nonpolar) and include alanine ("Ala" or simply "A"), glycine ("Gly" or simply "G"), valine ("Val" or simply "V"), leucine ("Leu" or simply "L"), isoleucine ("Ile" or simply "I"), proline ("Pro" or simply "P"), phenylalanine ("Phe" or simply "F"), tryptophan ("Trp" or simply "W"), tyrosine ("Tyr" or simply "Y"), and methionine ("Met" or simply "M").

Note that the hydrophobic amino acids can be further divided into the following groups.

Aliphatic amino acids: amino acids with fatty acid or hydrogen in the side chain, including Ala, Gly, Val, Ile, and Leu.

Aliphatic/branched-chain amino acids: amino acids with branched fatty acid in the side chain, including Val, Ile, and Leu.

Aromatic amino acids: amino acids having an aromatic ring in the side chain, including Trp, Tyr, and Phe.

Hydrophilic (also called polar) amino acids: amino acids that are hydrophilic (polar) and include serine ("Ser" or simply "S"), threonine ("Thr" or simply "T"), cysteine ("Cys" or simply "C"), asparagine ("Asn" or simply "N"), glutamine ("Gln" or simply "Q"), aspartic acid ("Asp" or simply "D"), glutamic acid ("Glu" or simply "E"), and lysine ("Lysine"; "Lys" or simply "K"), arginine ("Arg" or simply "R"), and histidine ("His" or simply "H").

Note that the hydrophilic amino acids can be further divided into the following groups.

Acidic amino acids: amino acids with an acidic side chain, including Asp and Glu.

Basic amino acids: amino acids with a basic side chain, including Lys, Arg, and His.

Neutral amino acids: amino acids with a neutral side chain, including Ser, Thr, Asn, Gln, and Cys.

Gly and Pro can also be classified as "amino acids affecting the direction of the main chain", and amino acids containing a sulfur molecule in the side chain, Cys and Met, can be classified as "sulfur-containing amino acids".

As used herein, the "amino acids" include not only natural amino acids but also non-natural amino acids. Examples of the non-natural amino acids include N-alkylamino acids, in which the above-described natural amino acid is N-alkylated, and those in which the nitrogen forming a peptide bond is modified with a branched or unbranched lower (e.g., C1-C5, preferably C1-C3, more preferably C1) alkyl group. The N-alkylamino acids are preferably N-ethylamino acids, N-butylamino acids, or N-methylamino acids, and more preferably N-methylamino acids. In addition, examples of the non-natural amino acids include D-type amino acids (also called D-amino acids), β-amino acids, γ-amino acids, amino acid mutants, chemically modified amino acids such as amino acid derivatives, and amino acids, such as norleucine and ornithine, which are not used as building blocks of proteins *in vivo.* Further, the examples include amino acids in which the side chain of a natural amino acid further contains a functional group or is replaced by other functional group (e.g., amino acids with, for example, a side chain arylene or alkylene group moiety with substitution and/or addition, amino acids with an increased number of carbon atoms in an arylene, alkylene or alkyl group in the side chain, amino acids with a substituted aromatic ring in a side chain, heterocyclized or cyclocondensed amino acids).

Note that a natural amino acid side chain may contain or be replaced by a structure such as a functional group. This can impart a property different from that of a natural amino acid. For example, Dap is an amino acid with an amino group in the side chain of alanine. Here, the amino group is added to exhibit a property of basic, polar amino acid, which differs from alanine belonging to a nonpolar amino acid group. In other words, non-natural amino acids with similar side chain properties can be included in the above-described groups obtained by classifying natural amino acids based on their common side chain properties. For example, N-methylarginine (MeR), an amino acid in which the main chain nitrogen atom of arginine, which belongs to basic amino acids, is methylated, is a non-natural amino acid, but is basic. Thus, it can be classified as a basic amino acid. As such, non-natural amino acids that exhibit side-chain properties similar to those of a certain amino acid can also be included as targets for conservative amino acid substitution. Note that D-amino acids such as de(D-glutamic acid) can be classified as D-amino acids or according to the nature of their side chains. N-methylamino acids can be classified as N-alkylamino acids or classified according to the nature of the side chain of the original amino acid that is not N-methylated.

Non-natural amino acids include, but is not limited thereto, N-methylamino acids and D-amino acids, and more specifically, da, de, dhgl, dq, dk, dyae, df, dkCOpipzaa, Tbg, 3Py6CON, 3Py6NH2, Hgl, alT, Hgn, SMe, DabAc, KCOpipzaa, Meda, PeG, MeG, MedkCOpipzaa, Ahp, pHPeG, pMeOPeG, MsMeapG, 3OMePeG, PpG, pFPeG, mCPeG, pCPeG, 4HPpG, 4OMePpG, 4PypG, 4COOPeG, 3COOPeG, 3FPeG, P4Sh, Hse, Qmm, Qdm, QPEG8Me, 5Inda, F4OMe, HseMe, Acb, dkAc, dnle, t4amCh, Nle, MeC, MeA, MeS, MeN, Hpr, Hgn(Qglucamine-NH2), Hgn(KCOpipzaa-NH2), Qglucamine, and MeCt and other amino acids.

The "pharmaceutically acceptable salt thereof' means a salt of any of the peptides. Examples of the pharmaceutically acceptable salt include salts of mineral acids (e.g., sulfuric acid, hydrochloric acid, phosphoric acid), salts of organic acids (e.g., acetic acid, oxalic acid, lactic acid, tartaric acid, fumaric acid, maleic acid, methanesulfonic acid, benzenesulfonic acid), salts of amines (e.g., trimethylamine methylamine), and salts of metal ions (e.g., sodium, potassium, or calcium ions). For compounds that come to contain moisture over time, such moisture is also included in the pharmaceutically acceptable salt thereof.

In addition, the peptide may also be isomers such as stereoisomers thereof. The peptide may have one or more stereocenters, which can exist independently in either the (R) or (S) configuration. As used herein, the peptide may be an optically active form or a racemate, and the phrase "may be an isomer" means that it includes a racemate, an optically active form, a positional isomer, and a stereoisomer, or any combination thereof. In addition, in one embodiment, a mixture of one or more isomers may be allowed. Note that the "peptide stereoisomer" means a stereoisomer of the peptide. References herein to "peptide" include its isomers unless otherwise mentioned.

In addition, the peptide may also be a solvate. Solvation means a phenomenon in which ions generated by ionization of solute molecules (peptides or conjugates) are surrounded and associated with solvent molecules through electrostatic forces, hydrogen bonding, or the like, whereby the solute becomes dispersed in the solvent. The type of solvent is not particularly limited. When the solvent is water, it is especially called "hydrate".

The following peptides are examples of peptides confirmed to have CA9 binding activity in the Examples herein:
da-Tbg-3Py6CON-Hgl-Meda-Ahp-PeG-S-3Py6NH2-HseMe-dk-Y (SEQ ID No. 1);
a peptide in which MeC, C, or MeCt is attached, as an amino acid residue at position 13, to the amino acid sequence set forth in SEQ ID No. 1; and
a peptide in which MeA, MeS, MeN, Hpr, G, or P is attached, as an amino acid residue at position 13, to the amino acid sequence of SEQ ID No. 1 and MeG, G, da, or Meda is further attached as an amino acid residue at position 14.

In one embodiment, the peptide of the present invention is a peptide in which
(1) MeC, C, or MeCt is attached, as an amino acid residue at position 13, to the amino acid sequence set forth in SEQ ID No. 1; or
(2) MeA, MeS, MeN, Hpr, G, or P is attached, as an amino acid residue at position 13, to the amino acid sequence of SEQ ID No. 1 and MeG, G, da, or Meda is further attached as an amino acid residue at position 14.

The above (1) and (2) differ in the amino acid residue at position 13. In one embodiment, when the peptide of the present invention is a cyclic peptide, the number of amino acid residues contained in the cyclic structure is not limited and may be 13 or 14. If an additional amino acid(s) is present at a terminus thereof, the number may be such a number or more.

In one embodiment, the peptide of the present invention satisfies the following one or two or more requirements:
the amino acid residue at position 1 (in SEQ ID No. 1) is da, dq, de, dhgl, or dk;
the amino acid residue at position 2 is I or Tbg;
the amino acid residue at position 3 is 3Py6NH2 or 3Py6CON;
the amino acid residue at position 4 is S, Hgl, Hgl(PEG8Me), alT, Hgn, SMe, KCOpipzaa, or DabAc;
the amino acid residue at position 5 is PeG, MeG, Meda, or MedkCOpipzaa;
the amino acid residue at position 7 is PeG, pMeOPeG, MsMeapG, 3OMePeG, pHPeG, PpG, pFPeG, mCPeG, pCPeG, 4HPpG, 4OMePpG, 4PypG, 4COOPeG, 3COOPeG, or 3FPeG;
the amino acid residue at position 8 is H, S, P, D, KCOpipzaa, Q(PEG8Me), P4Sh, Hse, SMe, Qmm, or Qdm;
the amino acid residue at position 9 is 3Py6NH2, 5Inda, Y, or F4OMe;
the amino acid residue at position 10 is Ahp, HseMe, or E; and
the amino acid residue at position 11 is dk, G, da, dq, dkCOpipzaa, dk(t4amCh), de, Acb, or dkAc.

The above options for amino acid residues at positions 1 to 12 in one of the above embodiments may be selected in any combination. The requirements include, but are not limited thereto, 1 or more, 2 or more, 3 or more, 4 or more, 5 or more, 6 or more, 7 or more, 8 or more, 9 or more, 10 or more, or 11 or more requirements described above. Preferably, all of the above 12 requirements are satisfied.

In one embodiment, the peptide of the present invention satisfies the following one or two or more requirements:
the amino acid residue at position 1 (in SEQ ID No. 1) is da, dq, de, dhgl, or dk;
the amino acid residue at position 2 is I or Tbg;
the amino acid residue at position 3 is 3Py6NH2 or 3Py6CON;
the amino acid residue at position 4 is S, Hgl, Hgl(PEG8Me), alT, Hgn, SMe, KCOpipzaa, or DabAc;
the amino acid residue at position 5 is PeG, MeG, Meda, or MedkCOpipzaa;
the amino acid residue at position 7 is PeG, pMeOPeG, MsMeapG, 3OMePeG, pHPeG, PpG, pFPeG, mCPeG, pCPeG, 4HPpG, 4OMePpG, 4PypG, 4COOPeG, 3COOPeG, or 3FPeG;
the amino acid residue at position 8 is H, S, P, D, KCOpipzaa, Q(PEG8Me), P4Sh, Hse, SMe, Qmm, or Qdm;
the amino acid residue at position 9 is 3Py6NH2, 5Inda, Y, or F4OMe;
the amino acid residue at position 10 is Ahp, HseMe, or E; and
the amino acid residue at position 11 is dk, G, da, dq, dkCOpipzaa, dk(t4amCh), de, Acb, or dkAc,
and wherein
(1) MeC, C, or MeCt is attached, as an amino acid residue at position 13, to the amino acid sequence set forth in SEQ ID No. 1 or
(2) MeA, MeS, MeN, Hpr, G, or P is attached, as an amino acid residue at position 13, to the amino acid sequence of SEQ ID No. 1 and MeG, G, da, or Meda is further attached as an amino acid residue at position 14.

The above options for amino acid residues at positions 1 to 12 in one of the above embodiments may be selected in any combination. The requirements include, but are not limited thereto, 1 or more, 2 or more, 3 or more, 4 or more, 5 or more, 6 or more, 7 or more, 8 or more, 9 or more, 10 or more, or 11 or more requirements described above. Preferably, all of the above 12 requirements are satisfied.

In one embodiment, the peptide of the present invention satisfies the following one or two or more requirements:
the amino acid residue at position 2 (in SEQ ID No. 1) is Tbg;
the amino acid residue at position 3 is 3Py6NH2;
the amino acid residue at position 5 is Meda;
the amino acid residue at position 7 is PeG or pHPeg;
the amino acid residue at position 9 is 3Py6NH2 or 5Inda; and
the amino acid residue at position 10 is HseMe.

The above options for amino acid residues at positions 1 to 10 in one of the above embodiments may be selected in any combination. The requirements include, but are not limited thereto, 1 or more, 2 or more, 3 or more, 4 or more, or 5 or more requirements described above. Preferably, all of the above 6 requirements are satisfied.

In one embodiment, the peptide of the present invention satisfies the following one or two or more requirements:
the amino acid residue at position 2 (in SEQ ID No. 1) is Tbg;
the amino acid residue at position 3 is 3Py6NH2;
the amino acid residue at position 5 is Meda;
the amino acid residue at position 7 is PeG or pHPeg;
the amino acid residue at position 9 is 3Py6NH2 or 5Inda; and
the amino acid residue at position 10 is HseMe,
and wherein
(1) MeC, C, or MeCt is attached, as an amino acid residue at position 13, to the amino acid sequence set forth in SEQ ID No. 1 or
(2) MeA, MeS, MeN, Hpr, G, or P is attached, as an amino acid residue at position 13, to the amino acid sequence of SEQ ID No. 1 and MeG, G, da, or Meda is further attached as an amino acid residue at position 14.

The above options for amino acid residues at positions 1 to 10 in one of the above embodiments may be selected in any combination. The requirements include, but are not limited thereto, 1 or more, 2 or more, 3 or more, 4 or more, or 5 or more requirements described above. Preferably, all of the above 6 requirements are satisfied.

In one embodiment, the peptide of the present invention satisfies the following requirements:
the amino acid residue at position 2 (in SEQ ID No. 1) is Tbg;
the amino acid residue at position 3 is 3Py6NH2;
the amino acid residue at position 5 is Meda;
the amino acid residue at position 7 is PeG or pHPeg;
the amino acid residue at position 9 is 3Py6NH2 or 5Inda; and
the amino acid residue at position 10 is HseMe.

In one embodiment, the peptide of the present invention is provided such that
the amino acid residue at position 2 (in SEQ ID No. 1) is Tbg;
the amino acid residue at position 3 is 3Py6NH2;
the amino acid residue at position 5 is Meda;
the amino acid residue at position 7 is PeG or pHPeg;
the amino acid residue at position 9 is 3Py6NH2 or 5Inda; and
the amino acid residue at position 10 is HseMe,
and wherein
(1) MeC, C, or MeCt is attached, as an amino acid residue at position 13, to the amino acid sequence set forth in SEQ ID No. 1 or
(2) MeA, MeS, MeN, Hpr, G, or P is attached, as an amino acid residue at position 13, to the amino acid sequence of SEQ ID No. 1 and MeG, G, da, or Meda is further attached as an amino acid residue at position 14.

In one embodiment, the peptide of the present invention satisfies the following one or two or more requirements:
the amino acid residue at position 1 (in SEQ ID No. 1) is de(PEG8Me), dk, dyae, or dkCOpipzaa;
the amino acid residue at position 2 is I or Tbg;
the amino acid residue at position 3 is 3Py6NH2 or 3Py6CON;
the amino acid residue at position 4 is K, KCOpipzaa, Hgn(Qglucamine-NH2), Hgn(KCOpipzaa-NH2), or Qglucamine;
the amino acid residue at position 5 is PeG, MeG, Meda, or MedkCOpipzaa;
the amino acid residue at position 7 is PeG, pMeOPeG, MsMeapG, 3OMePeG, pHPeG, PpG, pFPeG, mCPeG, pCPeG, 4HPpG, 4OMePpG, 4PypG, 4COOPeG, 3COOPeG, or 3FPeG;
the amino acid residue at position 8 is K;
the amino acid residue at position 9 is 3Py6NH2, 5Inda, Y, or F4OMe;
the amino acid residue at position 10 is Ahp, HseMe, or E; and
the amino acid residue at position 11 is dk(F) or dk(PEG8c).

The above options for amino acid residues at positions 1 to 12 in one of the above embodiments may be selected in any combination. The requirements include, but are not limited thereto, 1 or more, 2 or more, 3 or more, 4 or more, 5 or more, 6 or more, 7 or more, 8 or more, 9 or more, 10 or more, or 11 or more requirements described above. Preferably, all of the above 12 requirements are satisfied.

In addition, in one embodiment, an amino acid(s) contained in the peptide of the present invention may be further modified. A modification is, for example, the attachment of other compound to the side chain terminus of an amino acid. For example, a low or medium molecular weight compound such as PEG or glycan may be attached to the side chain terminus of lysine, aspartic acid, glutamic acid, arginine, glutamine, or tyrosine, or other amino acid may be attached.

The above peptide is, in one embodiment, a cyclic peptide. The "cyclic peptide" means a peptide in which two amino acids are bonded and all or part of which is cyclic. Examples of the above-mentioned peptide include those in which amino acids in a peptide form a cross-linked structure, those in which a cyclic structure is formed by lactam ring formation or macrocyclization reaction, and those having a lasso peptide-like structure. In other words, the cyclic peptide may have a part with a cyclic structure and may have a linear chain part.

Peptides generally have poor metabolic stability *in vivo,* and their large size makes it difficult for them to permeate the cell membrane. To address such issues, a peptide cyclization technique has been adopted. It has been suggested that the peptide cyclization increases protease resistance and thus increases their metabolic stability and restricts conformational changes, thereby increasing their rigidity and improving membrane permeability and affinity for a target protein.

In one embodiment, the peptide has a cyclic structure in which a chloroacetylated amino acid (preferably N-terminal amino acid (amino acid residue at position 1)) is bonded to a cysteine residue (including substituted cysteine, such as N-methylcysteine) contained in the peptide. In one embodiment, the peptide has a cyclic structure in which an N-terminal amino acid (amino acid residue at position 1) is bonded to a cysteine residue (including an optionally substituted cysteine residue or a compound containing a -SH group inside the structure) contained in the peptide. In one embodiment, the peptide has a cyclic structure in which an N-terminal amino acid (amino acid residue at position 1) is bonded to a cysteine residue (including substituted cysteine, such as N-methylcysteine) at position 13 contained in the peptide. In one embodiment, the peptide has a cyclic structure in which a chloroacetylated N-terminal amino acid (amino acid residue at position 1) is bonded to the cysteine residue at position 13 contained in the peptide or to an -SH group contained in a compound (group) having an -SH group. Examples of the compound (group) having an - SH group include MeCt. The "chloroacetylation" may also be "halogen acetylation" using other halogen. Further, the "acetylation" may also be "acylation" by an acyl group other than the acetyl group. In one embodiment, the peptide has a cyclic structure obtained by bonding a chloroacetylated amino acid residue at position 1 in the amino acid sequence set forth in SEQ ID No. 1 to a cysteine residue included in the peptide. In one embodiment, the peptide may be a structure in which an amino acid sequence listed in any one of SEQ ID Nos. 1-103 and a cysteine or a substituted cysteine contained in the peptide are bonded via an acetyl group. That is, as used herein, for example, the term "cyclic peptide consisting of SEQ ID No. XX" includes not only the amino acid sequence set forth in SEQ ID No. XX, but also a cyclic structure formed by bonding an acetyl group attached to the amino acid at position 1 contained in the amino acid sequence to S (sulfur atom) contained in a cysteine or substituted cysteine or a compound having an -SH group.

Some amino acids herein may be modified for cyclization of a peptide. The present invention also encompasses amino acids that have been partially modified as such. For example, as described above, a chloroacetyl group (ClAc group) may be added to the N-terminal amino acid, and may be bonded to a cysteine residue or an amino acid residue having an -SH group in the peptide to form a ring. Such various (natural/non-natural) amino acids containing a chloroacetyl group are also included in the amino acids herein.

In addition, in one embodiment, the peptide has a cyclic structure in which an N-terminal amino acid (amino acid residue at position 1) and a C-terminal amino acid (amino acid in the most C-terminal portion if an amino acid residue(s) such as an amino acid residue at position 13 or 14 is added to a C-terminal side of the amino acid sequence set forth in SEQ ID No. 1) are bonded. In one embodiment, the peptide has a cyclic structure in which an amino group of an N-terminal amino acid (amino acid residue at position 1) is bonded to a carboxyl group of an amino acid at position 13 included in the peptide. In addition, in one embodiment, an additional amino acid is further added to an amino acid residue at position 13, and a cyclic structure may be formed in which an N-terminal amino acid (amino acid residue at position 1) may be bonded to, for example an amino acid residue at position 14 or 15 obtained by adding a further additional amino acid residue(s) to the amino acid residue at position 13. In one embodiment, the peptide has a cyclic structure in which an amino group of an amino acid residue at position 1 in the amino acid sequence set forth in SEQ ID No. 1 included in the peptide is bonded to a carboxy group of an amino acid residue at position 14. In addition, in one embodiment, the peptide has a cyclic structure in which an amino group of an N-terminal amino acid (amino acid residue at position 1) is bonded to a functional group present at a side chain terminus of a C-terminal amino acid included in the peptide.

In one embodiment, the peptide comprises or consists of the amino acid sequence set forth in any one of SEQ ID Nos. 2-54. In one embodiment, the peptide is a cyclic peptide comprising or consisting of the amino acid sequence set forth in any one of SEQ ID Nos. 2-54. The peptide is a peptide consisting of the amino acid sequence set forth in any one of SEQ ID Nos. 2-54 or a peptide consisting of an amino acid sequence having 1 to 13 or 14 (preferably 1 to 8, 1 to 7, 1 to 6, 1 to 5, 1 to 4, 1 to 3, 2, or 1) amino acid residue substitutions, deletions, insertions, or additions therein. Note that for a sequence to which amino acid residues are added, a sequence having many more amino acid residues (e.g., 1 to 14 residues) may be added to the sequence, as long as the added portion is a linker. In addition, the peptide consisting of an amino acid sequence having the amino acid residue substitutions, deletions, insertions, or additions is preferably for example, those having CA9 binding activity.

In addition, in one embodiment, the peptide comprises or consists of the amino acid sequence set forth in any one of SEQ ID Nos. 55-103. In one embodiment, the peptide is a cyclic peptide comprising or consisting of the amino acid sequence set forth in any one of SEQ ID Nos. 55-103. The peptide is a peptide consisting of the amino acid sequence set forth in any one of SEQ ID Nos. 55-103 or a peptide consisting of an amino acid sequence having 1 to 13 or 14 (preferably 1 to 8, 1 to 7, 1 to 6, 1 to 5, 1 to 4, 1 to 3, 2, or 1) amino acid residue substitutions, deletions, insertions, or additions therein. Note that for a sequence to which amino acid residues are added, a sequence having many more amino acid residues (e.g., 1 to 14 residues) may be added to the sequence, as long as the added portion is a linker. In addition, the peptide consisting of an amino acid sequence having the amino acid residue substitutions, deletions, insertions, or additions is preferably for example, those having CA9 binding activity. In one embodiment, the peptide of the present invention does not contain a peptide comprising or consisting of SEQ ID No. 103.

Further, in one embodiment, the peptide comprises or consists of the amino acid sequence set forth in any one of SEQ ID Nos. 55-102. In one embodiment, the peptide is a cyclic peptide comprising or consisting of the amino acid sequence set forth in any one of SEQ ID Nos. 55-103. The peptide may be a peptide consisting of the amino acid sequence set forth in any one of SEQ ID Nos. 55-102 or a peptide consisting of an amino acid sequence having 1 to 13 or 14 (preferably 1 to 8, 1 to 7, 1 to 6, 1 to 5, 1 to 4, 1 to 3, 2, or 1) amino acid residue substitutions, deletions, insertions, or additions therein.

Furthermore, in one embodiment, the peptide may be a peptide consisting of the amino acid sequence set forth in SEQ ID No. 2 or 21 or a peptide consisting of an amino acid sequence having 1 to 13 or 14 (preferably 1 to 8, 1 to 7, 1 to 6, 1 to 5, 1 to 4, 1 to 3, 2, or 1) amino acid residue substitutions, deletions, insertions, or additions therein.

The peptide may contain an additional amino acid residue(s) in addition to the amino acid sequence of SEQ ID No. 1. The peptide may contain, without limitation, an additional amino acid residue(s) in addition to the amino acid sequence set forth in any one of SEQ ID Nos. 2-54, 55-102, or 55-103.

The "additional amino acid residue(s)" may be included in the peptide forming the cyclic structure, or the additional amino acid residue(s) may be added like a linker from the cyclic peptide. In one embodiment, it may be added to a side chain of an amino acid residue contained in the cyclic peptide. The peptide and the number of amide bonds (number and length of amino acids) in the peptide moiety are not limited.

In addition, an additional linker may also be added to the cyclic peptide. Examples of the linker include the above-described amino acid linker (peptide linker), a chemical linker, a fatty acid linker, a nucleic acid linker, and a sugar chain linker. The linker may also be a complex of, for example, a chemical linker and a peptide linker. The chemical linker may be, for example, a PEG linker consisting of 1-24 ethylene glycol units. The linker may also be a fatty acid linker containing a divalent chemical moiety derived from a fatty acid. The amino acid (peptide) linker may be a linker containing at least one amino acid. It is possible to use, for example, a glycine-rich peptide such as a peptide having the sequence [Gly-Gly-Gly-Gly-Gly-Ser]ₙ (where n is 1, 2, 3, 4, 5, or 6) as described in U.S. Patent No. 7,271,149 or a serine-rich peptide linker described in U.S. Patent No. 5,525,491. It is also possible to use a peptide linker in which two or more of amino acids such as K, dk, F, G, df, Nle, dnle, or t4amCh and amino acids are connected. Note that in the peptide linker, the bond between amino acids or between an amino acid and a chemical linker may be linked through the side chain of the amino acid. A linker may be added without limitation to change the physical properties (e.g., solubility) of the peptide.

The linker may be added at any position. For example, it may be linked to an amino acid residue located at the C-terminal side, or to an amino acid residue contained in a cyclic peptide. In one embodiment, the above peptide contains a linker at the C-terminus. Preferred are those bonded to a side chain of an amino acid residue contained in a cyclic peptide or compounds having a Cys or-SH group located on the C-terminal side, or bonded to any amino acid residue. More preferred are those bonded to a side chain of an amino acid residue at position 1, 4, 8, or 11.

In one embodiment, the peptide is a peptide in which a linker and/or a payload can bind to the C-terminus of the peptide.

In one embodiment, the peptide is a peptide in which an amino acid residue at position 1, 4, 8, or 11 or an amino acid residue or additional amino acid located on the C-terminal side is an amino acid residue to which a linker and/or a payload can bind.

The amino acid residue to which a linker and/or a payload can bind may be an amino acid residue having a functional group to which a linker and/or a payload can bind at a side chain terminus of the amino acid residue or an amino acid residue having a functional group to which a linker and/or a payload can bind at a side chain terminus or a C-terminus thereof in the case of an amino acid residue located on the C-terminal side or an additional amino acid residue. Note that the payload is described later.

In one embodiment, the amino acid residue to which a linker and/or a payload can bind has a structure represented by the following Formula (II): 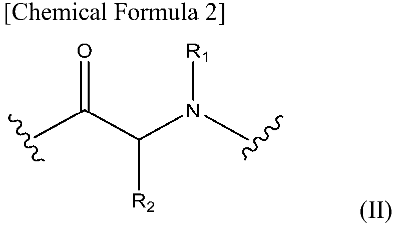
wherein in the formula (II),
R₁ is H or a C₁₋₃ alkyl group;
R₂ is R₂:C₁₋₆ alkyl-NH-R₃, C₁₋₆ alkyl-C₆ aryl-O-C₁₋₃ alkyl-NH-R₃, C₁₋₆ alkyl-NH(=O)-CH(C₁₋₆ alkylphenyl)-NH-R₃, C₁₋₆ alkyl-NH(=O)-CH(C₁₋₆ alkyl)-NH-R₃, or C₁₋₆ alkyl-NH(=O)-C₃₋₈ cycloalkyl-C₁₋₃ alkyl-NH-R₃, and
R₃ is H or any functional group.
R₃ is H or any functional group, including functional groups protected by any protecting group. The structure should be such that a linker and/or a payload can bind to R₃. The structure to which a linker and/or a payload can bind may be, for example, a functional group such as an amide group, or even a known functional group or compound used to attach a compound, such as an alkyne compound or an azide group used in click chemistry. Examples of the "protecting group" include, but are not particularly limited to, a 9-fluorenylmethyloxycarbonyl (Fmoc) group, a tert-butyl (tertBu) group, an allyloxycarbonyl (Alloc) group, and a tert-butoxycarbonyl (Boc) group.

Further, the peptide may form a multimer via, for example, a linker. The number of peptides in the multimer is not limited. In one embodiment, the multimer is a dimer, trimer, tetramer, pentamer, hexamer, octamer, or more. The multimer may contain multiple copies of the same peptide or multiple different peptides.

In one embodiment, the above peptide preferably has CA9 binding activity.

CA9 refers to CA9 (Carbonic anhydrase IX, carbonic anhydrase-9) and is one of the 15 carbonic anhydrases and is a homodimeric transmembrane protein localized on the cell membrane, thereby playing an important role in intracellular pH maintenance, etc. CA9 is known to be expressed under hypoxic conditions and is particularly known to be expressed in specific cancers of the tumor, cervix, uterine body, ovary, kidney, esophagus, lung, breast, brain, gastrointestinal tract, liver, pancreas, head and neck, salivary gland, body cavity, and skin. Therefore, it has been suggested that CA9 is a common marker of tumor hypoxia in many solid tumors.

It is also known that CA9 can be used not only as a marker to evaluate the presence or absence of tumors and their malignancy grades, but also as a marker of poor prognosis in cervical, rectal, breast, lung, and brain tumors.

The term "CA9" or "CA-9" as used herein refers to the carbonic anhydrase-9 possessed by mammals, preferably rodents such as mice and primates such as humans, unless otherwise specified. More preferably, the term refers to human CA9. Note that the term "human CA9" herein refers to the natural-type CA9 (e.g., Gene ID: 768) possessed by humans. Incidentally, it is known that the amino acid sequences of, for example, human, mouse, and rat CA9 are similar sequences.

The CA9 binding activity herein indicates specific binding to CA9, especially human CA9. As used herein, unless otherwise stated, the CA9 binding activity means the activity of binding to human CA9.

The state of binding of the peptide of the present invention to CA9 can be expressed using, without limitation, affinity constant KA, dissociation constant KD, association rate constant kon, and dissociation rate constant koff as indicators.

The affinity constant KA and the dissociation constant KD are indicators of the binding affinity, namely association strength, between two molecules in equilibrium, and the dissociation constant KD is obtained by inverting the affinity constant KA. The smaller the value of the dissociation constant KD, the stronger the binding. Meanwhile, the rate of association/dissociation reaction between two molecules in equilibrium is indicated by the association rate constant kon and the dissociation rate constant koff, which are calculated by the reaction kinetic analysis. Here, KD = koff/kon. Thus, even in the case of equivalent dissociation constants KD, there are two cases: one is a case of slow association but slow dissociation (both kon and koff values are small), and the other is a case of rapid association and rapid dissociation (both kon and koff values are large). Their respective binding retention states are quite different.

The state of binding of the peptide to CA9 can be expressed using these affinity constant KA, dissociation constant KD, association rate constant kon, and dissociation rate constant koff. They can be determined using any intermolecular interaction assay well-known to those skilled in the art.

The state of binding of the peptide to CA9 can be measured by known methods. For example, the state can be measured by surface plasmon resonance spectral analysis (SPR). Examples of the surface plasmon resonance spectral analysis that can be used include, but are not limited to, the BIACORE system (Cytiva), which is a biosensor (biointermolecular interaction analyzer).

One index of the CA9 binding activity of the peptide is the dissociation constant KD. The lower the dissociation constant KD, the higher the binding activity (affinity). According to surface plasmon resonance spectral analysis, the dissociation constant KD for the binding of the peptide to, for example, human CA9 is not limited but is 50 nM or less, 30 nM or less, 20 nM or less, 15 nM or less, 10 nM or less, 8 nM or less, 6 nM or less, 5 nM or less, 4 nM or less, 3 nM or less, 2 nM or less, or 1 nM or less. The lower limit of the dissociation constant KD for the binding of the peptide to human CA9 is not particularly limited. The dissociation constant KD of the peptide is not limited but is 0.01 nM or more, 0.05 nM or more, 0.1 nM or more, 0.2 nM or more, 0.3 nM or more, 0.4 nM or more, or 0.5 nM or more. The dissociation constant KD of the peptide is not limited but is 0.01-50 nM, preferably 0.05-30 nM, more preferably 0.2-15 nM, particularly preferably 0.3-10 nM, and most preferably 0.4-5 nM.

The binding of the peptide to CA9 can also be examined by other known binding ability measuring assay such as ELISA (enzyme-linked immunosorbent assay). ELISA may be performed, for example, by using CA9 beads and the peptide with an HA tag sequence added to the C-terminus to test the binding activity. For example, fluorescence-activated cell sorting (FACS) can also be used.

The binding of the peptide to CA9 is not limited but is preferably non-covalent.

In one embodiment, the peptide has a cyclic structure represented by a peptide portion included in the chemical formula of any one of Examples 1-1 to 1-16 and Examples 11-1 to 11-5.

The peptide with CA9 binding activity is effective in the evaluation, diagnosis, and/or treatment of various CA9-mediated diseases.

In one embodiment, the present invention relates to the above peptide with CA9 binding activity.

In one embodiment, the present invention relates to use of the above peptide for binding to CA9.

In one embodiment, the present invention relates to the above peptide used for binding to CA9.

In the present invention, the binding of the peptide of the present invention to CA9 is binding *in vitro* or *in vivo.*

The matters described in other sections also apply to this section, unless otherwise noted.

### 3. Production of Peptide

The peptide of the present invention can be produced by any known method for peptide production. Examples include the following.

The matters described in other sections also apply to this section, unless otherwise noted.

A chemical synthesis method such as a liquid-phase method, a solid-phase method, and a hybrid method in which liquid-phase and solid-phase methods are combined; and a genetic recombinant method, etc.

In the solid-phase method, for example, the hydroxyl group of a hydroxylated resin is esterified with the carboxy group of the first amino acid, α-amino group of which is protected by a protecting group (usually the C-terminal amino acid of the peptide of interest). The esterification catalyst used may be a known dehydration-condensation agent such as 1-mesitylene sulfonyl-3-nitro-1,2,4-triazole (MSNT), dicyclohexylcarbodiimide (DCC), or diisopropylcarbodiimide (DIC).

Next, the protecting group of the α-amino group of the first amino acid is eliminated and a second amino acid in which all functional groups other than the carboxy group of the main chain are protected is added to activate the carboxy group, and the first and second amino acids are coupled. Further, the α-amino group of the second amino acid is deprotected and a third amino acid in which all functional groups other than the carboxy group of the main chain are protected is added to activate the carboxy group, and the second and third amino acids are coupled. This procedure is repeated until a peptide of the desired length is synthesized, and all the functional groups are then deprotected.

Examples of the resin for solid-phase synthesis include Merrifield resin, MBHA resin, Cl-Trt resin, SASRIN resin, Wang resin, Rink amide resin, HMFS resin, Amino-PEGA resin (Merck), and HMPA-PEGA resin (Merck). These resins may be washed with a solvent (e.g., dimethylformamide (DMF), 2-propanol, methylene chloride) before use.

Examples of the protecting group for α-amino group include a benzyloxycarbonyl (Cbz or Z) group, a tert-butoxycarbonyl (Boc) group, a 9-fluorenylmethyloxycarbonyl (Fmoc) group, a benzyl group, an allyl group, and an allyloxycarbonyl (Alloc) group. The Cbz group may be deprotected, for example, with hydrofluoric acid or by hydrogenation, the Boc group may be deprotected with trifluoroacetic acid (TFA), and the Fmoc group may be deprotected by treatment with piperidine or pyrrolidine.

The α-carboxy group may be protected using, for example, methyl ester, ethyl ester, allyl ester, benzyl ester, *tert*-butyl ester, or cyclohexyl ester.

The carboxy group may be activated using a condensation agent. Examples of the condensation agent include dicyclohexylcarbodiimide (DCC), diisopropylcarbodiimide (DIC), 1-ethyl-3-(3-dimethylaminopropyl)carbodiimide (EDC or WSC), (1H-benzotriazol-1-yloxy)tris(dimethylamino)phosphonium hexafluorophosphate (BOP), and 1-[bis(dimethylamino)methyl]-1H-benzotriazolium-3-oxide hexafluorophosphate (HBTU).

The peptide chain may be cleaved from the resin by treatment with an acid such as TFA or hydrogen fluoride (HF).

The peptide may be produced by genetic recombination method (translationsynthesis system) while using a nucleic acid encoding the peptide described above. The nucleic acid encoding the peptide may be DNA or RNA.

The nucleic acid encoding the peptide may be prepared by a known method or an equivalent method. For example, the nucleic acid can be synthesized with an automatic synthesizer. The resulting DNA may contain a restriction enzyme recognition site(s) for vector insertion. Alternatively, a nucleotide sequence encoding an amino acid sequence may be incorporated, so that the resulting peptide chain can be cut out by enzyme or other means.

To inhibit degradation by host-derived proteases, a chimeric protein expression method, in which a target peptide is expressed as a chimeric peptide with other peptide, can also be used. In this case, it is possible to use, as the above nucleic acid, a nucleic acid encoding the target peptide and a peptide fused to the target peptide.

Then, an expression vector is prepared using the nucleic acid encoding the peptides. The nucleic acid can be incorporated downstream of the promoter of the expression vector either as is or by digestion with restriction enzyme(s) or by adding a linker, etc. Examples of the vector include: an E. coli-derived plasmid (e.g., pBR322, pBR325, pUC12, pUC13, pUC18, pUC19, pUC118, pBluescript II); a *Bacillus subtilis*-derived plasmid (e.g., pUB110, pTP5, pC1912, pTP4, pE194, pC194); a yeast-derived plasmid (e.g., pSH19, pSH15, YEp, YRp, YIp, YAC); a bacteriophage (e.g., e-phage, M13 phage); a virus (e.g., retrovirus, vaccinia virus, adenovirus, adeno-associated virus (AAV), cauliflower mosaic virus, tobacco mosaic virus, baculovirus); or a cosmid, etc.

The promoter can be selected, if appropriate, according to the type of host. If the host is an animal cell, for example, an SV40 (simian virus 40)-derived promoter or a CMV (cytomegalovirus)-derived promoter can be used. If the host is *E. coli,* the trp promoter, T7 promoter, or lac promoter, for example, can be used.

The expression vector may have, for example, a DNA replication origin (ori), a selection marker (e.g., antibiotic resistance, nutrient requirement), an enhancer, a splicing signal, a poly-A addition signal, and/or a tag (e.g., FLAG, HA, GST, GFP)-encoding nucleic acid.

Next, an appropriate host cell is transformed with the expression vector. The host may be selected, if appropriate, in view of the relation to the vector. Examples of the host used include *E. coli, Bacillus subtilis,* a bacterium belonging to the genus *Bacillus,* yeast, an insect or insect cell, and an animal cell, etc. Examples of the animal cell used include an HEK293T cell, a CHO cell, a COS cell, a myeloma cell, a HeLa cell, and a Vero cell. The transformation may be performed according to the type of host by a known method such as lipofection, a calcium phosphate method, electroporation, microinjection, particle gun, or other known methods. The transformants are cultured according to a routine procedure to express a peptide of interest.

The peptide is purified from the transformant culture by collecting the cultured cells, by suspending the cells in an appropriate buffer solution, by, for instance, sonication or freeze-thawing to destroy the cells, and then by centrifugation or filtration to obtain a crude extract. If the peptide is secreted into the culture solution, the supernatant is collected.

Purification from the crude extract or culture supernatant can also be performed by a known method or an equivalent method (e.g., chlorination, dialysis, ultrafiltration, gel filtration, SDS-PAGE, ion exchange chromatography, affinity chromatography, reversephase high-performance liquid chromatography).

A known method or an equivalent method may be used to convert the resulting peptide in a free form to a salt, or the peptide in a salt form to a free form.

In one embodiment, the translation synthesis system may be a cell-free translation system. The cell-free translation system generally allows an expressed product to be obtained in a highly pure form without purification. The cell-free translation system contains, for example, ribosomal proteins, aminoacyl-tRNA synthetase (ARS), ribosomal RNA, amino acids, rRNA, GTP, ATP, translation initiation factor (IF), elongation factor (EF), termination factor (RF), and ribosome regeneration factor (RRF), and other factors necessary for translation. *E. coli* extract or wheat germ extract may be added to increase expression efficiency. In addition, rabbit red blood cell extract or insect cell extract may also be added.

Energy may be continuously supplied to the system containing them by using dialysis to produce, in a non-limiting manner, several hundred µg to several mg/mL of the protein. The system may also include RNA polymerase for transcription from the gene DNA. As a commercially available cell-free translation system that can be used, examples of the E. coli-derived system include Roche Diagnostics' RTS-100 (registered trademark), Gene Frontier's PURESYSTEM, or NEW ENGLAND Biolabs PURExpress *In Vitro* Protein Synthesis Kit; and examples of the system using wheat germ extract include those from ZOEGENE Corporation and CellFree Sciences.

In the cellular translation system, instead of aminoacyl-tRNA synthesized by natural aminoacyl-tRNA synthetase, an artificial aminoacyl-tRNA in which the desired amino acid or hydroxy acid is linked (acylated) to the tRNA may be used. Such aminoacyl-tRNA can be synthesized using an artificial ribozyme.

Examples of such a ribozyme include flexizyme (H. Murakami, H. Saito, and H. Suga, (2003), Chemistry & Biology, Vol.10, 655-662; and WO 2007/066627, etc.). The flexizyme is also known as and called the original Flexizyme (Fx) or a modified form such as dinitrobenzylflexizyme (dFx), enhanced Flexizyme (eFx), or aminoflexizyme (aFx).

By using the tRNA, generated by flexizyme, to which the desired amino acid or hydroxy acid is linked, the desired codon can be associated with the desired amino acid or hydroxy acid for translation. A special amino acid may be used as the desired amino acid. For example, the non-natural amino acid required for the cyclization described above can also be introduced into the peptide linked by this method.

The peptide may be chemically synthesized using various methods routinely used in the art. Examples of the method include stepwise solid-phase synthesis, semi-synthesis of peptide fragment via conformationally assisted re-ligation, and chemical ligation. The synthesis of the peptide is a chemical synthesis using various solid-phase techniques as described, for example, in K. J. Jensen, P. T. Shelton, S. L. Pedersen, Peptide Synthesis and Applications, 2nd Edition, Springer, 2013. The preferred strategy is based on a combination of a Fmoc group that temporarily protects the α-amino group and allows for selective removal by a base, and a protecting group that temporarily protects the side chain functional group and is stable under de-Fmoc conditions. The selection of such general peptide side chain is described in the above Peptide Synthesis and Applications, 2nd edition and G. B. Fields, R. L. Noble, Solid Phase Peptide Synthesis Utilizing 9-Fluorenylmethoxycarbonyl Amino Acids, Int. J. Peptide Protein Res. 35, 1990, 161-214, etc. Examples of the preferred peptide side chain protecting group include: a benzyl, tert-butyl, or trityl (Trt) group for the hydroxy group of serine or threonine; a 2-bromobenzyloxycarbonyl or *tert*-butyl group for the hydroxy group of tyrosine; a Boc, methyltetrazole thiol (Mtt), Alloc, or ivDde group for the amino group of the lysine side chain; a Trt or Boc group for the imidazole group of histidine; a 2,2,4,6,7-pentamethyldihydrobenzofuran-5-sulfonyl (Pbf) group for the guanidyl group of arginine; a tert-butyl, allyl, or 3-methylpentane (Mpe) group for the carboxyl group of, for instance, glutamic acid or aspartic acid; a Trt group for the carboxamide group of glutamine or asparagine; and a Trt or monomethoxytrityl (Mmt) group for the thiol group of cysteine.

The peptide can be synthesized in a stepwise manner on the solid-phase resin described above. The α-amino protecting groups of the C-terminal amino acid used and all amino acids and the peptide used in the synthesis should be selectively removed in the synthetic process. Preferably, the above-described solid-phase resin is used to initiate the process. The C-terminal carboxyl group of the peptide with the N-terminus appropriately protected with a Fmoc group, etc. or the C-terminal carboxyl group of a Fmoc groupprotected amino acid is made into an activated ester with an appropriate reagent and then attached to the amino group on the solid-phase resin. Subsequent elongation of the peptide chain can be achieved by sequentially repeating the removal of the N-terminal protecting group (Fmoc group), followed by condensation with a protected amino acid derivative, according to the amino acid sequence of the peptide of interest. Note that the peptide of interest can be released at the final stage. For example, as the release conditions, TFA solution containing water/silylhydride/thiol as a scavenger in TFA, as described in, for instance, Teixeira, W. E. Benckhuijsen, P. E.de Koning, A. R. P. M. Valentijn, J. W. Drijfhout, Protein Pept. Lett. 2002, 9, 379-385, can be used for the release. A typical example is TFA/Water/TIS/DODT (volume ratio 92.5:2.5:2.5:2.5).

The peptide described herein may be synthesized using a single or multichannel peptide synthesizer, such as CEM's Liberty Blue synthesizer or Biotage's Syro I synthesizer or their successors.

The carboxy group may be activated using a condensation agent. Examples of the condensation agent include dicyclohexylcarbodiimide (DCC), diisopropylcarbodiimide (DIPCDI), 1-ethyl-3-(3-dimethylaminopropyl)carbodiimide (EDC or WSC), (1H-benzotriazol-1-yloxy)tris(dimethylamino)phosphonium hexafluorophosphate (BOP), and 1-[bis(dimethylamino)methyl]-1H-benzotriazolium-3-oxide hexafluorophosphate (HBTU).

The peptide can be cyclized according to a known method. For example, the peptide may be designed to contain two or more cysteine residues, so that a cyclic structure can be formed through a disulfide bond after translation, but is not limited thereto. In addition, Goto's method (Y. Goto, et al., ACS Chem. Biol. 3 120-129 (2008)) may be used to synthesize a peptide with a chloroacetyl group at the N-terminus by genetic code reprogramming technique. In the peptide, a cysteine residue containing a sulfur molecule may be placed for cyclization. This causes the spontaneous nucleophilic attack of the mercapto group on the chloroacetyl group after translation, and the peptide is cyclized through a thioether bond. Other combination of amino acids that are bonded to form a ring may be placed, for cyclization, within the peptide by genetic code reprogramming technique. Alternatively, the peptide may be cyclized by placing an L-2-amino adipic acid residue in the peptide and bonding it to the N-terminal main chain amino group. Alternatively, the peptide may be cyclized by bonding, through an amide bond, an amide group of an amino acid residue on the N-terminal side to a carboxy group of an amino acid residue on the C-terminal side. In addition, the peptide may be cyclized by bonding an amino group of an N-terminal amino acid (amino acid residue at position 1) to a functional group present at a side chain terminus of a C-terminal amino acid included in the peptide. In this way, any known cyclization method can be used without restriction.

The matters described in other sections also apply to this section, unless otherwise noted.

### 4. Conjugate (Complex)

The present invention relates to a complex containing a peptide and a payload (also sometimes called a "peptide conjugate", a "peptide complex", or simply a "conjugate"), and a pharmaceutically acceptable salt thereof. The peptide in the conjugate includes all forms (including salts, isomers, solvates, etc.) as described in the section "2. Peptide". References herein to "peptide conjugate", "conjugate", or "peptide complex" also include references to a pharmaceutically acceptable salt thereof, an isomer, or a solvate thereof, as long as there is no particular contradiction.

In one embodiment, the conjugate of the present invention represents a complex in which the peptide of the present invention and a payload are attached with or without a linker.

In one embodiment, in the conjugate of the present invention, a side chain of an amino acid residue in the peptide of the present invention is attached, with or without a linker, to a payload.

In one embodiment, in the conjugate of the present invention, the amino acid residue at position 13 in SEQ ID No. 1 is attached, with or without a linker, to any payload.

In one embodiment, in the conjugate of the present invention, the amino acid residue at position 1, 4, 8, or 11 in SEQ ID No. 1 is attached to a payload as shown in the following formula (I): 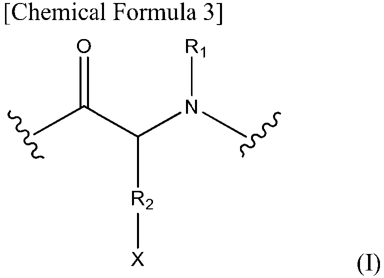
wherein in the formula (I),
R₁ is H or a C₁₋₃ alkyl group;
R₂ is C₁₋₆ alkyl-NH-, C₁₋₆ alkyl-C₆ aryl-O-C₁₋₃ alkyl-NH-, C₁₋₆ alkyl-NH(=O)-CH(C₁₋₆ alkylphenyl)-NH-, C₁₋₆ alkyl-NH(=O)-CH(C₁₋₆ alkyl)-NH-, or C₁₋₆ alkyl-NH(=O)-C₃₋₈ cycloalkyl-C₁₋₃ alkyl-NH-; and
X is any payload.

Note that regarding R₂, C₁₋₆ alkyl-NH- is a residue in a modified form in which the alkyl chain is extended in the residue of K and the side chain of K; C₁₋₆ alkyl-C₆ aryl-O-C₁₋₃ alkyl-NH- is a residue in a modified form in which the alkyl chain is extended in the residue of dyae and the side chain of dyae; C₁₋₆ alkyl-NH(=O)-CH(C₁₋₆ alkylphenyl)-NH- is a residue in a compound formed by bonding a modified form, in which the alkyl chain is extended in the residue of dk-df and the side chain of dk, to a modified form, in which the alkyl chain is extended in the side chain of df; C₁₋₆ alkyl-NH(=O)-CH(C₁₋₆ alkyl)-NH- is a residue in a compound formed by bonding a modified form, in which the alkyl chain is extended in the residue of dk-dnle and the side chain of dk, to a modified form, in which the alkyl chain is extended or shortened in the side chain of dnle; and C₁₋₆ alkyl-NH(=O)-C₃₋₈ cycloalkyl-C₁₋₃ alkyl-NH- is a residue in a compound formed by bonding a modified form, in which the alkyl group is extended in the residue of dk-t4amCh and the side chain of dk, to a modified form in which the number of carbon atoms contained in the cycloalkyl structure of the side chain of t4amCh is increased or decreased.

Note that 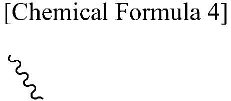 in the compound (I) indicates that the carboxy or amide group is bonded to an amide or carboxy group of an adjacent amino acid residue in the amino acid sequence.

The C₁₋₃ alkyl herein means a monovalent group derived from a linear and branched saturated C₁₋₃ aliphatic hydrocarbon group by removing one optional hydrogen atom. Specific examples include methyl, ethyl, n-propyl, and isopropyl.

The C₁₋₆ alkyl herein means a monovalent group derived from a linear and branched saturated C₁₋₆ aliphatic hydrocarbon by removing one optional hydrogen atom. Specific examples include methyl, ethyl, n-propyl, isopropyl, n-butyl, isobutyl, sec-butyl, t-butyl, 1-methylpropyl, n -pentyl, isopentyl, 2-methylbutyl, 1,1-dimethylpropyl, 1-ethylpropyl, hexyl, and 4-methylpentyl.

The C₁₋₆ alkylphenyl herein means a structure obtained by attaching a phenyl group to a monovalent group derived from a linear and branched saturated C₁₋₆ aliphatic hydrocarbon by removing one optional hydrogen atom.

The C₃₋₈ cycloalkyl herein means a monovalent group derived from a cyclic saturated C₃₋₈ aliphatic hydrocarbon by removing one optional hydrogen atom.

Note that the term "conjugate" of the present invention includes, but is not limited to, the conjugates represented by Conjugate No. 1-81, such as Conjugates No. 1-69 and 74-81, and also includes conjugates in which the payloads in those conjugates are replaced by different payloads. The conjugate replaced by a different payload may be, for example, a conjugate in which the payload (DOTA, DOTALu, SulfoCy5, DOTAZr, DOTALu-df, DOTALu-dnle, DOTALu-t4amCh, DOTALu-F, DOTALu-Nle, SulfoCy5-PEG8c, DOTACu, DOTAGA, dDOTAGA, rNOTAGA, DOTA-#Ga (Ga (gallium) bound to DOTA), and DOTALa (La (lanthanum) bound to DOTA)) of Conjugate Nos. 1-81 shown in Table 8 or Table 18 (in one embodiment, Conjugate Nos. 1-69 and 74-81) is replaced by other payload.

The conjugate described as Conjugate No. 1 may, for example, be modified such that the payload sulfoCy5 attached to the linker G-PEG10c-K is replaced by a chelator such as DOTA, or in the conjugate, a radioactive element is further attached to the chelator such as DOTA. Alternatively, in the conjugate, the sulfoCy5 may be attached to a payload other than a radioisotope, such as an antibody. In addition, for example, in the conjugate described as Conjugate No. 2, the payload, namely a chelator DOTA attached via the linker K, may be further attached to a radioisotope. For instance, in the conjugate described as Conjugate No. 4, the payload, namely a chelator DOTA attached via the linker K, may be attached to ¹⁷⁷Lu, which is a radioisotope, in place of Lu (non-radioactive lutetium).

### Payload

In the present invention, the payload refers to a known functional molecule. The functional molecule is a molecule that exhibits a desired function when it binds to or acts on a target, without any particular restriction. Examples of the desired function include, in one embodiment, a pharmacologically active function, a labeling function, and a target site delivery function. Note that examples of the "payload" herein include a pharmacologically active compound, a labeled compound, a fluorescent substance, a peptide, a protein, a nucleic acid, and a molecule used in a drug delivery system, and the molecule may be a low-molecular-weight compound, a medium-molecular-weight compound, and a high-molecular-weight compound.

The pharmacologically active compound herein refers to a compound having pharmacological activity. The pharmacologically active compound is preferably, for example, a high-molecular-weight compound (e.g., an antibody, a protein, a nucleic acid), a medium-molecular weight compound (e.g., a peptide), and a low-molecular-weight compound that have pharmacological activity. In addition, the compound may be provided such that a low-molecular-weight compound having pharmacological activity, etc. is included in a liposome or micelle, etc.

Also, the "payload" herein includes any cytotoxic agent that is toxic to cells, and in particular, kills cells. Examples include, without limitation, taxol, cytochalasin B, gramicidin D, ethidium bromide, emetine, mitomycin, etoposide, teniposide, vincristine, vinblastine, colchicine, doxorubicin, daunorubicin, dihydroxyanthracenedione, mitoxantrone, mithramycin, actinomycin D, 1-dihydrotestosterone, glucocorticoid, procaine, tetracaine, lidocaine, propranolol, and puromycin, as well as analogs or homologs thereof. In addition, the term "payload" herein also includes therapeutic agents having anticancer activity, including, but not limited to, antimetabolites (e.g., methotrexate, 6-mercaptopurine, 6-thioguanine, cytarabine, fludarabine, 5-fluorouracil, decarbazine); alkylating agents (e.g., mechlorethamine, thiotepa, chlorambucil, melphalan, carmustine (BSNU) and lomustine (CCNU), cyclophosphamide, busulfan, dibromomannitol, streptozotocin, mitomycin C, *cis-*dichlorodiammineplatinum(II) (DDP), namely cisplatin); anthracyclines (e.g., daunorubicin (formerly referred to as "daunomycin"), doxorubicin); antibiotics (e.g., dactinomycin (formerly referred to as "actinomycin"), bleomycin, mithramycin, anthramycin (AMC)); and antimitotic agents (e.g., vincristine, vinblastine). In one preferred embodiment, the therapeutic agent is a cytotoxic agent. In other embodiment, the therapeutic agent is an immunosuppressive agent. In still other embodiment, the therapeutic agent is GM-CSF. In one preferred embodiment, the therapeutic agent is doxorubicin, cisplatin, bleomycin, sulfate, carmustine, chlorambucil, cyclophosphamide, or ricin A.

The labeled compound herein refers to a compound labeled with a dye, a fluorescent substance, a tag, or a radioisotope. Among them, the radioisotope-labeled compound refers to a compound (e.g., a low-molecular weight, medium-molecular weight compound or antibody) labeled with a radioisotope. The labeled compound may be a dye, a fluorescent substance, a tag, or a radioisotope itself. The radioisotope may be coordinated with a chelator such as DOTA.

The radioisotope herein may be any known radioisotope. In one embodiment, examples of the radioisotope includes, but are not limited to, actinium-225, actinium-227 (²²⁵Ac, ¹¹⁷Ac), astatine-211 (²¹¹At), bismuth-212 and bismuth-213 (²¹²Bi, ²¹³Bi), copper-61, copper-62, copper-64 and copper-67 (⁶¹Cu, ⁶²Cu, ⁶⁴Cu, ⁶⁷Cu), gallium-64, gallium-67 and gallium-68 (⁶⁴Ga, ⁶⁷Ga and ⁶⁸Ga), indium-111 (¹¹¹In), iodine-123, iodine-124, iodine-125 or iodine-131 (¹²³I, ¹²⁴I, ¹²⁵I, ¹³¹I), lead-203, lead-212 (²⁰3Pb, ²¹²Pb), ruthenium-97 (⁹⁷Ru), lutetium-177 (¹⁷⁷Lu), radium-223 (²²³Ra), samarium-153 (¹⁵³Sm), scandium-44 and scandium-47 (⁴⁴Sc, ⁴⁷Sc), iron-52 (⁵²Fe), arsenic-72, arsenic-76 (⁷²As, ⁷⁶As), erbium-169 (¹⁶⁹Er), strontium-89, strontium-90 (⁸⁹Sr, ⁹⁰Sr), technetium-99, technetium-94 (⁹⁹mTc, ⁹⁴mTc), yttrium-86 and yttrium-90 (⁸⁶Y, ⁹⁰Y), chromium-51 (⁵¹Cr), rhenium-186, rhenium-188 (¹⁸⁶Re, ¹⁸⁸Re), zirconium-89 (⁸⁹Zr), manganese-52, manganese-51 (⁵²Mn, ⁵¹Mn), terbium-149, terbium-152, terbium-155, terbium-161 (¹⁴⁹Tb, ¹⁵²Tb, ¹⁵⁵Tb, ¹⁶¹Tb), ytterbium-169, ytterbium-175 (¹⁶⁹Yb, ¹⁷⁵Yb), rhodium-105 (¹⁰⁵Rh), dysprosium-166 (¹⁶⁶Dy), holmium-166 (¹⁶⁶Ho), samarium-153 (¹⁵³Sm), promethium-149 (¹⁴⁹Pm), promethium-151 (¹⁵¹Pm), thulium-172 (¹⁷²Tm), tin-121 (¹²¹Sn), praseodymium-142, praseodymium-143 (¹⁴²Pr, ¹⁴³Pr), gold-198, gold-199 (¹⁹⁸Au, ¹⁹⁹Au), bromine-75, bromine-76, bromine-77, bromine-80, bromine-82 (⁷⁵Br, ⁷⁶Br, ⁷⁷Br, ⁸⁰Br, ⁸²Br), fluorine-18 (¹⁸F), scandium-43, scandium-44, scandium-47 (⁴³Sc, ⁴⁴Sc, ⁴⁷Sc), astatine-211 (²¹¹At), thorium-227, thorium-226 (²²⁷Th, ²²⁶Th), lanthanum-140 (¹⁴⁰La), rubidium-82 (⁸²Rb), phosphorus-32 (³²P), silver-111 (¹¹¹Ag), and erbium-165 (¹⁶⁵Er).

As used herein, the term "chelator" refers to an organic compound that binds to a metal ion to form a cyclic complex structure in order to stably bind to a radioisotope, particularly a metal radioisotope. In one embodiment, examples of the chelator include EDTA (ethylenediaminetetraacetic acid), DPTA (diethylenetriaminepentaacetic acid), 1,4,8,11-tetraazatetradecane, 1,4,8,11-tetraazatetradecane-1,4,8,11-tetraacetic acid, 1-oxa-4,7,12,15-tetraazaheptadecane-4,7,12,15-tetraacetic acid, DOTAGA (α-(2-carboxyethyl)-1,4,7,10-tetraazacyclododecane-1,4,7,10-tetraacetic acid) (including the racemic form rDOTAGA), TMT (6,6"-bis[N,N",N"'-tetra(carboxymethyl)aminomethyl]-4'-(3-amino-4-methoxyphenyl)-2,2':6',2"-terpyridine), DOTA (1,4,7,10-tetraazacyclododecane-N,N',N",N‴-tetraacetic acid), TCMC (tetra-primary amide derivative of DOTA), DO3A (1,4,7,10-tetraazacyclododecane-1,4,7-tris(acetic acid)-10-(2-thioethyl)acetamide), CB-DO2A (4,10-bis(carboxymethyl)-1,4,7,10-tetraazabicyclo[5.5.2]tetradecane), NOTA (1,4,7-triazacyclononane-triacetic acid), Diamsar (3,6,10,13,16,19-hexaazabicyclo[6.6.6]eicosane-1,8-diamine), DTPA (pentetic acid or diethylenetriaminepentaacetic acid), CHX-A"-DTPA ([(R)-2-amino-3-(4-isothiocyanatophenyl)propyl]-trans-(S,S)-cyclohexane-1,2-diamine-pentaacetic acid), TETA (1,4,8,11-tetraazacyclotetradecane-1,4,8,11-tetraacetic acid), Te2A (4,11-bis(carboxymethyl)-1,4,8,11-tetraazabicyclo[6.6.2]hexadecane), HBED, DFO (deferoxamine), DFOsq (DFO-squaramide), HOPO (3,4,3-(LI-1,2-HOPO)), and NODAGA (1,4,7-triazacyclononane-1-1glutamic acid-4,7-acetic acid) (including the racemic form rNODAGA), and modified forms thereof. Preferred is DOTA, DOTAGA, or NODAGA. If optical isomers exist in each chelator, they are included.

In one embodiment, the payload is preferably a radioisotope coordinated with a chelator. For example, without limitation, structures in which a radioactive isotope of lutetium, zirconium, copper, or gallium is coordinated with DOTA may be included.

In one embodiment, the payload may be a radioisotope not coordinated with a chelator. The compound may be one used for evaluation methods or imaging using a radioisotope. For example, without limitation, it may be a known compound containing a radioisotope, which compound is used as a radioligand imaging agent in positron emission tomography (e.g., PET, SPECT), as described below. For example, without limitation, the compound may be ¹⁸F-fluorodeoxyglucose (¹⁸F-FDG), ¹⁵O-H₂O, ¹¹C-methionine, ¹¹C-acetic acid, ¹¹C-choline, ¹¹C-raclopride, ¹¹C-flumazenil, ¹³N-ammonia, ¹⁸F-sodium fluoride, ⁸²Rb+ (⁸²RbCl), ¹⁸F-flurpiridaz, ¹²³I-IMP, ⁹⁹mTc-ECD, ⁹⁹Tc-HMPAO, ¹²³I-iomazenil, ¹²³I-ioflupane, ¹¹¹In-DTPA, ⁹⁹mTcO4- (Na⁹⁹mTcO4), ⁹⁹mTc-MIBI, ²⁰¹Tl+ (²⁰1TlCl), ⁹⁹mcTc-tetrofosmin, ⁹⁹mcTc-human serum albumin, ¹³¹O-adosterol, ⁹⁹mTc-phytate, ⁹⁰Y-zevalin, ⁶⁷Ga+ (⁶⁷GaC₆H₅O₇), ¹¹¹In-ibritumomab tiuxetan, ¹¹¹In-pentetreotide, or the like.

As used herein, the term "protein" refers to any protein that exists *in vivo* or exhibits a useful function *in vivo.* Examples include proteins having pharmacological activity and proteins having molecular recognition activity. Specifically, examples include fibronectin, avidin, antibodies, proteins having an Fc region, immune checkpoint proteins, and enzymes such as proteolytic enzymes.

As used herein, the term "nucleic acid" refers to any polymer of nucleotides. Specific examples include DNA and/or RNA. As used herein, the nucleic acid may be modified. Examples of such modifications include 2'-methoxyethyl (MOE) modification, use of 2'-deoxynucleosides in RNA, internucleoside linkages via phosphorothioate bonds, internucleoside linkages via phosphodiester bonds, and conversion of cytosine to 5-methylcytosine.

As used herein, the term "drug delivery system" refers to a system to deliver an active ingredient into a target cell. Examples include water-soluble polymers, nano-sized particles (nanospheres), and carriers such as liposomes and micelles. The drug delivery system (DDS) molecule may contain an additional medicament such as a low-molecular-weight compound, a protein, a peptide, a nucleic acid, and/or a vaccine.

In one embodiment, the present invention relates to the above conjugate with CA9 binding activity.

In one embodiment, the present invention relates to use of the above conjugate for binding to CA9.

In one embodiment, the present invention relates to the above conjugate used to bind to CA9.

In the present invention, the binding of the conjugate of the present invention to CA9 is binding *in vitro* or *in vivo.*

The matters described in other sections also apply to this section, unless otherwise noted.

### 5. Composition, etc.

The present invention also relates to a composition containing a peptide or conjugate of the present invention or a pharmaceutically acceptable salt thereof. The composition is not limited, and may be a pharmaceutical composition (a medical composition), a diagnostic composition, or a research composition. In one embodiment, the pharmaceutical composition is a composition used in radiotherapy (an embodiment of therapeutic composition).

The present invention also relates to an imaging agent containing the conjugate of the present invention. The imaging agent can also be used as a diagnostic composition. In one embodiment, the diagnostic composition is an imaging agent.

The matters described in other sections also apply to this section, unless otherwise noted.

### Pharmaceutical composition

In one embodiment, the present invention relates to a pharmaceutical composition containing the above peptide or conjugate, or a pharmaceutically acceptable salt thereof.

In one embodiment, the above pharmaceutical composition has CA9 binding activity.

In one embodiment, the pharmaceutical composition is a pharmaceutical composition for preventing or treating a CA9-associated disease or symptom.

The term "CA9-associated disease or symptom" includes known CA9-associated diseases or symptoms. Examples include tumors, especially malignant tumors and solid tumors.

In one embodiment, examples of the CA9-associated disease include tumors of the cervix and uterus, breast, ovary, digestive tract (including esophagus, stomach, small intestine, colon, and rectum), kidney, liver, gallbladder and bile duct system, pancreas (including pancreatic duct and exocrine pancreas), lung, head and neck, salivary glands, bladder and urinary tract, body cavity, epithelial origin, etc. The examples also include cancers of the head and neck, ovary, fallopian tube, peritoneum, vagina, vulva, penis, uterine cervix, myometrium, endometrium, thyroid gland, adrenal gland, prostate, skin, and appendages thereof; hematologic malignancies; borderline malignancies including premalignant hematologic disorders and lymphoid lineage hematologic malignancies and related diseases; myeloid lineage hematologic malignancies and related diseases; tumors of mesenchymal origin; tumors of the central or peripheral nervous system; endocrine tumors; tumors of the eye and its appendages; germ cell and trophoblastic tumors; and pediatric and embryonal tumors. Preferably, the cancer includes, without limitation, renal cell carcinoma, head and neck cancer, urothelial carcinoma, pancreatic ductal carcinoma, and colon cancer, etc.

In one embodiment, the pharmaceutical composition is a composition used in radiotherapy (an embodiment of therapeutic composition). In this case, a component in the pharmaceutical composition contains a radioisotope. Preferably, it is a conjugate containing a radioisotope as a payload. Note that the radiotherapy is a treatment method in which irradiation with radiation or administration of a radiation-emitting substance to the affected area damages DNA and other substances in the cells of a tumor, especially cancer, and kills the cells.

The pharmaceutical composition may contain the peptide or conjugate itself, and may contain a pharmaceutically acceptable salt of the peptide, or an isomer or a solvate thereof. The "peptide" and "conjugate" herein may include a pharmaceutically acceptable salt thereof, or an isomer or a solvate thereof, unless otherwise specified. The pharmaceutical composition preferably contains an effective amount of the peptide or conjugate as an active ingredient.

The dosage form of the pharmaceutical composition herein is not particularly limited and may be administered orally or parenterally. Examples of the parenteral administration include an injection (e.g., an intramuscular, intravenous, or subcutaneous injection) and transdermal or transmucosal (nasal, oral, ocular, transpulmonary, transvaginal, rectal) administration.

The pharmaceutical composition may be modified in various ways in view of the easy-to-metabolize or easy-to-excrete characteristics of the polypeptide. For example, polyethylene glycol (PEG) or sugar chain may be added to the polypeptide to increase the residence time in blood and decrease the antigenicity. In addition, for example, a biodegradable polymer compound (e.g., polylactic acid glycol (PLGA)), porous hydroxyapatite, liposome, surface-modified liposome, an emulsion prepared with unsaturated fatty acid, nanoparticles, or nanospheres may be used as a sustained release base material, and the polypeptide can be encapsulated in them. For transdermal administration, a weak electric current can be applied to the skin surface for stratum corneum permeation (iontophoresis method).

The pharmaceutical composition may be made into a preparation by using the active ingredient as it is, or by adding a pharmaceutically acceptable carrier, excipient, and/or additive. etc. Examples of the dosage form include liquid (e.g., an injection), dispersion, suspension, tablets, pills, powder, suppositories, pulvis, fine particles, granules, capsules, syrup, lozenges, inhalant, ointment, eye drops, nose drops, ear drops, and cataplasm. The preparation can be made by a routine procedure using, for example, an excipient, a binder, a disintegrant, a lubricant, a dissolving agent, a dissolution aid, a colorant, an odor correcting agent, a stabilizer, an emulsifier, an absorption enhancer, a surfactant, a pH modifier, a preservative, and/or an antioxidant as appropriate.

Examples of the components used for formulation include, but are not limited to, purified water, saline, phosphate buffer, dextrose, glycerol, a pharmaceutically acceptable organic solvent (e.g., ethanol), animal or vegetable oil, lactose, mannitol, glucose, sorbitol, crystalline cellulose, hydroxypropyl cellulose, starch, corn starch, anhydrous silica, magnesium aluminum silicate, collagen, polyvinyl alcohol, polyvinyl pyrrolidone, carboxy vinyl polymer, sodium carboxymethylcellulose, sodium polyacrylate, sodium alginate, water-soluble dextran, sodium carboxymethyl starch, pectin, methylcellulose, ethylcellulose, xanthan gum, gum arabic, tragacanth, casein, agar, polyethylene glycol, diglycerin, glycerin, propylene glycol, vaseline, paraffin, octyldodecyl myristate, isopropyl myristate, higher alcohols, stearyl alcohol, stearic acid, and human serum albumin.

In view of the fact that peptides are generally difficult to be absorbed transmucosally, the pharmaceutical composition may contain an absorption enhancer for improving absorption of a poorly absorbable agent. Examples of such an absorption enhancer include: a surfactant (e.g., a polyoxyethylene lauryl ether compound, sodium lauryl sulfate, saponin); a salt of bile acid (e.g., glycolic acid, deoxycholic acid, taurocholic acid); a chelator (e.g., EDTA, salicylic acid); a fatty acid compound (e.g., caproic acid, capric acid, lauric acid, oleic acid, linoleic acid, a mixed micelle); or an enamine derivative, an N-acyl collagen peptide, N-acylamino acid, a cyclodextrin compound, a chitosan compound, and a nitric oxide donor.

If the pharmaceutical composition is a pill(s) or a tablet(s), it may be coated with a sugar coating or a gastric-soluble or enteric-soluble substance.

If the pharmaceutical composition is an injection, it may contain distilled water for injection, physiological saline, propylene glycol, polyethylene glycol, vegetable oil, and/or an alcohol compound, etc. In addition, it is possible to add a wetting agent, an emulsifier, a dispersant, a stabilizer, a dissolving agent, a dissolution aid, and/or a preservative, etc.

The pharmaceutical composition may be targeted not only to humans but also to non-human mammals or birds. Examples of the non-human mammals include non-human primates (e.g., monkeys, chimpanzees, gorillas), domestic animals (e.g., pigs, cows, horses, sheep), or dogs, cats, rats, mice, guinea pigs, and rabbits, etc.

In particular, the dose when administered to a human varies depending on the symptoms, age, sex, body weight, and sensitivity of the patient as well as the administration method, the administration interval, the type of active ingredient, and the type of preparation. The dose is not limited and the dose of, for example, from 30 µg to 100 g, from 100 µg to 500 mg, or from 100 µg to 100 mg can be administered in one or several separate portions. For injection, 1 µg/kg to 3000 µg/kg or 3 µg/kg to 1000 µg/kg may be administered once or in several separate doses depending on the patient's body weight.

In one embodiment, the present invention relates to a method of preventing or treating a CA9-associated disease or symptom by administering a peptide or conjugate of the present invention (comprising a pharmaceutically acceptable salt, isomer, or solvate thereof). The treatment method of the present invention comprises, without limitation, a step of administering a peptide or conjugate of the present invention to a subject. The subject of administration and the method of administration are substantially the same as those described in the section "pharmaceutical composition". Also, without limitation, the treatment method of the present invention is intended to deliver a pharmaceutically active ingredient to any target site (affected area) by using the peptide or conjugate of the present invention, and comprises a step of administering the peptide or conjugate of the present invention to a subject.

In one embodiment, the present invention relates to use of a peptide or conjugate of the present invention or a pharmaceutically acceptable salt thereof for preventing or treating a CA9-associated disease or symptom.

In one embodiment, the present invention relates to use of a peptide or conjugate of the present invention or a pharmaceutically acceptable salt thereof for the manufacture of a pharmaceutical composition for preventing or treating a CA9-associated disease or symptom.

In one embodiment, the present invention relates to use of a peptide or conjugate of the present invention or a pharmaceutically acceptable salt thereof as a pharmaceutical composition for preventing or treating a CA9-associated disease or symptom.

In one embodiment, the present invention relates to a peptide or conjugate of the present invention or a pharmaceutically acceptable salt thereof to be used in a method of preventing or treating a CA9-associated disease or symptom.

In one embodiment, the present invention relates to a peptide or conjugate of the present invention or a pharmaceutically acceptable salt thereof to be used as a pharmaceutical composition for preventing or treating a CA9-associated disease or symptom.

### Diagnostic Composition and Imaging Agent

The present invention also relates to a diagnostic composition comprising a peptide or conjugate of the present invention or a pharmaceutically acceptable salt thereof for diagnosing a CA9-associated disease or symptom.

The peptide or conjugate of the present invention may also be used as a composition for diagnosing a CA9-associated disease or symptom. As a diagnostic agent, it may be a detection agent for determining whether a subject suffers from a CA9-associated disease or symptom, for diagnosing the severity of the disease, or for predicting a poor prognosis of the disease. When used as a detection agent, the peptide or conjugate may be detectably labeled, may be a conjugate containing a detectable payload, or may be a conjugate containing a known imaging agent as a payload.

The present invention includes, in one embodiment, an imaging agent containing the conjugate. The imaging agent can also be used as a diagnostic composition. In one embodiment, the diagnostic composition is an imaging agent. As used herein, the "imaging agent" is a compound having one or more properties that make it possible to detect directly or indirectly its presence and/or location, enabling diagnostic imaging to be performed. Examples of such an imaging agent include a protein, a peptide, a medium- or small-molecular-weight compound, and a radioisotope itself, all of which incorporate a labeling moiety that enables detection.

The labeling moiety that enables detection may be, for example, an enzyme such as peroxidase or alkaline phosphatase; a radioisotope (which may be coordinated with a chelator or bonded to a compound such as glucose); a fluorescent substance such as fluorescein isothiocyanate (FITC), rhodamine, dansyl chloride, phycoerythrin, tetramethylrhodamine isothiocyanate, fluorescein amidite (FAM), eosin, carboxyfluorescein, erythrosin, carboxytetramethylrhodamine (TAMRA), tetramethylrhodamine (TMR), sulforhodamine, or near-infrared fluorescent materials; or a luminescent substance such as luciferase, luciferin, or aequorin. Alternatively, an antibody labeled with such a luminescent substance or the like may also be used. In addition, an antibody labeled with nanoparticles such as gold colloids and quantum dots may be used for detection.

For example, a complex of the antibody and the peptide may be prepared, and the antibody or the peptide may be labeled to form a labeled complex. By administering and detecting the labeled complex, the severity or the like of a CA9-associated disease or symptom can be determined. Also, in immunoassay, the peptide may be labeled with biotin and detected by binding the biotin to avidin or streptavidin labeled with, for example, an enzyme.

Among immunoassays, an ELISA using enzyme labeling is preferred because of its simplicity and rapid antigen measurement. For example, an antibody is immobilized on a solid-phase carrier, a sample is added and reacted, and then the labeled peptide is added and reacted. After washing, it may be subjected to a reaction with an enzyme substrate for development, and the absorbance may be measured to detect the severity or the like of a CA9-associated disease or symptom. After the reaction of the sample with the antibody immobilized on the solid-phase carrier, an unlabeled peptide may be added, and the antibody against the peptide may be enzyme-labeled and added further. The antibody may be immobilized on or inside the surface of the solid-phase carrier.

The enzyme substrate used may be, for instance, 3,3'-diaminobenzidine (DAB), 3, 3', 5, 5'-tetramethylbenzidine (TMB), or o-phenylenediamine (OPD) in the case where the enzyme is peroxidase and may be, for instance, p-nitrophenyl phosphate (pNPP) in the case of alkaline phosphatase.

The "solid-phase carrier" herein is not limited as long as the carrier can be used to immobilize the antibody. Examples include a glass-, metal-, or resin-made microtiter plate, substrate, or bead, and a nitrocellulose, nylon, or PVDF membrane. The target substance may be immobilized on the solid-phase carrier in accordance with a known method.

As used herein, the term "diagnostic imaging" includes imaging techniques such as immunohistochemistry and immunofluorescence staining; optical imaging techniques such as positron emission tomography (PET, including PET-CT) and single-photon emission computed tomography (SPECT); and non-invasive (molecular) diagnostic imaging techniques including magnetic resonance imaging (MRI), iron oxide nanoparticles, and carbon-coated iron-cobalt nanoparticles. The imaging agent of the present invention can be used for any diagnostic imaging by appropriately selecting a labeling moiety that enables detection. Preferably, it is an imaging agent for use in PET, PET-CT, or SPECT, which is optical imaging. In one embodiment, the present invention relates to a radioligand imaging agent that contains a conjugate of the present invention and is used for positron emission tomography.

The present invention also relates to a diagnostic kit comprising a peptide or conjugate of the present invention. The diagnostic kit comprises a reagent and a tool (including, but is not limited to, any or all of the peptide or conjugate of the present invention, an antibody, a solid-phase carrier, buffer solution, enzyme reaction stopping solution, and/or a microplate reader) necessary for the above detection.

The present invention relates to a method of diagnosing a CA9-associated disease or symptom, the method comprising using a peptide or conjugate of the present invention. The "diagnosis method" includes *in vivo* or *in vitro* diagnosis methods. The method of the present invention comprises, without limitation, a step of administering a peptide or conjugate of the present invention to a subject or a sample obtained from the subject. The method of the present invention comprises, without limitation, a step of administering a peptide or conjugate of the present invention to a subject or a sample obtained from the subject, and detecting binding of the peptide or conjugate of the present invention to CA9. The subject of administration and the method of administration are substantially the same as those described in the section "Pharmaceutical composition". Examples of the "sample obtained from the subject" include blood, urine, stool, tear fluid, nasal discharge, and tissue sections.

The present invention also relates to a method of detecting a disease by using the peptide or conjugate of the present invention. The disease can be detected by, for instance, laboratory technicians or researchers other than physicians, at research institutes (including educational institutions such as universities), and companies, etc. In one embodiment, the method of detecting a disease does not include medical treatment. The diagnosis method preferably uses PET, PET-CT or SPECT.

The present invention relates to use of a peptide or conjugate of the present invention for diagnosing a CA9-associated disease or symptom.

The present invention relates to use of a peptide or conjugate of the present invention for the manufacture of a diagnostic composition for diagnosing a CA9-associated disease or symptom.

The present invention relates to use of a peptide or conjugate of the present invention as a diagnostic composition for diagnosing a CA9-associated disease or symptom.

The present invention relates to a peptide or conjugate of the present invention in use of a method of diagnosing a CA9-associated disease or symptom.

The present invention relates to a peptide or conjugate of the present invention in use as a diagnostic composition for diagnosing a CA9-associated disease or symptom.

The present invention also relates to a tester comprising a peptide or conjugate of the present invention. The present invention also relates to a diagnostic or detection tester comprising a peptide or conjugate of the present invention.

### Research Composition

The present invention also relates to a research composition comprising a peptide or conjugate of the present invention. The "research composition" includes those used by, for instance, researchers, technicians, students, or doctors in research institutes (including educational institutions such as universities), companies, and hospitals, etc.

The research composition can be used, for example, for detection of CA9 or detection of CA9-associated disease or symptom.

The carrier for immobilizing the peptide or conjugate herein is not particularly limited. Examples include a glass-, metal-, or resin-made microtiter plate, substrate, or bead, and a nitrocellulose, nylon, or PVDF membrane.

The present invention also includes a method of detecting CA9, by using a peptide or conjugate of the present invention. The "detection method" includes *in vivo* or *in vitro* detection methods. The method of the present invention comprises, without limitation, a step of administering a peptide or conjugate of the present invention to a subject or a sample obtained from the subject. The method of the present invention comprises, without limitation, a step of administering a peptide or conjugate of the present invention to a subject or a sample obtained from the subject, and detecting binding of the peptide or conjugate of the present invention to CA9. The subject of administration and the method of administration are substantially the same as those described in the section "Pharmaceutical composition". Examples of the "sample obtained from the subject" include blood, urine, stool, tear fluid, nasal discharge, and tissue sections.

The present invention also includes use of a peptide or conjugate of the present invention for detection of CA9.

The present invention also relates to a diagnostic or detection kit comprising a peptide or conjugate of the present invention. The diagnostic or detection kit comprises a reagent and a tool (including, but is not limited to, any or all of the peptide of the present invention, an antibody, a solid-phase carrier, buffer solution, enzyme reaction stopping solution, and/or a microplate reader) necessary for the above detection.

In one embodiment, the present invention includes a method of delivering any payload to a subject by using a peptide or conjugate of the present invention. The method of the present invention comprises, without limitation, a step of producing a conjugate containing a peptide of the present invention and any payload and administering the peptide to a subject. The subject of administration and the method of administration are substantially the same as those described in the section "Pharmaceutical composition".

In one embodiment, the present invention relates to a method of using a peptide or conjugate of the present invention to deliver any payload to any part (affected area) of a subject. In the method of the present invention, a method of delivering to any part of a subject comprises, without limitation, a step of producing a conjugate containing a peptide of the present invention and any peptide and administering the peptide to the subject. The subject of administration and the method of administration are substantially the same as those described in the section "Pharmaceutical composition".

The matters described in other sections also apply to this section, unless otherwise noted.

### 7. Test Method

The present invention also relates to a method of testing a peptide or a pharmaceutically acceptable salt thereof, comprising testing at least one of
a) solubility in a solvent;
b) CA9 binding activity;
c) cell and/or tissue toxicity; or
d) toxicity to an experimental animal, wherein
the peptide or pharmaceutically acceptable salt thereof is the peptide or pharmaceutically acceptable salt thereof according to the present invention.

The present invention also relates to a conjugate testing method comprising testing a conjugate in terms of at least one of:
a) solubility in a solvent;
b) CA9 binding activity;
c) cell and/or tissue toxicity; or
d) toxicity to an experimental animal, wherein
the conjugate is a conjugate of the present invention.

The "solubility in a solvent" can be measured using known methods. In measuring solubility, the solvent is not limited and may be freely selected depending on the intended purpose. As for the method of solubility measurement, any of the known methods can be appropriately selected depending on the type of solvent. Without limitation, the solubility may be the solubility obtained when the peptide or conjugate is dissolved in a known solvent such as water, glycerol, PBS, or DMSO.

The "CA9 binding activity" may be measured, for example, as described in "2. Peptide", etc.

The "cell and/or tissue toxicity" can be measured using known methods. For example, testing for the cell and/or tissue toxicity may be a known toxicity assessment test using cells and/or tissues, and may be a method performed *in vitro.* The cells and/or tissues may be, but are not limited to, cells and/or tissues that are normally evaluated and tested for toxicity for pharmaceutical products.

The "toxicity to an experimental animal" can be measured using known methods. For example, the experimental animal(s) is not particularly limited as long as they are those commonly used, and examples thereof include mice, rats, guinea pigs, gerbils, hamsters, ferrets, rabbits, dogs, cats, pigs, goats, horses, cattle, birds (e.g., chickens, quails), monkeys, and non-human primates (e.g., cynomolgus monkeys, marmosets, rhesus monkeys).

In addition, the above toxicity evaluation tests may be, but are not limited to, safety studies that are usually conducted in non-clinical studies of pharmaceutical products. Examples include general toxicity studies (single-dose and repeated-dose toxicity studies), genotoxicity studies (Ames test, chromosomal aberration test, *in vitro* micronucleus test), carcinogenicity studies, reproductive and developmental toxicity studies (ICH-I, II, III), local irritation studies (ocular and skin irritation studies), other toxicity studies (skin sensitization test, phototoxicity test, antigenicity test), and chemical and bioanalytical evaluations (TK/PK).

The matters described in other sections also apply to this section, unless otherwise noted.

### 8. Combinatory Use, Combination

The peptide or conjugate of the present invention may be used in combination with a medicament for preventing or treating other CA9-associated disease or symptom. In one embodiment, the present invention relates to a combination of the peptide or conjugate and a medicament for preventing or treating other CA9-associated disease or symptom.

The peptide or conjugate and a medicament for preventing or treating other CA9-associated disease or symptom may be administered simultaneously or sequentially. Preferably, the peptide or conjugate and a medicament for preventing or treating other CA9-associated disease or symptom are administered so as to achieve an additive effect of both, preferably a synergetic effect. When administered sequentially, the order of administration does not matter. When administered sequentially, it is preferable, without limitation, that both are administered within 2 hours, within 1 hour, within 30 min, within 20 min, within 15 min, within 10 min, or within 5 min.

The matters described in other sections also apply to this section, unless otherwise noted.

### EXAMPLES

Hereinafter, the present invention is further described in detail with reference to Examples. The present invention, however, is not limited to them. Those skilled in the art may easily make modifications and changes to the present invention based on the description herein, and they are included in the technical scope of the present invention. Note that the compound names given in the following Reference Examples and Examples do not necessarily follow the IUPAC nomenclature. Also note that abbreviations may be used for the sake of simplicity of description, but these abbreviations are as stated above.

Raw materials, building blocks, reagents, acids, bases, solid-phase resins, and solvents used in the chemical synthesis of the compounds were either commercially available products as they were or, when otherwise specified, were synthesized using organic chemistry. Note that a protecting group-containing amino acids were commercially available products as they were.

As for the numbering of peptide residues, the amino acid residue that is ClAc-modified is counted as residue 1, and the subsequent residues are counted as residue 2, residue 3, and so on, toward the resin. The common amino acids used are listed below, and each side chain protecting group is indicated in parentheses.
Fmoc-Ile-OH;
Fmoc-Ser(Trt)-OH;
Fmoc-Ser(tBu)-OH;
Fmoc-His(Boc)-OH;
Fmoc-Pro-OH;
Fmoc-Asp(OMpe)-OH;
Fmoc-Glu(tBu)-OH;
Fmoc-Y(tBu)-OH;
Fmoc-Gly-OH;
Fmoc-Cys(Trt)-OH;
Boc-Phe-OH;
Fmoc-Lys(Boc)-OH.

In addition, for non-natural amino acids, the following and those listed in the abbreviations were used.
Fmoc-da-OH;
Fmoc-de(tBu)-OH;
Fmoc-dgln(Trt)-OH;
Fmoc-dhgl(tBu)-OH;
Fmoc-dkCOpipzaa(tBu)-OH;
Fmoc-Tbg-OH;
Fmoc-3Py6CON-OH;
Fmoc-3Py6NH2(Boc)-OH;
Fmoc-Hgl(tBu)-OH;
Fmoc-alT(tBu)-OH;
Fmoc-Hgn(Trt);
Fmoc-SMe-OH;
Fmoc-DacAc-OH;
Fmoc-KCOpipzaa(tBu)-OH;
Fmoc-N-Me-da-OH;
Fmoc-PeG-OH;
Fmoc-MeG-OH;
Fmoc-MedkCOpipzaa(tBu)-OH;
Fmoc-Ahp-OH;
Fmoc-pHPeG(tBu)-OH;
Fmoc-pMeOPeG-OH;
Fmoc-MsMeapG-OH;
Fmoc-3OMePeG-OH;
Fmoc-PpG-OH;
Fmoc-pFPeG-OH;
Fmoc-mCPeG-OH;
Fmoc-pCPeG-OH;
Fmoc-4HPpG-OH;
Fmoc-4OMePpG-OH;
Fmoc-4PypG-OH;
Fmoc-4COOPeG(tBu)-OH;
Fmoc-3COOPeG(tBu)-OH;
Fmoc-3FPeG-OH;
Fmoc-Hse(Trt)-OH;
Fmoc-P4Sh(tBu)-OH;
Fmoc-Qmm-OH;
Fmoc-Qdm-OH;
Fmoc-QPEG8Me-OH;
Fmoc-5Inda-OH;
Fmoc-F4OMe-OH;
Fmoc-HseMe-OH;
Fmoc-Acb-OH;
Fmoc-dkAc-OH;
Boc-df-OH;
Boc-dnle-OH;
Fmoc-t4amCh-OH;
Boc-Nle-OH;
Fmoc-N-Me-Cys(Trt)-OH;
Fmoc-N-Me-Ala-OH;
Fmoc-N-Me-Ser(Trt)-OH;
Fmoc-N-Me-Asn(Trt)-OH;
Fmoc-Hpr-OH;
Boc-PEG8c-OH;
Fmoc-PEG10c-OH;
Fmoc-dk(Boc)-OH;
Fmoc-dp-OH;
Fmoc-dyae(Boc)-OH:
Alloc-dk(Fmoc)-OH;
Boc-PEG8c-OH;
Fmoc-de(Allyl)-OH;
Fmoc-Hgl(Allyl)-OH;
Fmoc-dk(Alloc)-OH;
Fmoc-Qglucamine(acetonide)-OH.

The structure of each chemically synthesized peptide was determined by ESI-MS(+) in mass spectrometry to confirm the molecular weight calculated by considering the amino acids used according to the target sequence and the building blocks used as necessary. Note that the term "ESI-MS(+)" indicates electrospray ionization mass spectrometry performed in positive ion mode. The detected masses are reported in "m/z" units. Note that when the molecular weight was approximately larger than 1000, the compound was frequently detected as a divalent or trivalent ion.

### Example 1: Synthesis of Cyclic Peptides and Synthesis of Conjugates

The chemically synthesized peptide was identified based on the retention time obtained by any of the following analytical methods (detection: at a UV wavelength of 225 nm; Shimadzu "SPD-M20A").

### Analytical condition A

Column: Kinetex (registered trademark) EVO C18 2.6 µm, 2.1 ID × 150 mm, 100 Å
Mobile phase: A = 0.025% TFA in water; B = 0.025% TFA in CH₃CN
Temperature: 60°C
Flow rate: 0.5 mL/min
Gradient: 20-60/7.15 min, 60-95/0.3 min.

### Analytical condition B

Column: Kinetex (registered trademark) EVO C18 2.6 µm, 2.1 ID × 150 mm, 100 Å
Mobile phase: A=0.025% TFA in water; B=0.025% TFA in CH₃CN
Temperature: 60°C
Flow rate: 0.5 mL/min
Gradient: 5-45/7.15 min, 45-95/0.3 min.

Peptide chain elongation on a solid-phase resin was performed by the Fmoc method using a commercially available resin as a starting material. Specifically, the target peptides were each synthesized using Sieber amide resin (WATANABE CHEMICAL INDUSTRIES, LTD.) by starting with the removal of the Fmoc group and repeating the introduction of each Fmoc amino acid and the deprotection of the Fmoc group. Unless otherwise stated, the peptide chain elongation was performed by automated synthesis, and Liberty Blue or Liberty blue HT from CEM was used as a solid-phase synthesizer, and the peptide was synthesized according to the manufacturer's instruction. For the introduction of each residue, double coupling was performed by repeating the peptide coupling reaction twice if necessary. On the other hand, for the introduction of some amino acids, the resin was removed from the automatic synthesizer and the reaction was carried out using reagents prepared on the fly, etc. (manual coupling).

For example, to a peptide chain from which the Fmoc group had been removed and which was immobilized on a solid-phase resin, about 4 equivalents of Fmoc amino acids, about 8 equivalents of DIC, and about 4 equivalents of Oxyma pure were added, followed by a step of shaking 1-2 times under one of the reaction conditions listed in the table below, and the resin was then washed with DMF.

**[Table 2]**

| Method | Temperature (°C) | Time (min) |
|---|---|---|
| 1-1 | 90 | 3-15 |
| 1-2 | 75 | 10-30 |
| 1-3 | 25-50 | 15-60 |
| 1-4 | 105 | 2-10 |

The removal of the Fmoc group was performed, for example, according to the following method. The peptide chain that was after the Fmoc amino acid introduction and was immobilized on a solid-phase resin was shaken 1-2 times under one of the conditions shown in the table below, and the resin was then washed with DMF.

**[Table 3]**

| Method | Reagent | Temperature | Time |
|---|---|---|---|
| 2-1 | 20% Piperidine DMF solution | 50-90°C | 1-5 min |
| 2-2 | 20% Piperidine DMF solution | Room temperature | 1-5 min |
| 2-3 | 4% Pyrrolidine/83 mM Oxyma Pure DMF solution | 110°C | 1-5 min |
| 2-4 | 10-25% Pyrrolidine DMF solution | 50-90°C | 1-5 min |
| 2-5 | 10-25% Pyrrolidine DMF solution | Room temperature | 1-5 min |

Following the method described above, introduction of each Fmoc amino acid and deprotection of the Fmoc group were repeated to elongate the peptide chain to the desired peptide chain, and then chloroacetyl groups were introduced. The introduction method is one of the methods listed in the table below. After removing the Fmoc group from the Fmoc-protected peptide immobilized on the solid-phase resin as obtained in the previous step, a solution containing about 5-10 equivalents of chloroacetic acid, about 5-10 equivalents of the condensation agent, and about 5-10 equivalents of the additive in the following solvents was added to the solid-phase resin. The resulting mixture was shaken at room temperature for 30 to 60 min.

**[Table 4]**

| Method | Condensation agent | Additive | Solvent |
|---|---|---|---|
| 3-1 | DIPCI(DIC) | HOSu | DMF/DCM |
| 3-2 | HATU | DIPEA | DMF |
| 3-3 | DIPCI | HOSu | DCM/NMP |

Deprotection of side chains and cleavage from the solid-phase resin were performed by adding one of the reaction agent cocktails (mixture of TFA/H₂O/TIS/DODT) listed in the table below to the peptide attached to the resin after introduction of chloroacetyl group, and shaking the resin at room temperature for 30 to 120 min.

**[Table 5]**

| Method | TFA/H₂O/TIS/DODT(v/v/v/v) |
|---|---|
| 4-1 | 92.5/2.5/2.5/2.5 |
| 4-2 | 90/2.5/2.5/5 |

The reaction solution was then filtered through a frit, collected, and the filtrate was added to a mixed solvent (e.g., 1/1, v/v) of excess diisopropyl ether or diisopropyl ether and hexane or diethyl ether and hexane cooled to 0°C to form a clouded precipitate. To obtain this precipitate solid, centrifugation was performed and the supernatant was decanted. The resulting solid was washed again with a small amount of diethyl ether cooled to 0°C and dried under reduced pressure.

The solid obtained above was used in a peptide cyclization reaction. For the peptide cyclization reaction, the peptide was dissolved in DMSO or a mixed solvent of DMSO/H₂O, MeCN/H₂O, or DMSO/MeCN/H₂O to a final concentration of 4 mM to 5 mM based on the number of moles of solid-phase resin, and 5 to 35 equivalents of triethylamine was added. The mixture was then shaken at room temperature for 2 hours to overnight. The resulting reaction solution was concentrated under reduced pressure and then purified.

The obtained residue was purified by reversed-phase preparative HPLC according to one of the methods in the table below.

**[Table 6]**

| Method | Column | Mobile phase A | Mobile phase B | Temperature | Flow rate |
|---|---|---|---|---|---|
| P-A | Waters XBridge (registered trademark), C18 5 µm, 50x150mm | 0.1% TFA in H₂O | 0.1% TFA in CH₃CN | 40°C | 120 mL/min |
| P-B | Waters XBridge (registered trademark), C18 5 µm, 30x150mm | 0.1% TFA in H₂O | 0.1% TFA in CH₃CN | 40°C | 45 mL/min |
| P-C | Waters XBridge (registered trademark), C18 5 µm, 50x250mm | 0.1% TFA in H₂O | 0.1% TFA in CH₃CN | 50°C | 120 mL/min |
| P-D | Waters XSelect (registered trademark), C18 5 µm, 50x150mm | 0.1% TFA in H₂O | 0.1% TFA in CH₃CN | 40°C | 120 mL/min |

The specific structures of the synthesized peptides and conjugates, etc. are shown below.

Examples 1-1 to 1-3 are related to the synthesis of each peptide.

### Example 1-1: Synthesis of Peptide SEQ ID No. 45

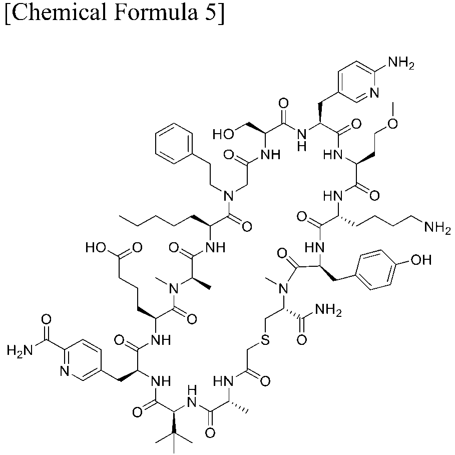

Sieber amide resin (WATANABE CHEMICAL INDUSTRIES, LTD.; 0.60 mmol/g, 1.67 g) was used to synthesize the target peptide. At that time, CEM's Liberty Blue was used as a solid-phase synthesizer for synthesis according to the manufacturer's instruction.

For removal of Fmoc on the solid-phase resin, the reaction was carried out once with 10% pyrrolidine (in DMF) at 75°C for 3 min.

For the introduction of each residue, 0.21 M Fmoc-AA (in DMF)/1.0 M DIC (in DMF)/0.5 M Oxyma pure (in DMF) (4.2 equivalents/8 equivalents/4 equivalents) were used per equivalent of resin, and the reaction was carried out once for 10 min at 75°C. For the introduction of each of residues 3 and 9, 0.21 M Fmoc-AA (in NMP)/1.0 M DIC (in DMF)/0.5 M Oxyma pure (in DMF) (4.2 equivalents/8 equivalents/4 equivalents) were used, and for each of residues 4 and 6, the reaction was carried out twice for 20 min at 75°C. The reaction was carried out twice for residue 12 at 75°C for 30 min. The reaction was carried out once for residue 13 at 50°C for 30 min.

After the introduction of each residue, the reaction was carried out once for 3 min at 75°C with 4% pyrrolidine + 83 mM Oxyma pure (in DMF) on the solid-phase resin having the Fmoc-protected peptide immobilized for removal of the Fmoc group. The reaction was carried out twice for residue 4 by using 10% pyrrolidine-containing DMF solution at room temperature for 5 min. For each of residues 6 and 12, the reaction was carried out, using 10% pyrrolidine-containing DMF solution, once at room temperature for 5 min and once for 10 min.

The Fmoc group of the α-amino group was removed by the reaction performed once at 75°C for 3 min using 10% pyrrolidine-containing DMF solution for the solid-phase resin, on which the Fmoc-protected peptide obtained in the previous step was immobilized. This was further shaken with 1.0 M ClAcNHS (10 equivalents in DMF) for 60 min at room temperature to introduce the chloroacetyl group.

For the deprotection of side chains and the cleavage from the solid-phase resin, the resin obtained after the chloroacetyl group introduction step was first washed once with DMF, methylene chloride, and diethyl ether, and then dried under reduced pressure. Subsequently, the solid-phase resin was divided into about half. The reaction agent cocktail (10 mL; a mixture of TFA/H₂O/TIS/DODT at a volume ratio of 92.5:2.5:2.5:2.5) was then added to a reaction vessel, and the mixture was shaken at room temperature for 40 min. The reaction solution was collected by filtration through a frit. The solid-phase resin remaining in the reaction vessel was shaken again with the cocktail for cleavage, and the solution component was collected from the frit and mixed with the above filtrate. When the filtrate was divided into 4 portions and each was added to 40 mL of a diethyl ether/hexane (1/1) mixed solvent, a precipitate occurred.

The resulting mixture was centrifuged, and the solution was then decanted. The resulting solid was washed again with diethyl ether and then dried under reduced pressure. The solid (peptide) obtained was collectively used in the next cyclization reaction. The cyclization reaction of the peptide was performed by dissolving the peptide in water/acetonitrile (1/1) to a final concentration of 5 mM based on the number of moles of the solid-phase resin; adding 10 equivalents of triethylamine; and stirring the mixture at room temperature for 60 min. The reaction was stopped by adding acetic acid to the reaction solution. The resulting reaction solution was concentrated under reduced pressure by using Genevac EZ-II Elite.

The crude product obtained was purified using the following conditions (column: Waters XBridge (registered trademark) C18 5 µm 50 × 250 mm; mobile phase: A = 0.1% TFA (in water), B = 0.1% TFA (in MeCN); temperature: 50°C; gradient (%B): 4.2-0.0% over 0.1 min, 0.0-0.0% over 4.9 min, 0.0-4.2% over 2 min, 4.2-28.6% over 3 min, 28.6-33.7% over 15 min, 33.7-60% over 3 min; flow rate: 118-18 mL/min over 0.1 min, 18-18 mL/min over 4.9 min, 18-118 mL/min over 2 min, then 118 mL/min). The target product-containing fractions were collected and lyophilized to obtain the peptide of interest.

The purity of the target product was calculated from the area ratio of LC/MS (UV wavelength: 225 nm) chromatogram under the analytical conditions. The purity of the target product was 96.7%.
Analytical condition A: retention time = 2.86 min
ESI-MS(+): observed m/z = 862.37 (M+2H)²⁺

### Example 1-2: Synthesis of Peptide SEQ ID No. 46

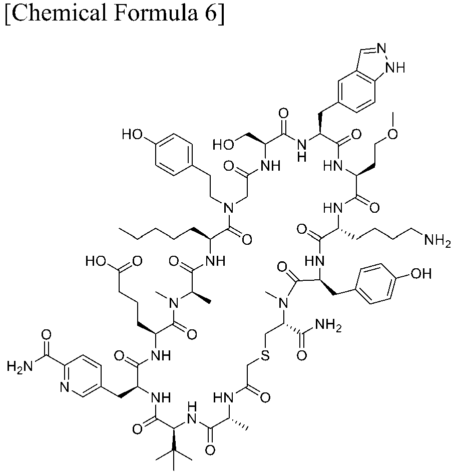

Sieber amide resin (WATANABE CHEMICAL INDUSTRIES, LTD.; 0.60 mmol/g, 1.67 g) was used to synthesize the target peptide. At that time, CEM's Liberty Blue was used as a solid-phase synthesizer for synthesis according to the manufacturer's instruction.

For removal of Fmoc on the solid-phase resin, the reaction was carried out once with 10% pyrrolidine (in DMF) at 75°C for 3 min.

For the introduction of each residue, 0.21 M Fmoc-AA (in DMF)/0.5 M HATU (in DMF)/1.0 M DIPEA (in DMF) (4.2 equivalents/4 equivalents/8 equivalents) were used per equivalent of resin, and the reaction was carried out once for 10 min at 75°C. For the introduction of each of residues 3 and 9, 0.21 M Fmoc-AA (in NMP)/0.5M HATU (in DMF)/1.0 M DIPEA (in DMF) (4.2 equivalents/4 equivalents/8 equivalents) were used, and for each of residues 4, 6, and 12, the reaction was carried out twice for 10 min at 75°C. The reaction was carried out once for residue 13 at 40°C for 30 min.

After the introduction of each residue, the reaction was carried out once for 3 min at 75°C with 4% pyrrolidine + 83 mM Oxyma pure (in DMF) on the solid-phase resin having the Fmoc-protected peptide immobilized for removal of the Fmoc group. For each of residues 4, 6, and 12, the reaction was carried out, using 10% pyrrolidine-containing DMF solution, twice at room temperature for 5 min.

The Fmoc group of the α-amino group was removed by the reaction performed once at 75°C for 3 min using 10% pyrrolidine-containing DMF solution for the solid-phase resin, on which the Fmoc-protected peptide obtained in the previous step was immobilized. This was further shaken with 0.25 M ClAcNHS (5 equivalents in DMF) for 60 min at room temperature to introduce the chloroacetyl group.

For the deprotection of side chains and the cleavage from the solid-phase resin, the resin obtained after the chloroacetyl group introduction step was first washed once with DMF, methylene chloride, and diethyl ether, and then dried under reduced pressure. Subsequently, the solid-phase resin was divided into about half. The reaction agent cocktail (20 mL; a mixture of TFA/H₂O/TIS/DODT at a volume ratio of 92.5:2.5:2.5:2.5) was then added to a reaction vessel, and the mixture was shaken at room temperature for 30 min. The reaction solution was collected by filtration through a frit. The solid-phase resin remaining in the reaction vessel was shaken again with the cocktail for cleavage, and the solution component was collected from the frit and mixed with the above filtrate. When the filtrate was divided into 8 portions and each was added to 40 mL of a diisopropylether/hexane (1/1) mixed solvent, a precipitate occurred.

The resulting mixture was centrifuged, and the supernatant was then decanted. The resulting solid was washed again with diethyl ether and then dried under reduced pressure. The solid (peptide) obtained was collectively used in the next cyclization reaction. The cyclization reaction of the peptide was performed by dissolving the peptide in water/acetonitrile (1/1) to a final concentration of 2.5 mM based on the number of moles of the solid-phase resin; adding 10 equivalents of triethylamine; and stirring the mixture at room temperature for 16 hours. The reaction was stopped by adding acetic acid to the reaction solution. The resulting reaction solution was concentrated under reduced pressure by using Genevac HT-12.

The resulting crude product was purified using the following conditions (column: Waters XSelect (registered trademark) C18 5 µm 50 × 150 mm; mobile phase: A = 0.1% TFA (in water), B = 0.1% TFA (in MeCN); temperature: 40°C; gradient (%B): 5.0-5.0% over 2 min, 5.0-25.0% over 1 min, 25.0-30.0% over 8 min, 30.0-60.0% over 1 min; flow rate: 20-20 mL/min over 1 min, 20-120 mL/min over 1 min, then 120 ml/min). The target product-containing fractions were collected and lyophilized to obtain the peptide of interest.

The purity of the target product was calculated from the area ratio of LC/MS (UV wavelength: 225 nm) chromatogram under the analytical conditions. The purity of the target product was 96.3%.
Analytical condition A: retention time = 3.30 min
ESI-MS(+): observed m/z = 882.36 (M+2H)²⁺

### Example 1-3: Synthesis of Peptide SEQ ID No. 47

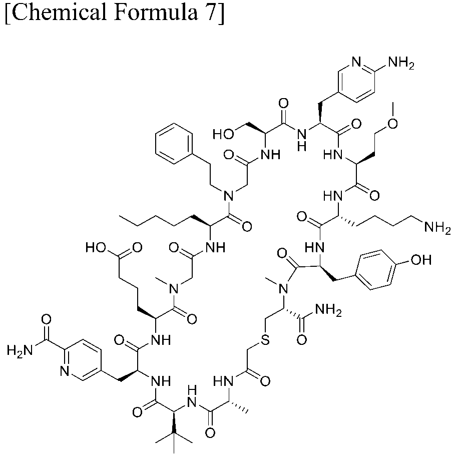

Sieber amide resin (WATANABE CHEMICAL INDUSTRIES, LTD.; 0.57 mmol/g, 0.53 g) was used to synthesize the target peptide. At that time, CEM's Liberty Prime was used as a solid-phase synthesizer for synthesis according to the manufacturer's instruction.

For removal of Fmoc on the solid-phase resin, the reaction was carried out once with 10% pyrrolidine (in DMF) at 110°C for 1 min.

For the introduction of each residue, 0.21 M Fmoc-AA (in DMF)/2.0 M DIC (in DMF)/0.25 M Oxyma pure (in DMF) (4.2 equivalents/8 equivalents/4 equivalents) were used per equivalent of resin, and the reaction was carried out once for 2 min at 105°C. For the introduction of each of residues 3 and 9, 0.21 M Fmoc-AA (in NMP)/2.0 M DIC (in DMF)/0.25 M Oxyma pure (in DMF) (4.2 equivalents/8 equivalents/4 equivalents) were used, and for residue 4, the reaction was carried out twice for 3 min at 105°C. For each of residues 6 and 12, the reaction was carried out twice at 90°C for 10 min, and for residue 13, once at 40°C for 30 min.

After the introduction of each residue, the reaction was carried out once for 3 min at 110°C with 4% pyrrolidine + 83 mM Oxyma pure (in DMF) on the solid-phase resin having the Fmoc-protected peptide immobilized for removal of the Fmoc group. For each of residues 4, 6, and 12, the reaction was carried out, using 10% pyrrolidine-containing DMF solution, twice at room temperature for 1 min.

The Fmoc group of the α-amino group was removed by the reaction performed once at 110°C for 1 min using a 4% pyrrolidine/83 mM Oxyma Pure-containing DMF solution for the solid-phase resin, on which the Fmoc-protected peptide obtained in the previous step was immobilized. This and 0.1 M ClAcOH (in DMF)/0.1 M HATU (in DMF)/0.2 M DIEA (in DMF) (5 equivalents/5 equivalents/10 equivalents) were together shaken for 30 min at 25°C to introduce a chloroacetyl group.

For the deprotection of side chains and the cleavage from the solid-phase resin, the resin obtained after the chloroacetyl group introduction step was first washed once with DMF, methylene chloride, and diethyl ether, and then dried under reduced pressure. The reaction agent cocktail (14 mL; a mixture of TFA/H₂O/TIS/DODT at a volume ratio of 92.5:2.5:2.5:2.5) was then added to the solid-phase resin containing reaction vessel, which was shaken at room temperature for 60 min. The reaction solution was collected by filtration through a frit. The solid-phase resin remaining in the reaction vessel was shaken again with the cocktail for cleavage, and the solution component was collected from the frit and mixed with the above filtrate. When the filtrate was divided into 3 portions and each was added to 40 mL of a diisopropylether/hexane (1/1) mixed solvent, a precipitate occurred.

The resulting mixture was centrifuged, and the solution was then decanted. The resulting solid was washed again with diethyl ether and then dried under reduced pressure. The solid (peptide) obtained was collectively used in the next cyclization reaction. The cyclization reaction of the peptide was performed by dissolving the peptide in water/DMSO (1/9) to a final concentration of 5 mM based on the number of moles of the solid-phase resin; adding 10 equivalents of triethylamine; and stirring the mixture at room temperature overnight. The reaction was stopped by adding acetic acid to the reaction solution. The resulting reaction solution was concentrated under reduced pressure by using Genevac HT-12.

The resulting crude product was purified using the following conditions (column: Waters XBridge (registered trademark) C18 5 µm 50 × 150 mm; mobile phase: A = 0.1% TFA (in water), B = 0.1% TFA (in MeCN); temperature: 40°C; gradient (%B): 5.0-5.0% over 2 min, 5.0-27% over 1 min, 27-32% over 8 min, 32-60% over 1 min; flow rate: 20-20 mL/min over 1 min, 20-120 mL/min over 1 min, then 120 ml/min). The target product-containing fractions were collected and lyophilized, and the target product was again purified using the following conditions (column: Waters XSelect (registered trademark) C18 5 µm 30 × 150 mm; mobile phase: A = 1.0% AcOH (in water), B = 1.0% AcOH (in MeCN); temperature: 40°C; gradient (%B): 2.0-19% over 3 min, 19-24% over 8 min, 24-60% over 1 min; flow rate: 45 ml/min). The target product-containing fractions were collected and lyophilized to obtain the peptide of interest.

The purity of the target product was calculated from the area ratio of LC/MS (UV wavelength: 225 nm) chromatogram under the analytical conditions. The purity of the target product was 99.6%.
Analytical condition B: retention time = 5.71 min
ESI-MS(+): observed m/z = 855.30 (M+2H)²⁺
Examples 1-4 to 1-16 are related to the synthesis of each conjugate.

### Example 1-4: Synthesis of Conjugate No. 64

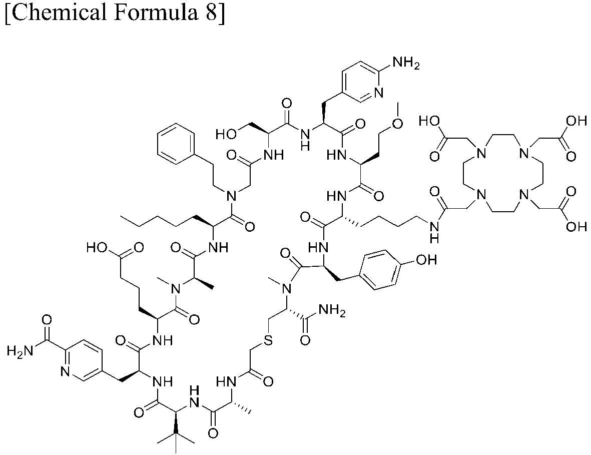

In DMF (4.84 mL), the peptide SEQ ID No. 45 (sequence: C1Ac-da-Tbg-3Py6CON-Hgl-Meda-Ahp-PeG-S-3Py6NH2-HseMe-dk-Y-MeC) (200 mg) synthesized in Example 1-1 and DIEA (225 mg) were dissolved, and DOTA-NHS ester hexafluorophosphate trifluoroacetate (147 mg) was added under ice-cooling. The reaction mixture was stirred at room temperature for 90 min.

The crude product obtained was purified using the following conditions (column: Waters XSelect (registered trademark) C18 5 µm 30 × 150 mm; mobile phase: A = 0.1% TFA (in water), B = 0.1% TFA (in MeCN); temperature: 50°C; gradient (%B): 4.0-0.0% over 0.1 min, 0.0-0.0% over 4.9 min, 0.0-4.0% over 1 min, 4.0-28.5% over 3 min, 28.5-33.6% over 9 min, 33.6-60% over 1 min; flow rate: 44-9 mL/min over 0.1 min, 9-9 mL/min over 4.9 min, 9-44 mL/min over 1 min, then 44 mL/min). The target product-containing fractions were collected and lyophilized to obtain the peptide of interest.

The purity of the target product was calculated from the area ratio of LC/MS (UV wavelength: 225 nm) chromatogram under the analytical conditions. The purity of the target product was 97.3%.
Analytical condition B: retention time = 5.74 min
ESI-MS(+): observed m/z = 704.13 (M+3H)³⁺

### Example 1-5: Synthesis of Conjugate No. 62

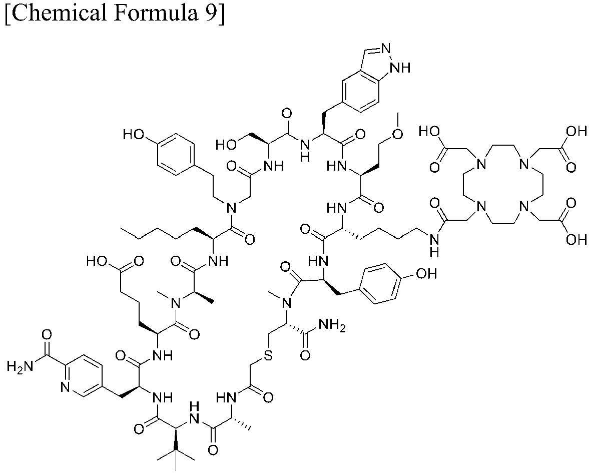

In DMF (2.51 mL), the peptide SEQ ID No. 46 (sequence: ClAc-da-Tbg-3Py6CON-Hgl-Meda-Ahp-pHPeG-S-5Inda-HseMe-dk-Y-MeC) (100 mg) synthesized in Example 1-2 and DIEA (117 mg) were dissolved, and DOTA-NHS ester hexafluorophosphate trifluoroacetate (76 mg) was added under ice-cooling. The reaction mixture was stirred at room temperature for 120 min.

The reaction mixture obtained was purified using the following conditions (column: Waters XBridge (registered trademark) C18 5 µm 50 × 250 mm; mobile phase: A = 0.1% TFA (in water), B = 0.1% TFA (in MeCN); temperature: 50°C; gradient (%B): 4.2-0.0% over 0.1 min, 0.0-0.0% over 4.9 min, 0.0-4.2% over 2 min, 4.2-25.6% over 3 min, 25.6-30.7% over 15 min, 30.7-60% over 3 min; flow rate: 118-18 mL/min over 0.1 min, 18-18 mL/min over 4.9 min, 18-118 mL/min over 2 min, then 118 mL/min). The target product-containing fractions were collected and lyophilized to obtain the peptide of interest.

The purity of the target product was calculated from the area ratio of LC/MS (UV wavelength: 225 nm) chromatogram under the analytical conditions. The purity of the target product was 96.6%.
Analytical condition A: retention time = 3.27 min
ESI-MS(+): observed m/z = 717.38 (M+3H)³⁺

### Example 1-6: Synthesis of Conjugate No. 65

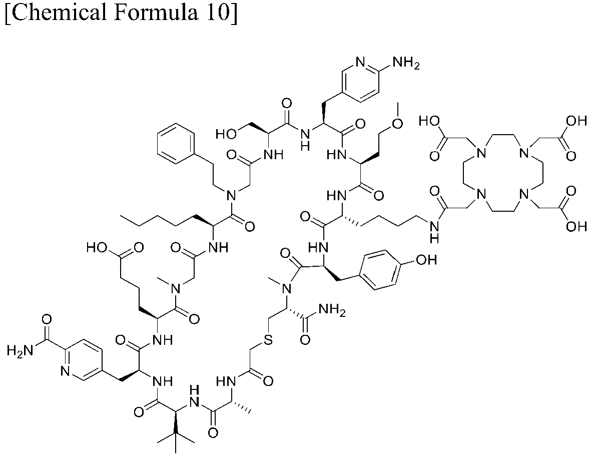

DOTA (0.42 g, CAS: 60239-18-1), DIEA (0.14 g), and N-hydroxysuccinimide (0.08 g, CAS: 6066-82-6) were dissolved in water (3.3 mL). Subsequently, under ice-cooling, 1-(3-dimethylaminopropyl)-3-ethylcarbodiimide hydrochloride (0.13 g, CAS: 25952-53-8), dissolved in water (1.9 mL), was added thereto. The reaction mixture was stirred under ice-cooling for 30 min.

In DMF (561 µL), the peptide SEQ ID No. 47 (sequence: ClAc-da-Tbg-3Py6CON-Hgl-MeG-Ahp-PeG-S-3Py6NH2-HseMe-dk-Y -MeC) (23 mg) synthesized in Example 1-3 and DIEA (13.0 mg) were dissolved, and the solution prepared above (561 µL) was added under ice-cooling. The reaction mixture was stirred at room temperature for 1 hour. DIEA (13.0 mg) was added to the reaction solution, the mixture was stirred for 1 hour, and the reaction was then quenched.

The resulting reaction mixture was purified using the following conditions (column: Waters XBridge (registered trademark) C18 5 µm 19 × 150 mm; mobile phase: A = 0.1% TFA (in water), B = 0.1% TFA (in MeCN); temperature: 50°C; gradient (%B): 5.0-28% over 3 min, 28-33% over 8 min, 33-60% over 1 min; flow rate: 17 ml/min). The target product-containing fractions were collected and lyophilized to obtain the peptide of interest.

The purity of the target product was calculated from the area ratio of LC/MS (UV wavelength: 225 nm) chromatogram under the analytical conditions. The purity of the target product was 97.6%.
Analytical condition A: retention time = 3.14 min
ESI-MS(+): observed m/z = 699.33 (M+3H)³⁺

### Example 1-7: Synthesis of Conjugate No. 26

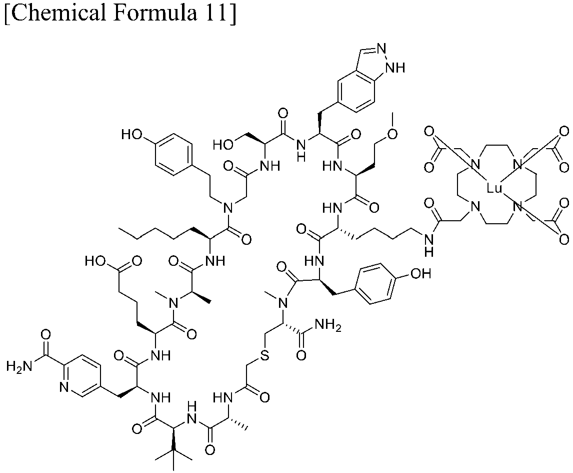

DOTA (0.79 g, CAS: 60239-18-1), DIEA (0.34 mL), and N-hydroxysuccinimide (0.15 g, CAS: 6066-82-6) were dissolved in water (7.2 mL). Subsequently, under ice-cooling, 1-(3-dimethylaminopropyl)-3-ethylcarbodiimide hydrochloride (0.25 g, CAS: 25952-53-8), dissolved in water (4.17 mL), was added thereto. The reaction mixture was stirred under ice-cooling for 60 min.

In DMF (1.00 mL), the peptide SEQ ID No. 46 (sequence: ClAc-da-Tbg-3Py6CON-Hgl-Meda-Ahp-pHPeG-S-5Inda-HseMe-dk-Y-MeC) (40 mg) synthesized in Example 1-2 and DIEA (31.6 µL) were dissolved, and the solution prepared above (1004 µL) was added under ice-cooling. The reaction mixture was stirred at room temperature for 60 min. DIEA (31.6 µL) was added to the reaction mixture and the mixture was stirred at room temperature for 60 min. To the reaction mixture were added lutetium(III) chloride hexahydrate (86.0 mg, CAS:15230-79-2) and ammonium acetate (17 mg, CAS:631-61-8) dissolved in water (4420 µL). The reaction mixture was stirred at 90°C for 1 hour.

The resulting reaction mixture was purified using the following conditions (column: Waters XSelect (registered trademark) C18 5 µm 30 × 150 mm; mobile phase: A = 1.0% AcOH (in water), B = 1.0% AcOH (in MeCN); temperature: 40°C; gradient (%B): 5.0-27% over 3 min, 27-32% over 8 min, 32-60% over 1 min; flow rate: 45 ml/min). The target product-containing fractions were collected and lyophilized to obtain the peptide of interest.

The purity of the target product was calculated from the area ratio of LC/MS (UV wavelength: 225 nm) chromatogram under the analytical conditions. The purity of the target product was 99.4%.
Analytical condition A: retention time = 3.48 min
ESI-MS(+): observed m/z = 774.79 (M+3H)³⁺

### Example 1-8: Synthesis of Conjugate No. 51

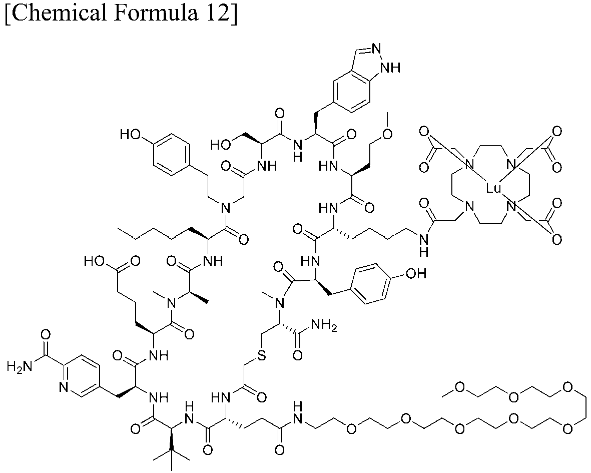

Sieber amide resin (WATANABE CHEMICAL INDUSTRIES, LTD., 0.60 mmol/g, 0.584 g) was used to synthesize the target peptide (sequence: ClAc-de(PEG8Me)-Tbg-3Py6CON-Hgl-Meda-Ahp-pHPeG-S-5Inda-HseMe-dk-Y-MeC) (SEQ ID No. 55). At that time, CEM's Liberty Blue was used as a solid-phase synthesizer for synthesis according to the manufacturer's instruction.

For removal of Fmoc on the solid-phase resin, the reaction was carried out once with 10% pyrrolidine (in DMF) at 90°C for 1 min.

For the introduction of each residue, 0.21 M Fmoc-AA (in DMF)/1.0 M DIC (in DMF)/0.5 M Oxyma pure (in DMF) (4.2 equivalents/8 equivalents/4 equivalents) were used per equivalent of resin, and the reaction was carried out once for 3 min at 90°C. For the introduction of residue 3, 0.21 M Fmoc-AA (in NMP)/1.0 M DIC (in DMF)/0.5 M Oxyma pure (in DMF) (4.2 equivalents/8 equivalents/4 equivalents) were used, and for each of residues 4, 6, and 12, the reaction was carried out twice for 10 min at 90°C. The reaction was carried out once for residue 13 at 40°C for 30 min. For residue 1, Fmoc-de(Allyl)-OH was used. The resulting solid-phase resin was shaken with 0.19 M Fmoc-OSu (in DMF, 5 equivalents) at room temperature for 1 hour, and then washed with DMF. The side chain of residue 1 was deprotected in DCM (15 mL) using tetrakis(triphenylphosphine)palladium(0) (CAS: 14221-01-3)/phenylsilane (CAS: 694-53-1) (0.2 equivalents/10 equivalents) per equivalent of resin. The reaction was carried out once for 60 min at room temperature. After deprotection of Allyl and washing with DCM and DMF, the elongation reaction from the side chain was carried out once, using H-PEG8Me/DIC/Oxyma pure (4.2 equivalents/8 equivalents/4 equivalents) per equivalent of resin obtained, in DMF (13 mL) at 75°C for 30 min.

After the introduction of each residue, the reaction was carried out once for 1 min at 90°C with 4% pyrrolidine + 83 mM Oxyma pure (in DMF) on the solid-phase resin having the Fmoc-protected peptide immobilized for removal of the Fmoc group. For each of residues 4, 6, and 12, the reaction was carried out, using 10% pyrrolidine-containing DMF solution, twice at room temperature for 1 min.

The Fmoc group of the α-amino group was removed by the reaction performed twice at room temperature for 1 min using 10% pyrrolidine-containing DMF solution for the solid-phase resin, on which the Fmoc-protected peptide obtained in the previous step was immobilized. This was further shaken with 0.19 M ClAcNHS (5 equivalents in DMF) for 60 min at room temperature to introduce the chloroacetyl group.

For the deprotection of side chains and the cleavage from the solid-phase resin, the resin obtained after the chloroacetyl group introduction step was first washed once with DMF, methylene chloride, and diethyl ether, and then dried under reduced pressure. The reaction agent cocktail (16 mL; a mixture of TFA/H₂O/TIS/DODT at a volume ratio of 92.5:2.5:2.5:2.5) was then added to a reaction vessel, which was shaken at room temperature for 30 min. The reaction solution was collected by filtration through a frit. The solid-phase resin remaining in the reaction vessel was shaken again with the cocktail for cleavage, and the solution component was collected from the frit and mixed with the above filtrate. When the filtrate was divided into 3 portions and each was added to 40 mL of a diethyl ether/hexane (1/1) mixed solvent, a precipitate occurred.

The resulting mixture was centrifuged, and the solution was then decanted. The resulting solid was washed again with diethyl ether and then dried under reduced pressure. The solid (peptide) obtained was collectively used in the next cyclization reaction. The cyclization reaction of the peptide was performed by dissolving the peptide in water/acetonitrile (1/1) to a final concentration of 3.0 mM based on the number of moles of the solid-phase resin; adding 10 equivalents of triethylamine; and stirring the mixture at room temperature for 15 hours. The reaction was stopped by adding acetic acid to the reaction solution. The resulting reaction solution was concentrated under reduced pressure by using Genevac EZ-II Elite.

The crude product obtained above was purified under the following conditions (column: Waters XSelect (registered trademark) C18 5 µm, 50 × 250 mm; mobile phase: A = 1.0% AcOH (in water) and B = 1.0% AcOH (in MeCN); temperature: 50°C; gradient (%B): 0-0% over 5.1 min, 0-4.2% over 1.9 min, 4.2-25.6% over 3 min, 25.6-30.7% over 15.5 min, 30.7-60% over 1.5 min, and 60-90% over 4 min; flow rate: 18-18 mL/min over 8 min, 18-118 mL/min over 2 min, then 118 mL/min). The target product-containing fractions were collected and lyophilized to obtain a cyclic peptide.

The cyclic peptide obtained was used as a raw material and subjected to the same reaction and purification conditions as in Example 1-7 to prepare the target peptide.

The purity of the target product was calculated from the area ratio of LC/MS (UV wavelength: 225 nm) chromatogram under the analytical conditions. The purity of the target product was 98.5%.
Analytical condition A: retention time = 4.12 min
ESI-MS(+): observed m/z = 915.83 (M+3H)³⁺

### Example 1-9: Synthesis of Conjugate No. 35

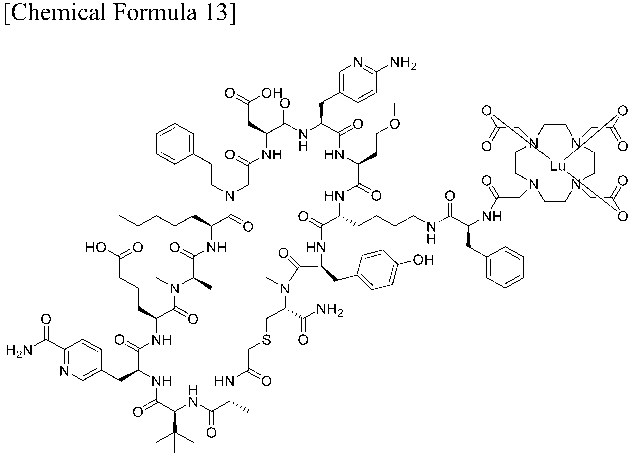

Sieber amide resin (WATANABE CHEMICAL INDUSTRIES, LTD., 0.54 mmol/g, 1.39 g) was used to synthesize the target peptide (sequence: ClAc-da-Tbg-3Py6CON-Hgl-Meda-Ahp-PeG-D-3Py6NH2-HseMe-dk(F)-Y-MeC) (SEQ ID No. 56). At that time, CEM's Liberty Blue was used as a solid-phase synthesizer for synthesis according to the manufacturer's instruction.

For removal of Fmoc on the solid-phase resin, the reaction was carried out once with 10% pyrrolidine (in DMF) at 75°C for 3 min.

For the introduction of each residue, 0.21 M Fmoc-AA (in DMF)/1.0 M DIC (in DMF)/0.5 M Oxyma pure (in DMF) (4.2 equivalents/8 equivalents/4 equivalents) were used per equivalent of resin, and the reaction was carried out once for 10 min at 75°C. For the introduction of residue 3, 0.21 M Fmoc-AA (in NMP)/1.0 M DIC (in DMF)/0.5 M Oxyma pure (in DMF) were used, and for the introduction of residue 9, 0.21 M Fmoc-AA (in NMP)/0.5 M HATU (in DMF)/1.0 M DIEA (in DMF) (4.2 equivalents/4 equivalents/8 equivalents) were used. The reaction was carried out once for residue 3 at 75°C for 30 min. Meanwhile, the reaction was carried out twice for residues 4, 6, and 12 at 75°C for 10 min. The reaction was carried out once for residue 13 at 50°C for 15 min. For residue 11, Fmoc-dk(Alloc)-OH was used. The resulting solid-phase resin was shaken with 0.38 M Fmoc-OSu (in DMF, 5 equivalents) at room temperature for 1 hour, and then washed with DMF. The side chain of residue 11 was deprotected in DCM/HFIP (30 mL/0.3 mL) using tetrakis(triphenylphosphine)palladium(0) (CAS: 14221-01-3)/phenylsilane (CAS: 694-53-1) (0.2 equivalents/10 equivalents) per equivalent of resin. The reaction was carried out once for 60 min at room temperature. After deprotection of Alloc and washing with DCM and DMF, the elongation reaction from the side chain was carried out once, using Boc-F-OH/HATU/DIEA (4.2 equivalents/4 equivalents/8 equivalents) per equivalent of resin obtained, in DMF (10 mL) at 75°C for 30 min.

After the introduction of each residue, the reaction was carried out twice for 5 min at room temperature with 10% pyrrolidine (in DMF) on the solid-phase resin having the Fmoc-protected peptide immobilized for removal of the Fmoc group. For each of residues 9, 10, 11, 12, and 13, the reaction was carried out once for 3 min at 75°C with 4% pyrrolidine + 83 mM Oxyma pure (in DMF).

The Fmoc group of the α-amino group was removed by the reaction performed once at room temperature for 1 min using 10% piperidine-containing DMF solution for the solid-phase resin, on which the Fmoc-protected peptide obtained in the previous step was immobilized. This was further shaken with 0.25 M ClAcNHS (10 equivalents in DMF) for 60 min at room temperature to introduce the chloroacetyl group.

For the deprotection of side chains and the cleavage from the solid-phase resin, the resin obtained after the chloroacetyl group introduction step was first washed once with DMF, methylene chloride, and diethyl ether, and then dried under reduced pressure. Subsequently, the solid-phase resins was divided into 3 portions, and each was put into a reaction vessel. The reaction agent cocktail (12 mL; a mixture of TFA/H₂O/TIS/DODT at a volume ratio of 92.5:2.5:2.5:2.5) was added thereto, and the reaction vessel was shaken at room temperature for 40 min. The reaction solution was collected by filtration through a frit. The solid-phase resin remaining in the reaction vessel was shaken again with the cocktail for cleavage, and the solution component was collected from the frit and mixed with the above filtrate. When the filtrate was divided into 2 portions and each was added to 30 mL of a diisopropylether/hexane (1/1) mixed solvent, a precipitate occurred.

The resulting mixture was centrifuged, and the supernatant was then decanted. The resulting solid was washed again with diethyl ether and then dried under reduced pressure. The solid (peptide) obtained was collectively used in the next cyclization reaction. The cyclization reaction of the peptide was performed by dissolving the peptide in DMSO/water/acetonitrile (1/2/2) to a final concentration of 5.0 mM based on the number of moles of the solid-phase resin; adding 10 equivalents of triethylamine; and stirring the mixture at room temperature for 1 hour. The reaction was stopped by adding acetic acid to the reaction solution. The resulting reaction solution was concentrated under reduced pressure by using Genevac EZ-II Elite.

The crude product obtained was purified using the following conditions (column: Waters XBridge (registered trademark) C18 5 µm 50 × 250 mm; mobile phase: A = 0.1% TFA (in water), B = 0.1% TFA (in MeCN); temperature: 50°C; gradient (%B): 5.3-1.1% over 0.1 min, 1.1-1.1% over 4.9 min, 1.1-5.3% over 2 min, 5.3-30.7% over 3 min, 30.7-35.8% over 15 min, 35.8-60% over 3 min; flow rate: 118-18 mL/min over 0.1 min, 18-18 mL/min over 4.9 min, 18-118 mL/min over 2 min, then 118 mL/min). The target product-containing fractions were collected and lyophilized to obtain a cyclic peptide.

The cyclic peptide obtained was used as a raw material and subjected to the same reaction and purification conditions as in Example 1-7 to prepare the target peptide.

The purity of the target product was calculated from the area ratio of LC/MS (UV wavelength: 225 nm) chromatogram under the analytical conditions. The purity of the target product was 98.7%.
Analytical condition A: retention time = 3.79 min
ESI-MS(+): observed m/z = 819.85 (M+3H)³⁺

### Example 1-10: Synthesis of Conjugate No. 38

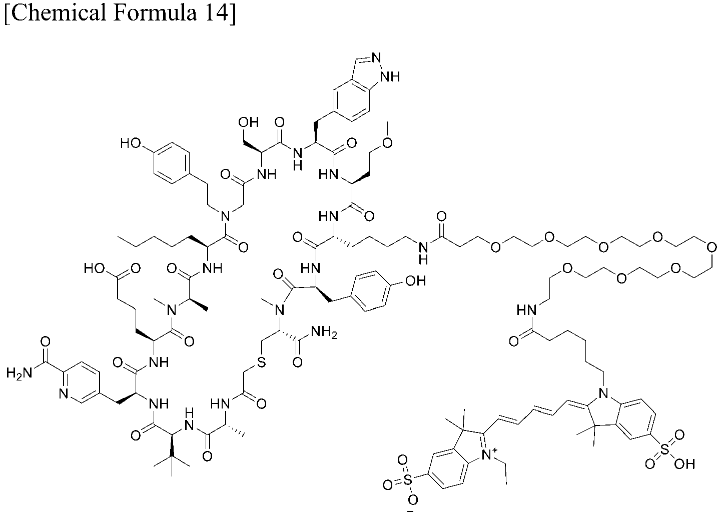

Sieber amide resin (WATANABE CHEMICAL INDUSTRIES, LTD., 0.60 mmol/g, 0.21 g) was used to synthesize the target peptide (sequence: ClAc-da-Tbg-3Py6CON-Hgl-Meda-Ahp-pHPeG-S-5Inda-HseMe-dk(PEG8c)-Y-MeC) (SEQ ID No. 57). At that time, CEM's Liberty Blue was used as a solid-phase synthesizer for synthesis according to the manufacturer's instruction.

For removal of Fmoc on the solid-phase resin, the reaction was carried out once with 10% pyrrolidine (in DMF) at 90°C for 1 min.

For the introduction of each residue, 0.21 M Fmoc-AA (in DMF)/1.0 M DIC (in DMF)/0.5 M Oxyma pure (in DMF) (4.2 equivalents/8 equivalents/4 equivalents) were used per equivalent of resin, and the reaction was carried out once for 3 min at 90°C. For residue 11, Alloc-dk(Fmoc)-OH was used. After elongation from residue 13 to residue 11 and removal of the side chain Fmoc group, Boc-PEG8c-OH was used for the elongation reaction from the side chain. The side chain of the Alloc group of the main chain was deprotected in DCM/HFIP (5 mL/0.1 mL) using tetrakis(triphenylphosphine)palladium(0) (CAS: 14221-01-3)/dimethylamineborane (CAS: 74-94-2) (0.2 equivalents/10 equivalents) per equivalent of resin. The reaction was carried out once for 60 min at room temperature. Subsequent elongation reactions were performed on the obtained solid-phase resin. For the introduction of residue 9, 0.21 M Fmoc-AA (in NMP)/1.0 M DIC (in DMF)/0.5 M Oxyma pure (in DMF) (4.2 equivalents/8 equivalents/4 equivalents) were used, and for each of residues 4, 6, 7, and 12, the reaction was carried out twice for 10 min at 90°C. The reaction was carried out once for residue 13 at 40°C for 20 min.

After the introduction of each residue, the reaction was carried out twice for 1 min at room temperature with 10% pyrrolidine (in DMF) on the solid-phase resin having the Fmoc-protected peptide immobilized for removal of the Fmoc group. For each of the side chains of residues 10 and 11, the reaction was carried out once for 1 min at 90°C with 4% pyrrolidine + 83 mM Oxyma pure (in DMF).

The Fmoc group of the α-amino group was removed by the reaction performed twice at room temperature for 1 min using 10% piperidine-containing DMF solution for the solid-phase resin, on which the Fmoc-protected peptide obtained in the previous step was immobilized. This was further shaken with 0.16 M ClAcNHS (5 equivalents in DMF/DCM = 1/1) for 60 min at room temperature to introduce the chloroacetyl group.

For the deprotection of side chains and the cleavage from the solid-phase resin, the resin obtained after the chloroacetyl group introduction step was first washed once with DMF, methylene chloride, and diethyl ether, and then dried under reduced pressure. The reaction agent cocktail (3 mL; a mixture of TFA/H₂O/TIS/DODT at a volume ratio of 90:2.5:2.5:5.0) was then added to a reaction vessel, which was shaken at room temperature for 120 min. The reaction solution was collected by filtration through a frit. The solid-phase resin remaining in the reaction vessel was shaken again with the cocktail for cleavage, and the solution component was collected from the frit and mixed with the above filtrate. When the filtrate was added to 35 mL of a diethyl ether/hexane (1/1) mixed solvent, a precipitate occurred.

The resulting mixture was centrifuged, and the supernatant was then decanted. The resulting solid was washed again with diethyl ether and then dried under reduced pressure. The solid (peptide) obtained was used in the next cyclization reaction. The cyclization reaction of the peptide was performed by dissolving the peptide in water/acetonitrile (1/1) to a final concentration of 5.0 mM based on the number of moles of the solid-phase resin; adding 10 equivalents of triethylamine; and stirring the mixture at room temperature for 1 hour. The reaction was stopped by adding acetic acid to the reaction solution. The resulting reaction solution was concentrated under reduced pressure by using Genevac EZ-II Elite.

The resulting residue was dissolved in DMSO/water (9/1) to a final concentration of 25 mM based on the number of moles of the solid-phase resin obtained, DIEA/SulfoCy5-NHS ester (5 equivalents/1.2 equivalents) were added, and the mixture was then stirred at room temperature for 1 hour. The reaction was stopped by adding acetic acid to the reaction solution.

The resulting reaction mixture was purified using the following conditions (column: Waters XSelect (registered trademark) C18 5 µm 19 × 150 mm; mobile phase: A = 0.1% TFA (in water), B = 0.1% TFA (in MeCN); temperature: 60°C; gradient (%B): 12-37% over 3 min, 37-42% over 8 min, 42-60% over 1 min; flow rate: 17 ml/min). The target product-containing fractions were collected and lyophilized to obtain the peptide of interest. The purity of the target product was calculated from the area ratio of LC/MS (UV wavelength: 225 nm) chromatogram under the analytical conditions. The purity of the target product was 96.0%.
Analytical condition A: retention time = 5.12 min
ESI-MS(+): observed m/z = 942.68 (M+3H)³⁺

### Example 1-11: Synthesis of Conjugate No. 67

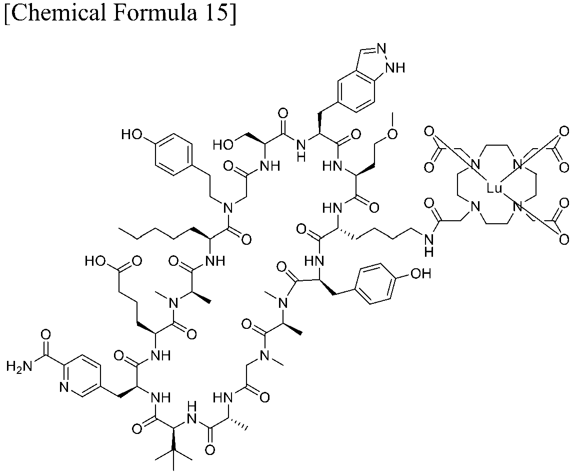

H-Ser(tBu)-Trt(2-Cl) resin (WATANABE CHEMICAL INDUSTRIES, LTD., 0.78 mmol/g, 1.28 g) was used to synthesize the target peptide (sequence: da-Tbg-3Py6CON-Hgl-Meda-Ahp-pHPeG-S-5Inda-HseMe-dk-Y-MeA-MeG) (SEQ ID No. 58). At that time, CEM's Liberty Blue was used as a solid-phase synthesizer for synthesis according to the manufacturer's instruction.

For the introduction of each residue, 0.21 M Fmoc-AA (in DMF)/0.5 M HATU (in DMF)/1.0 M DIPEA (in DMF) (4.2 equivalents/4 equivalents/8 equivalents) were used per equivalent of resin, and the reaction was carried out once for 30 min at room temperature. For the introduction of residue 3, 0.21 M Fmoc-AA (in NMP)/0.5 M HATU (in DMF)/1.0 M DIPEA (in DMF) (4.2 equivalents/4 equivalents/8 equivalents) were used, for the introduction of residue 9, 0.21 M Fmoc-AA (in NMP)/1.0 M DIC (in DMF)/0.5 M Oxyma pure (in DMF) (4.2 equivalents/8 equivalents/4 equivalents) were used, and for the introduction of each of residues 4, 6, 7, and 12, the reaction was carried out twice at room temperature for 30 min. The reaction was carried out once for residue 9 at room temperature for 60 min. The reaction was carried out twice for residue 13 at room temperature for 60 min.

After the introduction of each residue, the reaction was carried out twice for 5 min at room temperature with 10% pyrrolidine (in DMF) on the solid-phase resin having the Fmoc-protected peptide immobilized for removal of the Fmoc group. The reaction was carried out twice for residue 1 by using 10% pyrrolidine (in DMF) at room temperature for 1 min.

For the cleavage from the solid-phase resin, the reaction agent cocktail HFIP/DCM (9 mL, 1/4) was added, and the mixture was shaken for 60 min at room temperature. The resulting reaction mixture was concentrated under reduced pressure by using Genevac EZ-II Elite. The residue was solidified by adding diisopropyl ether, and after centrifugation, the solution was decanted. The resulting solid was washed again with diethyl ether and then dried under reduced pressure. The resulting residue was dissolved in DMF to a final concentration of 5 mM based on the number of moles of the solid-phase resin, DIEA/HATU (5 equivalents/1.2 equivalents) were added, and the mixture was then stirred at room temperature for 1 hour. The reaction was stopped by adding acetic acid to the reaction solution. The resulting reaction mixture was concentrated under reduced pressure by using Genevac HT-12. To the resulting residue was added the reaction agent cocktail (a mixture of TFA/H₂O/TIS at a volume ratio of 95/2.5/2.5) and shaken at room temperature for 40 min. When 40 mL of diisopropyl ether was added to the reaction mixture, a precipitate occurred.

The resulting mixture was centrifuged, and the supernatant was then decanted. The resulting solid was washed again with diethyl ether and then dried under reduced pressure.

The resulting residue was purified using the following conditions (column: Waters XSelect (registered trademark) C18 5 µm 50 × 150 mm; mobile phase: A = 0.1% TFA (in water), B = 0.1% TFA (in MeCN); temperature: 40°C; gradient (%B): 5-5% over 2 min, 5-27% over 1 min, 27-32% over 8 min, 32-60% over 1 min; flow rate: 20-20 mL/min over 1 min, 20-120 mL/min over 1 min, then 120 ml/min). The target product-containing fractions were collected and lyophilized.

The cyclic peptide obtained was used as a raw material and subjected to the same reaction and purification conditions as in Example 1-7 to prepare the target peptide.

The purity of the target product was calculated from the area ratio of LC/MS (UV wavelength: 225 nm) chromatogram under the analytical conditions. The purity of the target product was 99.7%.
Analytical condition A: retention time = 4.27 min
ESI-MS(+): observed m/z = 768.69 (M+3H)³⁺

### Example 1-12: Synthesis of Conjugate No. 56

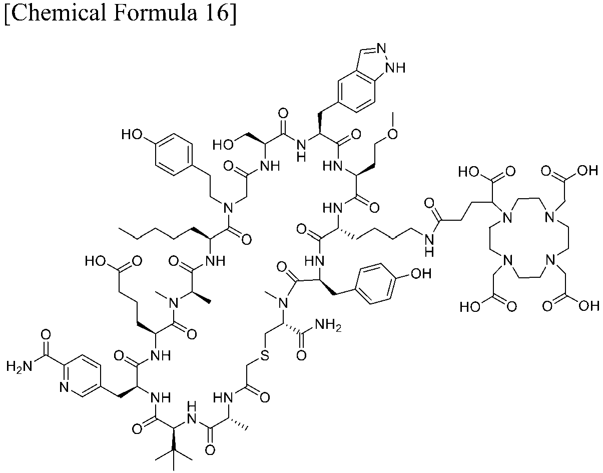

In DMSO (0.50 mL), the peptide SEQ ID No. 46 (sequence: ClAc-da-Tbg-3Py6CON-Hgl-Meda-Ahp-pHPeG-S-5Inda-HseMe-dk-Y-MeC) (20 mg) synthesized in Example 1-2 and DIEA (26.3 µL) were dissolved, and at room temperature, 2,2',2"-(10-(2,6-dioxotetrahydro-2H-pyran-3-yl)-1,4,7,10-tetraazacyclododecan-1,4,7-triyl)triacetic acid (8.1 mg, CAS: 1375475-53-8) was then added. The reaction mixture was stirred at room temperature for 5 hours.

The resulting reaction mixture was purified using the following conditions (column: Waters XBridge (registered trademark) C18 5 µm 19 × 150 mm; mobile phase: A = 0.1% TFA (in water), B = 0.1% TFA (in MeCN); temperature: 50°C; gradient (%B): 5.0-27% over 3 min, 27-32% over 8 min, 32-60% over 1 min; flow rate: 17 ml/min). The target product-containing fractions were collected and lyophilized to obtain the peptide of interest.

The purity of the target product was calculated from the area ratio of LC/MS (UV wavelength: 225 nm) chromatogram under the analytical conditions. The purity of the target product was 97.8%.
Analytical condition A: retention time = 3.40 min
ESI-MS(+): observed m/z = 741.40 (M+3H)³⁺

### Example 1-13: Synthesis of Conjugate No. 58

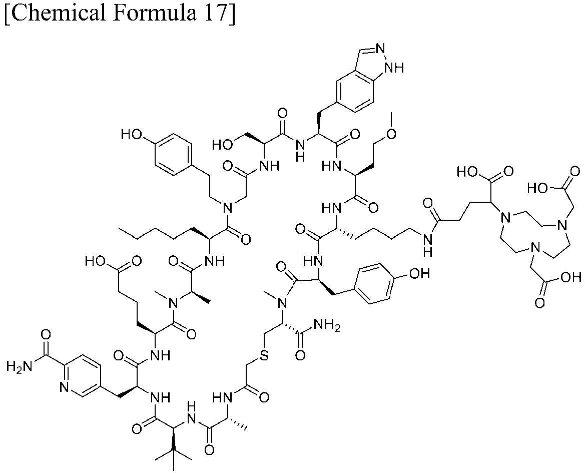

In DMSO (0.50 mL), the peptide SEQ ID No. 46 (sequence: ClAc-da-Tbg-3Py6CON-Hgl-Meda-Ahp-pHPeG-S-5Inda-HseMe-dk-Y-MeC) (20 mg) synthesized in Example 1-2 and DIEA (26.3 µL) were dissolved, and at room temperature, 2,2'-(7-(1-carboxy-4-((2,5-dioxopyrrolidin-1-yl)oxy)-4-oxobutyl)-1,4,7-triazonan-1,4-diyl)diacetic acid (14 mg, CAS: 1407166-70-4) was then added. The reaction mixture was stirred at room temperature for 7 hours.

The resulting reaction mixture was purified using the following conditions (column: Waters XBridge (registered trademark) C18 5 µm 19 × 150 mm; mobile phase: A = 0.1% TFA (in water), B = 0.1% TFA (in MeCN); temperature: 50°C; gradient (%B): 5.0-28% over 3 min, 28-33% over 8 min, 33-60% over 1 min; flow rate: 17 ml/min). The target product-containing fractions were collected and lyophilized to obtain the peptide of interest.

The purity of the target product was calculated from the area ratio of LC/MS (UV wavelength: 225 nm) chromatogram under the analytical conditions. The purity of the target product was 96.4%.
Analytical condition A: retention time = 3.70 min
ESI-MS(+): observed m/z = 1060.97 (M+2H)²⁺

### Example 1-14: Synthesis of Conjugate No. 21

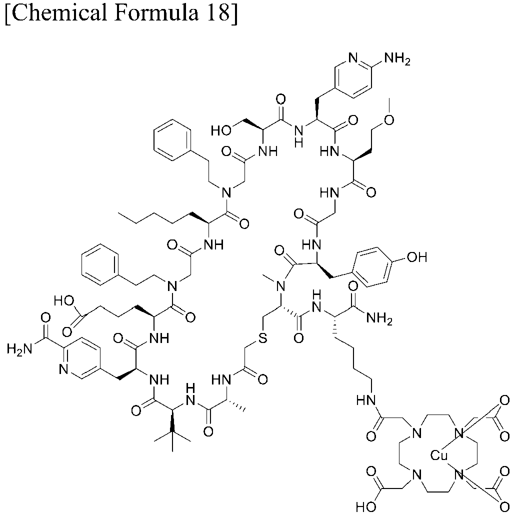

Conjugate No. 2 (10 mg) obtained by the same synthetic method as in Example 1-4 was dissolved in DMF (0.40 mL), and copper(II) chloride dihydrate (0.90 mg, CAS: 10125-13 -0) and 100 mM ammonium acetate aqueous solution (0.20 mL) were then dissolved. The reaction mixture was stirred at 40°C for 60 min.

The resulting reaction mixture was purified using the following conditions (column: Waters XSelect (registered trademark) C18 5 µm 30 × 150 mm; mobile phase: A = 1.0% AcOH (in water), B = 1.0% AcOH (in MeCN); temperature: 40°C; gradient (%B): 6.0-31% over 3 min, 31-36% over 8 min, 36-60% over 1 min; flow rate: 45 ml/min). The target product-containing fractions were collected and lyophilized to obtain the peptide of interest.

The purity of the target product was calculated from the area ratio of LC/MS (UV wavelength: 225 nm) chromatogram under the analytical conditions. The purity of the target product was 97.7%.
Analytical condition A: retention time = 4.32 min
ESI-MS(+): observed m/z = 768.95 (M+3H)³⁺

### Example 1-15: Synthesis of Conjugate No. 22

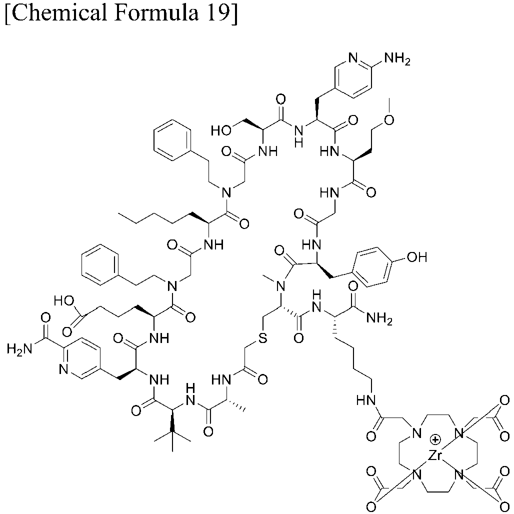

In DMF (0.40 mL), the cyclic peptide (sequence: ClAc-da-Tbg-3Py6CON-Hgl-PeG-Ahp-PeG-S-3Py6NH2-HseMe-G-Y-MeC-K) (SEQ ID No. 59) (15 mg) synthesized in substantially the same synthesis method as in Example 1-1 was dissolved, and DOTA-NHS ester hexafluorophosphate trifluoroacetate (7.8 mg) and DIPEA (0.018 mL) were added under ice-cooling. The reaction mixture was stirred at room temperature for 120 min. To the reaction mixture was added zirconium(IV) acetylacetonate (5.1 mg, CAS:17501-44-9), and the mixture was stirred at 50°C for 90 min, and then DOTA (10 mg) was added.

The reaction mixture obtained above was purified under the following conditions (column: Waters XSelect (registered trademark) C18 5 µm, 50 × 250 mm; mobile phase: A = 1.0% AcOH (in water) and B = 1.0% AcOH (in MeCN); temperature: 50°C; gradient (%B): 0-0% over 5.1 min, 0-4.2% over 1.9 min, 4.2-25.6% over 3 min, 25.6-30.7% over 15.5 min, 30.7-60% over 1.5 min, and 60-90% over 4 min; flow rate: 18-18 mL/min over 5.1 min, 18-118 mL/min over 1.9 min, then 118 mL/min). The target product-containing fractions were collected and lyophilized to obtain the peptide of interest.

The purity of the target product was calculated from the area ratio of LC/MS (UV wavelength: 225 nm) chromatogram under the analytical conditions. The purity of the target product was 99.7%.
Analytical condition A: retention time = 3.66 min
ESI-MS(+): observed m/z = 1165.62 (M+H)²⁺

### Example 1-16: Synthesis of Conjugate No. 45

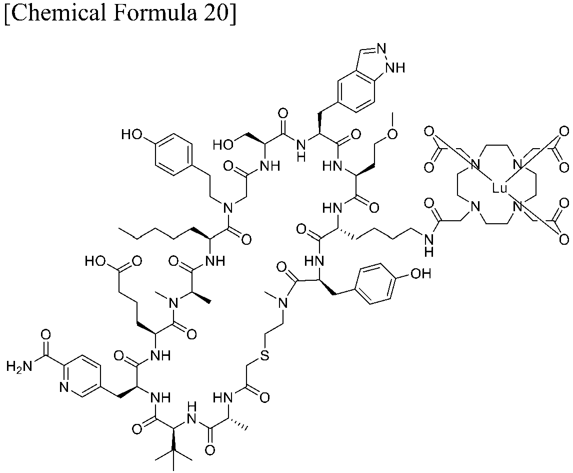

Using Cl-Trt(2-Cl) resin (WATANABE CHEMICAL INDUSTRIES, LTD.) and Fmoc-MeCt (CAS: 2932449-54-0) as starting materials, an immobilized solid-phase resin (1.007 mmol/g, 0.500 g) prepared in the same manner as described in Tetrahedron Letters, 43 (2002) 3419-3421 was used to synthesize the target peptide (sequence: ClAc-da-Tbg-3Py6CON-Hgl-Meda-Ahp-pHPeG-S-5Inda-HseMe-dk-Y-MeCt) (SEQ ID No. 60). At that time, CEM's Liberty Blue was used as a solid-phase synthesizer for synthesis according to the manufacturer's instruction. The cyclic peptide was synthesized as in Example 1-2, and the target peptide was obtained by subjecting the peptide to the same reaction and purification conditions as in Example 1-7 with respect to the introduction of a chelator and a metal.

The purity of the target product was calculated from the area ratio of LC/MS (UV wavelength: 225 nm) chromatogram under the analytical conditions. The purity of the target product was 99.5%.
Analytical condition A: retention time = 3.85 min
ESI-MS(+): observed m/z = 760.33 (M+3H)³⁺

### Example 2: Synthesis of Cyclic Peptides and Synthesis of Conjugates

According to the general method described in Example 1, each target peptide and a conjugate containing said peptide and a payload as listed in the following tables were synthesized.

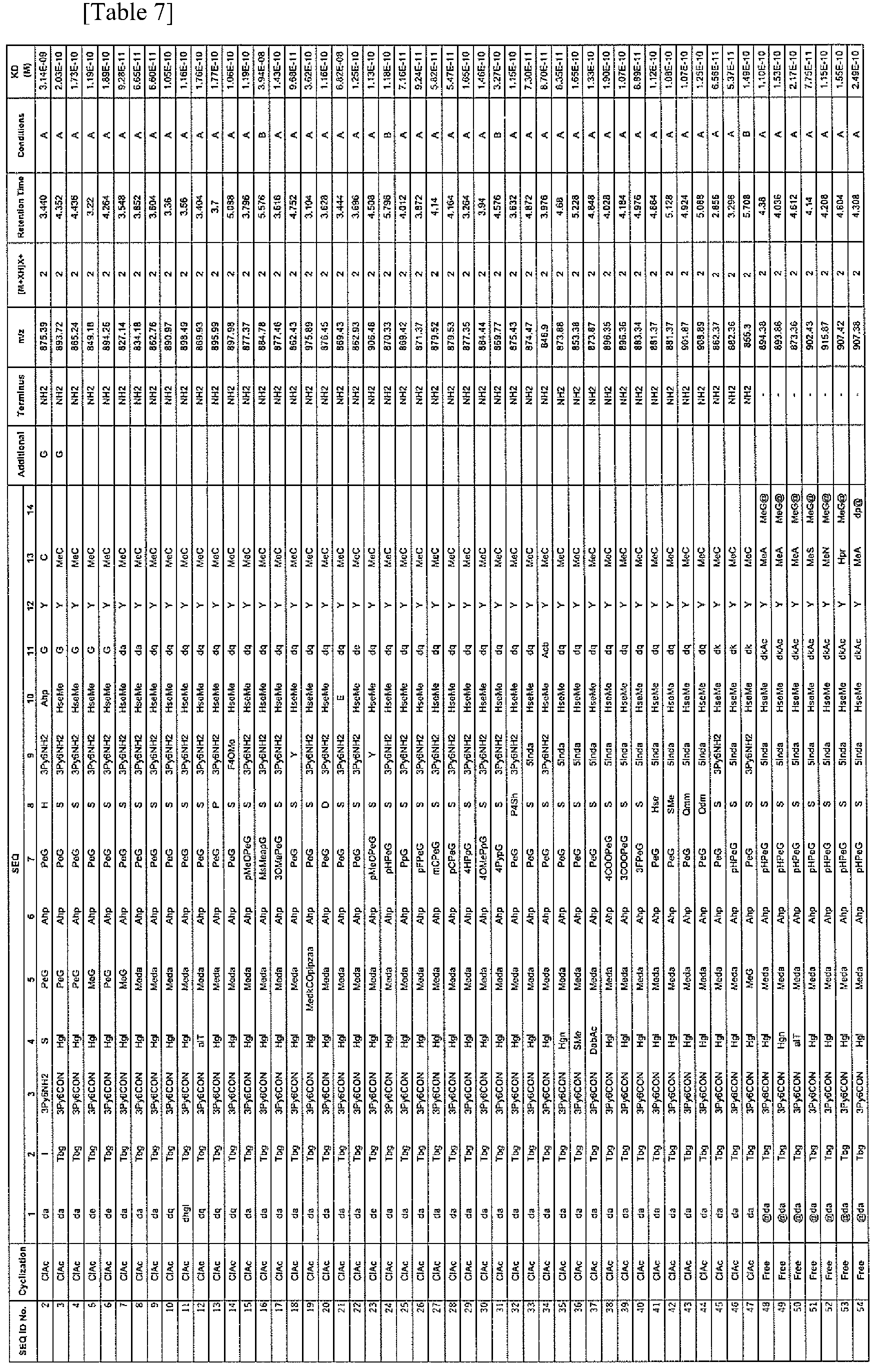

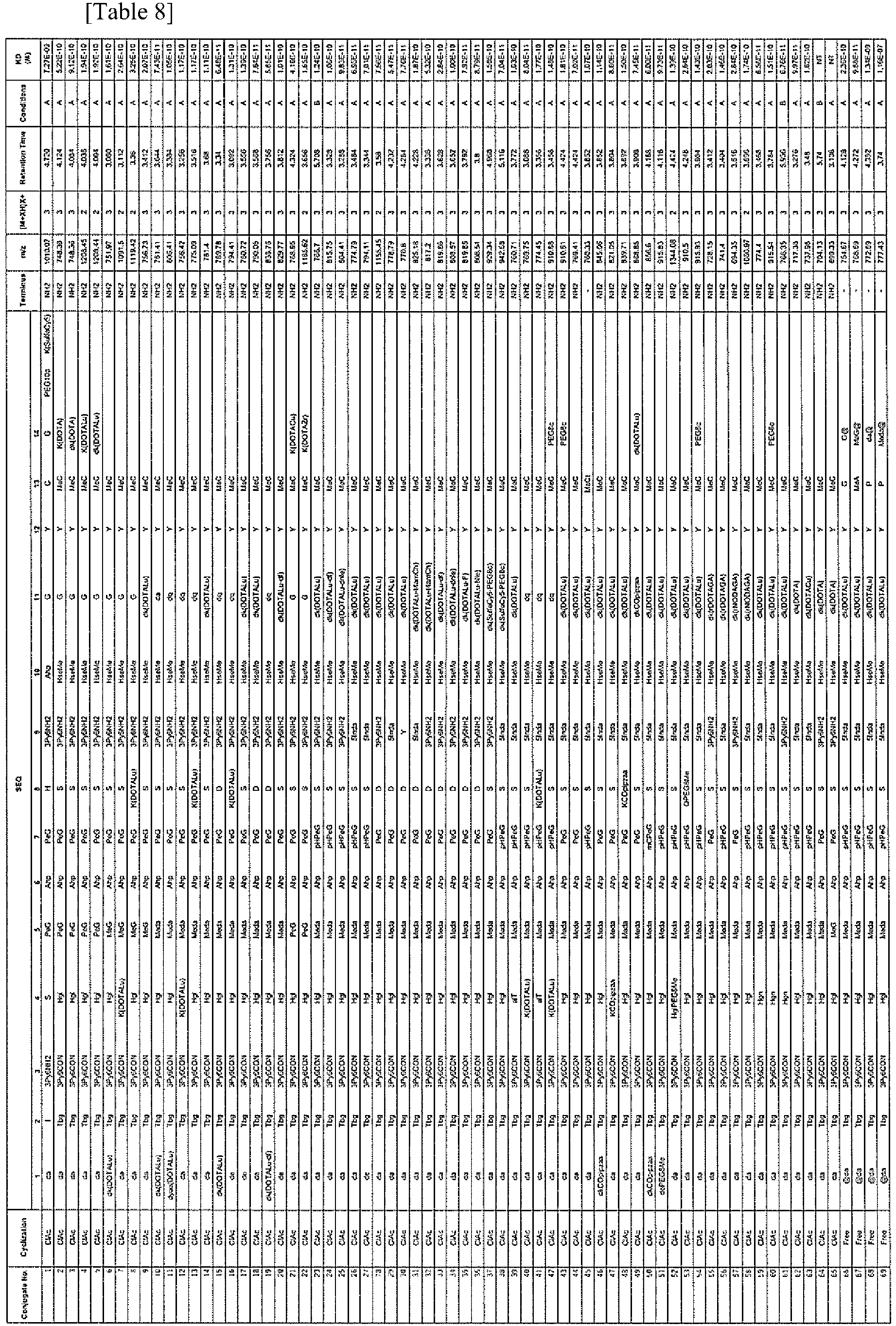

Table 7 lists the target peptides, analytical conditions, and retention times, and ESI-MS(+) observed values, and Table 8 lists the target conjugates, analytical conditions and retention times, and ESI-MS(+) observed values.

In Table 7, the "terminus" indicates the C-terminal functional group (in the tables, "-OH" indicates COOH and "-NH2" indicates CO(NH₂)), and the blank case indicates that no functional group is present at the C-terminus. With respect to "Cyclization" in the tables, entries with the description "ClAc" represent that cyclization was achieved by linking the amino acid at position 1 into which a chloroacetyl group was introduced with C or MeC present at the C-terminus, whereas the description "Free" indicates that the N-terminal amino group of the amino acid at position 1 is linked to the C-terminal carboxy group of the amino acid at position 13 or 14. In addition, the "Additional/Linker" in the tables indicates a linker or additional amino acid(s) that is not included in the cyclic structure. For example, in Ca9_No.1 (SEQ ID No. 3), the amino acid sequence X1-X13 forms a cyclic structure, and glycine is further added as a linker from the C of the C-terminal X13.

In Table 8, the same description rules are applied as in Table 7 above. Furthermore, in Table 8, the component described in parentheses () represents the payload. For example, the conjugate of Conjugate No. 1 indicates that the amino acid residues C at X1 and X13 form a cyclic structure, that a linker structure of G-PEG10c-K extends from the C at X13, and that SulfoCy5 is attached as a payload to the side chain of the terminal K. In addition, in the conjugate of Conjugate No. 6, the amino acid residues of MeC at X1 and X13 form a cyclic structure and the side chain of dk at X1 is attached to the payload, namely Lu-coordinated DOTA. Furthermore, in Conjugate No. 19 listed in Table 8, the amino acid residues of MeC at X1 and X13 likewise form a cyclic structure and the side chain of dk at X1 is attached via df to the payload, namely Lu-coordinated DOTA. Meanwhile, NT in Table 8 indicates "not tested".

### Example 3: Test of Evaluating Intermolecular Interaction of Peptide or Conjugate against Human CA9 by Surface Plasmon Resonance (SPR)

The intermolecular interactions of the peptides against human CA9 protein were examined by surface plasmon resonance (SPR) for the various peptides or conjugates synthesized in Examples 1 and 2, according to the methods described below. Specific test methods are described below.

### SPR measurement

Protein A sensor chip (Cytiva) was inserted into BiacoreT200 (Cytiva) and primed with running buffer: 1× HBS-P+ (Cytiva), 1.0 % DMSO (Fujifilm Wako Pure Chemical Corporation), and 50 µM zinc acetate dihydrate (Hampton research), and equilibrated at a flow rate of 30 µL/min. 50 nM Human CA9-Fc (SinoBiological) diluted with running buffer was reacted at a flow rate of 5 µL/min for 60 seconds and captured into a flow cell.

A solution containing each peptide or conjugate synthesized in Example 1 or 2 and prepared at 1 mM in DMSO solution was diluted with running buffer to a final concentration of 100 nM. Then, the peptide or conjugate solutions at 25 nM and 5 nM were further prepared. Depending on the affinity of the peptide or conjugate, the solution was diluted with running buffer to a final concentration of 40 nM. Then, the peptide or conjugate solutions at 10 nM and 2 nM were further prepared.

Using the above samples, the kinetics of each peptide or conjugate against human CA9 was obtained by SPR measurement. The kinetics evaluation model was Single Cycle Kinetics, and curve fitting was performed using Biacore T200 Evaluation Software Version 3.0 (Cytiva). The binding of each peptide or conjugate to human CA9 was evaluated by performing least-squares curve fitting on the obtained sensorgram to calculate the KD value.

Tables 7 and 8 show the results. Tables 7 and 8 have demonstrated that the peptides and conjugates of the present invention have the activity of binding to human CA9.

### Reference Examples: Synthesis of Non-Natural Amino Acids

The Reference Examples show examples of synthesis of various non-natural amino acids.

In the Reference Examples, the following equipment, abbreviations, analytical conditions, etc., were used unless otherwise specified.

Unless otherwise specified, the proton nuclear magnetic resonance (¹H-NMR) spectra in the following Synthesis Examples were measured using a JNM-ECP300 or JNM-ECX300 manufactured by JEOL Ltd., or an Ascend^{™} 500 manufactured by Bruker, in a deuterated chloroform or deuterated dimethyl sulfoxide solvent. The chemical shifts were expressed as δ values (ppm) with tetramethylsilane as an internal standard (0.0 ppm).

In the description of NMR spectra, "s" indicates a singlet, "d" indicates a doublet, "t" indicates a triplet, "q" indicates a quartet, "dd" indicates a doublet of doublets, "dt" indicates a doublet of triplets, "m" indicates a multiplet, "br" indicates a broad signal, "J" indicates a coupling constant, "Hz" indicates hertz, "CDCl₃" indicates deuterated chloroform, and "DMSO-d₆" indicates deuterated dimethyl sulfoxide.

High performance liquid chromatography (HPLC)/mass spectrometry (MS) was performed using either Waters ACQUITY UPLC H-Class/QDa, Waters ACQUITY UPLC H-Class/SQD2, or Shimadzu LC-20AD/Triple Tof5600.

In the description of HPLC/MS data, "ESI+" denotes the positive mode of electrospray ionization, and "(M+H)⁺" denotes a protonated molecular ion. "(M-C4H8+H)⁺" indicates an ion formed by protonation and elimination of a tert-butyl group.

In the description of HPLC/MS data, "ESI-" denotes the negative mode of electrospray ionization, and "(M-H)" denotes a deprotonated molecular ion..

### Analytical condition C

Column: ACQUITY (registered trademark) 1.7 µm BEH C18, 2.1 × 100 mm, Waters
Mobile phase A: 0.025% TFA in water
Mobile phase B: 0.025% TFA in CH₃CN
Column temperature: 60°C
Gradient (%B): 5-95% over 5.56 min, then 95% from 5.56 min to 7.22 min; flow rate: 0.6 mL/min
Detection: UV 254 nm

### Reference Example 1: Synthesis of Fmoc-4COOPeG(tBu)-OH

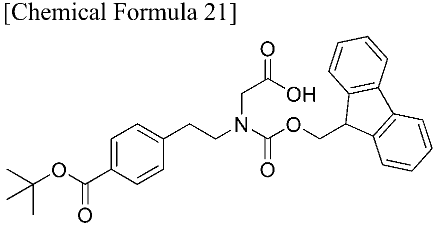

Methyl 2-aminoacetate hydrochloride (10 g, CAS: 5680-79-5) was dissolved in MeOH (200 mL, CAS: 67-56-1) at room temperature, and triethylamine (16.12 g, CAS: 121-44-8) and *tert-butyl* acrylate (20.42 g, CAS: 1663-39-4) were added. The reaction mixture was stirred at room temperature for 2 hours, and then concentrated under reduced pressure. The resulting residue (15 g) was dissolved in DCM (150 mL, CAS: 75-09-2), and DIPEA (35.69 g, CAS: 7087-68-5) and N-[(9H-fluoren-9-ylmethoxy)carbonyloxy]succinimide (22.12 g, CAS: 82911-69-1) were added thereto under ice-cooling. After stirring at room temperature for 2 hours, 1M hydrochloric acid (300 mL, CAS: 7647-01-0) was added to stop the reaction. After extraction with DCM, the resulting organic layer was washed with saturated saline and dried over sodium sulfate. The organic layer was filtered and then concentrated under reduced pressure, and the resulting residue was purified by silica gel column chromatography (petroleum ether/ethyl acetate = 1/1). The target-product-containing fractions were then concentrated under reduced pressure.

The product (31 g) obtained in the above reaction was dissolved in DCM (300 mL), and a 4 M HCl solution in 1,4-dioxane (300 mL) was added thereto under ice-cooling. The reaction mixture was stirred at room temperature for 2 hours, and then concentrated under reduced pressure. The resulting residue (25 g) was dissolved in DCM (150 mL) at room temperature, and 1-(3-dimethylaminopropyl)-3-ethylcarbodiimide hydrochloride (18.75 g, CAS: 25952 -53-8) and N-hydroxyphthalimide (11.70 g, CAS: 524-38-9) were added. The reaction mixture was stirred at room temperature for 2 hours. The reaction was then stopped by adding saturated aqueous sodium bicarbonate (200 mL). The resulting mixture was washed with saturated saline and dried over sodium sulfate. The mixture was filtered and then concentrated under reduced pressure, and the resulting residue was purified by silica gel flash column chromatography (petroleum ether/ethyl acetate = 1/1). The target-product-containing fractions were then concentrated under reduced pressure.

Under a nitrogen atmosphere, nickel(II) bromide trihydrate (4.64 g, CAS: 7789-49-3) and 4,4'-di-tert-butyl-2,2'-dipyridyl (4.57 g, CAS: 72914-19-3) were added to N,N-dimethylacetamide (300 mL, CAS: 127-19-5), and the mixture was stirred at room temperature for 15 min. tert-Butyl 4-bromobenzoate (29.19 g, CAS: 59247-47-1) and the product (30 g) obtained in the previous step were added to the resulting mixture, and zinc (18.56 g, CAS: 7440-66-6) was added thereto over 10 min at room temperature. The reaction mixture was stirred at room temperature for 30 min. The reaction was stopped by adding water to the mixture, and the insoluble material was filtered and washed with ethyl acetate. The filtrate was extracted with ethyl acetate, and the resulting organic layer was washed with saturated saline and dried over sodium sulfate. The organic layer was filtered and then concentrated under reduced pressure, and the resulting residue was purified by silica gel flash column chromatography (petroleum ether/ethyl acetate = 1/1). The target-product-containing fractions were then concentrated under reduced pressure.

The product obtained (20 g) was dissolved in isopropyl alcohol (600 mL, CAS: 67-63-0), and an aqueous solution containing calcium chloride (68.88 g, CAS: 10043-52-4) (150 mL) and an aqueous solution containing lithium hydroxide (3.72 g, CAS: 1310-65-2) (50 mL) were added thereto under ice-cooling. The reaction mixture was stirred at room temperature for 16 hours. A saturated aqueous solution containing sodium dihydrogen phosphate (CAS: 7558-80-7) was added to the mixture to adjust the pH to 5, and the mixture was then extracted with ethyl acetate. The combined organic layer was washed with saturated saline and then dried over sodium sulfate. After filtration and concentration under reduced pressure, the resulting residue was purified by reversed-phase silica gel flash column chromatography (column: C18 silica gel; gradient: 10 mM ammonium bicarbonate aqueous solution/acetonitrile = 95/5 to 0/100). The target product-containing fractions were concentrated under reduced pressure to afford the title compound (10.1 g).

1H NMR (500 MHz, DMSO-d6) δ 12.68 (br, 1H), 7.92-7.86 (m, 2H), 7.86-7.55 (m, 4H), 7.47-7.00 (m, 6H), 4.52-4.45 (m, 1H), 4.28-4.14 (m, 2H), 4.00-3.69 (m, 2H), 3.56-3.07 (m, 2H), 2.91-2.41 (m, 2H), 1.55-1.51 (m, 9H).

Analytical condition C: retention time = 5.44 min; ESI-MS(+) observed m/z = 446 (M-C4H8+H)⁺, theoretical m/z = 501.

### Reference Example 2: Synthesis of Fmoc-3COOPeG(tBu)-OH

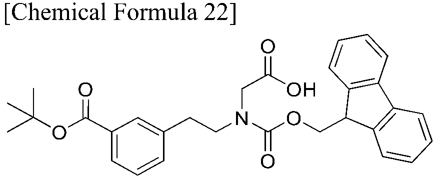

Fmoc-3COOPeG(tBu)-OH was obtained by substantially the same synthesis method as in Reference Example 1: Fmoc-4COOPeG(tBu)-OH. However, instead of *tert*-butyl 4-bromobenzoate, *tert*-butyl 3-bromobenzoate (CAS: 69038-74-0) was used as a raw material.

1H NMR (500 MHz, DMSO-d6) δ 12.70 (br, 1H), 7.92-7.82 (m, 2H), 7.81-7.55 (m, 4H), 7.52-7.10 (m, 6H), 4.51-4.32 (m, 1H), 4.32-4.09 (m, 2H), 4.09-3.74 (m, 2H), 3.53-3.12 (m, 2H), 2.92-2.46 (m, 2H), 1.55-1.47 (m, 9H).

Analytical condition C: retention time = 5.19 min; ESI-MS(+) observed m/z = 446 (M-C4H8+H)⁺, theoretical m/z = 501.

### Example 4: In Vivo Distribution of ⁶⁷Ga-Labeled Conjugate

### Peptide ⁶⁷Ga-Labeling

The following conjugates were synthesized by the method in Example 1or 2 or substantially the same method:
Conjugate No.65,
Conjugate No.64,
Conjugate No. 70 (the payload moiety of Conjugate No. 18 is a DOTA-only, lutetium-uncoordinated moiety in the conjugate (sequence: ClAc-de-Tbg-3Py6CON-Hgl-Meda-Ahp-PeG-D-3Py6NH2-HseMe-dk(DOTA)-Y-MeC) (DOTA attached to the side chain of dk at position 11 of the amino acid sequence set forth in SEQ ID No. 73)),
Conjugate No. 71 (the payload moiety of Conjugate No. 29 is a DOTA-only, lutetium-uncoordinated moiety in the conjugate (sequence: ClAc-da-Tbg-3Py6CON-Hgl-Meda-Ahp-PeG-D-5Inda-HseMe-dk(DOTA)-Y-MeC) (DOTA attached to the side chain of dk at position 11 of the amino acid sequence set forth in SEQ ID No. 77)),
Conjugate No. 72 (the payload moiety of Conjugate No. 15 is a DOTA-only, lutetium-uncoordinated moiety in the conjugate (sequence: ClAc-dk(DOTA)-Tbg-3Py6CON-Hgl-Meda-Ahp-PeG-D-3Py6NH2-HseMe-dq-Y-MeC) (DOTA attached to the side chain of dk at position 1 of the amino acid sequence set forth in SEQ ID No. 70)),
Conjugate No.62,
   and
Conjugate No. 73 (the payload moiety of Conjugate No. 31 is a DOTA-t4amCh-only, lutetium-uncoordinated moiety in the conjugate (sequence: ClAc-da-Tbg-3Py6CON-Hgl-Meda-Ahp-PeG-D-5Inda-HseMe-dk(DOTA-t4amCh)-Y-MeC) (DOTA attached via t4amCh to the side chain of dk at position 11 of the amino acid sequence set forth in SEQ ID No. 77)).

The labeling reaction solution (Table 9) containing each conjugate was heated at 95°C for 10 min for ⁶⁷Ga-labeling.

**[Table 9]**

| Table 9. Composition of labeling reaction solution | |
|---|---|
| Conjugate | 2.8 nmol/50 µL |
| DMSO | 1.43 - 1.69 µL/50 µL |
| Gallium chloride (⁶⁷Ga) | 10.36 MBq (test at 12:00 on labeling day) |
| Sodium acetate hydrate | 0.40 mol/L |
| Acetic acid | 0.27 mol/L |
| Ascorbic acid | 5.6 mg/mL |
| Gentisic acid | 1.28 mg/mL |

### Radio HPLC

Each ⁶⁷Ga-labeled conjugate (hereafter, also referred to as "test substance") synthesized as described above was mixed with acetonitrile, and the radiochemical purity was measured before and after administration while using TSK gel (registered trademark) ODS-80Ts QA, 4.6 mm ID × 250 mm, 5 µm (Tosoh Corporation). The results show that the purity was 95.61-91.27%.

### Radio TLC

TLC Silica Gel 60 RP-18 F₂₅₄s; Merck, 115389 spotted with the test substance was developed with a mixed solution of 2 mol/L ammonium acetate solution/acetone (1:1). Thereafter, radioactivity was measured using a radiochromatograph (energy range: 60-350 keV; collimator: 1.0 cm; V-shaped BGO crystal; measurement time: 10 min). The results show that the purity was 96.67-94.27%.

### Preparation of Experimental Animals (HT-29 Tumor-bearing Mice)

Five-week-old female BALB/cSlc-nu/nu mice were purchased from Japan SLC (Shizuoka, Japan) and used for the preparation of experimental animals. After acclimatization, CA9-positive human colon cancer-derived HT-29 cells were suspended at 5 × 10⁷ cells/mL in a 1:2 mixture of PBS (pH 7.4, 1×; Thermo Fisher Scientific, 10010-023) and VitroGel (The Well Bioscience, VHM01). A 0.1-mL portion of the suspension was subcutaneously inoculated into the right flank of (6-week-old) nude mice by using a disposable syringe with a needle.

The number of animals evaluated was 3 per group, for a total of 12 groups. Animals evaluated at the time of use weighed 18.2 g to 20.7 g and had tumor sizes of 216 to 908 mm³.

### In Vivo Distribution

Each test substance was administered as a single intravenous dose to HT-29 tumor-bearing mice. Autopsies were performed at 4, 24, and 72 hours after administration, and the radioactivity concentration (%ID/g) of each tissue was measured.

### Results

The results of the *in vivo* distribution of each ⁶⁷Ga-labeled compound are shown in Fig. 1, Fig. 2, and Fig. 3. Note that the "Conjugate No. XX" in the figure indicates each conjugate whose payload moiety in the conjugate described in the corresponding Conjugate No. is DOTA-⁶⁷Ga.
Fig. 1: Tissue distribution at 4 hours post-administration: %ID/g (mean ± SD, n = 3)
Fig. 2: Tissue distribution at 24 hours post-administration: %ID/g (mean ± SD, n = 3)
Fig. 3: Tissue distribution at 72 hours post-administration: %ID/g (mean ± SD, n = 3)

### Example 5: In Vivo Distribution and PET Imaging of ⁶⁴Cu Labeled Conjugate

### Peptide Conjugate ⁶⁴Cu-Labeling

The labeling reaction solution (Table 10) containing each peptide conjugate synthesized in Examples 1 or 2: Conjugate No. 64, Conjugate No. 62, Conjugate No. 2, Conjugate No. 56, or Conjugate No. 58 was heated at 95°C for 10 min for ⁶⁴Cu-labeling.

**[Table 10]**

| Table 10. Composition of labeling reaction solution | |
|---|---|
| Conjugate | 2.8 nmol/50 µL |
| DMSO | 1.43 - 1.64 µL/50 µL |
| Copper chloride (⁶⁴Cu) | 31.08 MBq (test at 12:00 on the day of administration) |
| Sodium acetate hydrate | 0.31 mol/L |
| Acetic acid | 0.21 mol/L |
| Ascorbic acid | 5.6 mg/mL |
| Gentisic acid | 1.28 mg/mL |

### Radio HPLC

Each ⁶⁴Cu-labeled conjugate (hereafter, also referred to as "test substance") synthesized as described above was mixed with acetonitrile, and the radiochemical purity was measured before and after administration while using TSK gel (registered trademark) ODS-80Ts QA, 4.6 mm ID × 250 mm, 5 µm (Tosoh Corporation). The results show that the purity was 94.14-100%.

### Radio TLC

TLC Silica Gel 60 RP-18 F₂₅₄s; Merck, 115389 spotted with the test substance was developed with a mixed solution of 2 mol/L ammonium acetate solution/acetone (1:1). Thereafter, radioactivity was measured using a radiochromatograph (energy range: 60-350 keV; collimator: 1.0 cm; V-shaped BGO crystal; measurement time: 10 min). The results show that the purity was 97.60-98.79%.

### Preparation of Experimental Animals (HT-29 Tumor-bearing Mice)

Five-week-old female BALB/cSlc-nu/nu mice were purchased from Japan SLC (Shizuoka, Japan) and used for the preparation of experimental animals. After acclimatization, CA9-positive human colon cancer-derived HT-29 cells were suspended at 5 × 10⁷ cells/mL in a 1:2 mixture of PBS (pH 7.4, 1×; Thermo Fisher Scientific, 10010-023) and VitroGel (The Well Bioscience, VHM01). A 0.1-mL portion of the suspension was subcutaneously inoculated into the right flank of (6-week-old) nude mice by using a disposable syringe with a needle.

The number of animals evaluated was 3 per group, for a total of 12 groups. Animals evaluated at the time of use weighed 17.7 g to 21.5 g and had tumor sizes of 165 to 523 mm³.

### In Vivo Distribution and PET Imaging

Each test substance was administered as a single intravenous dose of 80 µL/head to HT-29 tumor-bearing mice. Autopsies were performed at 4 and 24 hours after administration, and the radioactivity concentration (%ID/g) of each tissue was measured to evaluate the *in vivo* distribution. In addition, PET imaging was performed at 1, 4, and 23.5 hours after administration, and PET/CT images were collected on an Si78 (Bruker BioSpin MRI GmbH) under isoflurane gas anesthesia.

### Results

The results of the *in vivo* distribution of each ⁶⁴Cu-labeled compound are shown in Figs. 4 to 7.

Fig. 4: Tissue distribution at 4 hours post-administration: %ID/g (mean ± SD, n = 3)

Fig. 5: Tissue distribution at 24 hours post-administration: %ID/g (mean ± SD, n = 3)

Fig. 6: Tissue distribution at 4 hours post-administration: %ID/g (mean ± SD, n = 3)

Fig. 7: Tissue distribution at 24 hours post-administration: %ID/g (mean ± SD, n = 3)

### PET/CT Imaging

PET/CT images were collected under isoflurane gas anesthesia at 1, 4 and 23.5 hours after administration of ⁶⁴Cu-labeled compound (4.62-4.72 MBq/head). The results are shown in Figs. 8 to 13. Accumulation of each compound in tumor was observed. Note that the columns show the same individuals, e.g., the left column shows the results of the same individuals at 1 hour, 4 hours, and 23.5 hours after the administration. Meanwhile, the white arrows each indicate the localization of the implanted tumor.

Fig. 8: ⁶⁴Cu-labeled conjugate: Conjugate No. 64-⁶⁴Cu

Fig. 9: ⁶⁴Cu-labeled conjugate: Conjugate No. 62-⁶⁴Cu

Fig. 10: ⁶⁴Cu-labeled conjugate: Conjugate No. 2-⁶⁴Cu

Fig. 11: ⁶⁴Cu-labeled conjugate: Conjugate No. 56-⁶⁴Cu

Fig. 12: ⁶⁴Cu-labeled conjugate: Conjugate No. 58-⁶⁴Cu

### Example 6: Efficacy Study on ¹⁷⁷Lu-Labeled Conjugate

### Peptide ¹⁷⁷Lu-Labeling

The labeling reaction solution (Table 11) containing each conjugate synthesized in Examples 1 or 2: Conjugate No. 64, Conjugate No. 62, or Conjugate No. 65 was heated at 95°C for 10 min for ¹⁷⁷Lu-labeling.

**[Table 11]**

| Table 11. Composition of test substance | | |
|---|---|---|
| Name | Compound at Dose of 30 MBq | Compound at Dose of 60 MBq |
| Conjugate | 2.8 nmol/50 µL | 2.8 nmol/50 µL |
| DMSO | 1.43 - 1.56 µL | 1.56 µL |
| Lutetium chloride | 210 MBq/50 µL (test at 15:00 on labeling day) | 420 MBq/50 µL (test at 15:00 on labeling day) |
| Sodium acetate hydrate | 0.36 - 0.38 mol/L | 0.34 mol/L |
| Acetic acid | 0.24 - 0.25 mol/L | 0.23 mol/L |
| Ascorbic acid | 5.6 mg/mL | 5.6 mg/mL |
| Gentisic acid | 1.28 mg/mL | 1.28 mg/mL |

### Radio HPLC

Each ¹⁷⁷Lu-labeled conjugate test substance (hereafter, also referred to as "test substance") synthesized as described above was mixed with acetonitrile, and the radiochemical purity was measured before and after administration while using TSK gel (registered trademark) ODS-80Ts QA, 4.6 mm ID × 250 mm, 5 µm (Tosoh Corporation). The results show that the purity was 90.31-100%.

### Radio TLC

TLC Silica Gel 60 RP-18 F₂₅₄s; Merck, 115389 spotted with the test substance was developed with a mixed solution of 2 mol/L ammonium acetate solution/acetone (1:1). Thereafter, radioactivity was measured using a radiochromatograph (energy range: 60-350 keV; collimator: 1.0 cm; V-shaped BGO crystal; measurement time: 10 min). The results show that the purity was 97.25-98.23%.

### Preparation of Cabozantinib

As a Comparative Example, cabozantinib, a known medicament for renal cell carcinoma and hepatocellular carcinoma and also known to be effective against colorectal cancer, was used. Cabozantinib (Selleckchem; No. S1119) was dissolved in DMSO at 100 mg/mL, aliquoted, frozen, and stored. This was Cabozantinib DMSO solution. On the day of administration, the Cabozantinib DMSO solution was thawed and water for injection was added to reach a dosage concentration of 30 mg/mL. This was Cabozantinib solution.

### Preparation of Experimental Animals

Five-week-old female BALB/cSlc-nu/nu mice were purchased from Japan SLC (Shizuoka, Japan) and used for the preparation of experimental animals. After acclimatization, human colon cancer-derived HT-29 cells were suspended at 5 × 10⁷ cells/mL in a 1:2 mixture of PBS (pH 7.4, 1×; Thermo Fisher Scientific, 10010-023) and VitroGel (The Well Bioscience, VHM01). A 0.1-mL portion of the suspension was subcutaneously inoculated into the right flank of (6-week-old) nude mice by using a disposable syringe with a needle. The number of animals evaluated was 6 per group, for a total of 9 groups.

Animals evaluated at the time of use weighed 16.28 g to 20.05 g and had tumor sizes of 88 to 229 mm³.

### Efficacy Evaluation

Saline (Saline) and each ¹⁷⁷Lu-labeled peptide were administered intravenously at 30 MBq/head or 60 MBq/head in HT-29 tumor-bearing mice on Days 0, 11, and 18. In addition, the cabozantinib administration group was orally administered daily from Day 0 to Day 27 at a dose calculated to be 30 mg/kg based on body weight data on the day of administration. Body weight and tumor volume were measured twice a week until Day 49, and all surviving animals on the last day of measurement were euthanized.

### Tumor Size Results

The time course of tumor size for each group is shown in Fig. 13. Conjugate No. 64-¹⁷⁷Lu (30 MBq/head, 3 doses), Conjugate No. 62-¹⁷⁷Lu (30 MBq/head, 3 doses), and Conjugate No. 65-¹⁷⁷ Lu (60 MBq/head, a single dose) exhibited excellent anti-tumor effects.

### Body Weight Results

The time course of body weight for each group is shown in Fig. 14. Regarding Conjugate No. 64-¹⁷⁷Lu (30 MBq/head, 3 doses), Conjugate No. 62-¹⁷⁷Lu (30 MBq/head, 3 doses), and Conjugate No. 65-¹⁷⁷ Lu (60 MBq/head, a single dose), which exhibited excellent anti-tumor effects, no significant weight loss was observed.

### Example 7: In Vivo Distribution of ⁶⁴Cu- or ¹⁷⁷Lu-Labeled Conjugate (in VMRC-RCW Xenograft Model)

### Peptide ⁶⁴Cu- or ¹⁷⁷Lu-Labeling

Each peptide was labeled by heating ⁶⁴Cu-labeling reaction solution (Table 12) or ¹⁷⁷Lu-labeling reaction solution (Table 13) containing Conjugate No. 62 at 95°C for 10 min.

Composition of labeling reaction solution (Conjugate No.62-⁶⁴Cu)

**[Table 12]**

| Composition of labeling reaction solution (Conjugate No. 62-⁶⁴Cu) | |
|---|---|
| Component | Amount (in 50 µL) |
| Conjugate No.62 | 1.40nmol |
| DMSO | 1.52 µL |
| Copper chloride (⁶⁴Cu) | 30.6-31.6MBq (test at 12:00 on the day of administration) |
| Sodium acetate hydrate | 18.52 µmol |
| Acetic acid | 12.34 µmol |
| Ascorbic acid | 0.28mg |
| Gentisic acid | 64 µg |

Composition of labeling reaction solution (Conjugate No.62-¹⁷⁷Lu)

**[Table 13]**

| Composition of labeling reaction solution (Conjugate No. 62-¹⁷⁷Lu) | |
|---|---|
| Component | Amount (in 50 µL) |
| Conjugate No.62 | 2.79nmol |
| DMSO | 1.52 µL |
| Lutetium chloride (¹⁷⁷Lu) | 39.4 MBq (test at 12:00 on the day of administration) |
| Sodium acetate hydrate | 20.27 µmol |
| Acetic acid | 13.51 µmol |
| Ascorbic acid | 0.28mg |
| Gentisic acid | 64 µg |

### Radio HPLC

Conjugate No.62-⁶⁴Cu or Conjugate No.62-¹⁷⁷Lu synthesized as described above was mixed with acetonitrile, and the radiochemical purity was measured before and after administration while using TSK gel (registered trademark) ODS-80Ts QA, 4.6 mm ID × 250 mm, 5 µm (Tosoh Corporation). The results show that the purity was 100%.

### Radio TLC

TLC Silica Gel 60 RP-18 F₂₅₄s; Merck, 115389 spotted with each test substance (Conjugate No. 62-⁶⁴Cu or Conjugate No. 62-¹⁷⁷Lu) was developed with a mixed solution of 2 mol/L ammonium acetate solution/acetone (1:1). Thereafter, radioactivity was measured using a radiochromatograph (measurement time: 10 min). The results show that the purity was 98.26-99.27%.

### Preparation of Experimental Animals (VMRC-RCW Tumor-Bearing Mice)

Five-week-old female BALB/c-nu mice were purchased from Jackson Laboratory Japan (Kanagawa, Japan) and used for the preparation of experimental animals. After acclimatization, human renal cell carcinoma-derived VMRC-RCW cells were suspended at 5 × 10⁷ cells/mL in EMEM (FUJIFILM Wako Pure Chemical Corporation, Osaka, Japan). A 0.1-mL portion of the suspension was subcutaneously inoculated into the right flank of (6-week-old) nude mice by using a disposable syringe with a needle.

Animals evaluated at the time of use weighed 18.2 g to 21 g and had tumor sizes of 80 to 386 mm³.

### In Vivo Distribution

Each test substance was administered as a single intravenous dose to VMRC-RCW tumor-bearing mice. Autopsies were performed at 4, 24, and 48 hours after administration, and the radioactivity concentration (%ID/g) of each tissue was measured.

### Results

Fig. 15 shows the results of the *in vivo* distribution of Conjugate No.62-⁶⁴Cu and Fig. 16 shows the results of the *in vivo* distribution of Conjugate No.62-¹⁷⁷Lu.

### PET/CT Imaging

PET/CT images were collected under isoflurane gas anesthesia at 1, 4, 23.5, and 47.5 hours after administration of Conjugate No.62-⁶⁴Cu, and tumors were found to be clearly detected. Fig. 17 shows the images at each time point. Note that the columns show the same individuals, e.g., the left column shows the results of the same individual at 1 hour, 4 hours, 23.5 hours, and 47.5 hours after the administration.

### Example 8: Efficacy Study on ¹⁷⁷Lu-Labeled Peptide (in VMRC-RCW Xenograft Model)

### Peptide ¹⁷⁷Lu-Labeling

A ¹⁷⁷Lu-labeling reaction solution containing Conjugate No. 62 was prepared for administration at 30 MBq/head or 60 MBq/head (Table 14), and the labeling reaction was carried out by heating at 95°C for 10 min.

Composition of labeling reaction solution for Conjugate No.62-¹⁷⁷Lu

**[Table 14]**

| Composition of labeling reaction solution for Conjugate No. 62-¹⁷⁷Lu | | |
|---|---|---|
| Name | 30MBq/head | 60MBq/head |
| | Conjugate No.62-¹⁷⁷Lu | Conjugate No.62-¹⁷⁷Lu |
| Conjugate No.62 | 1.4nmol/50 µL | 2.8nmol/50 µL |
| DMSO | 1.52 µL/50 µL | 1.52 µL/50 µL |
| Lutetium chloride (hereinafter, ¹⁷⁷Lu) (Target value) | 210MBq/50 µL (test at 15:00 on labeling day) | 420MBq/50 µL (test at 15:00 on labeling day) |
| Sodium acetate hydrate | 0.37mol/L | 0.33-0.34mol/L |
| Acetic acid | 0.25mol/L | 0.22mol/L |
| Ascorbic acid | 5.6mg/mL | 5.6mg/mL |
| Gentisic acid | 1.28mg/mL | 1.28mg/mL |

### Radio HPLC

Conjugate No.62-¹⁷⁷Lu synthesized as described above was mixed with acetonitrile, and the radiochemical purity was measured before and after administration while using TSK gel (registered trademark) ODS-80Ts QA, 4.6 mm ID × 250 mm, 5 µm (Tosoh Corporation). The results show that the purity was 99.6-100%.

### Radio TLC

TLC Silica Gel 60 RP-18 F₂₅₄s; Merck, 115389 spotted with Conjugate No.62-¹⁷⁷Lu was developed with a mixed solution of 2 mol/L ammonium acetate solution/acetone (1:1). Thereafter, radioactivity was measured using a radiochromatograph (energy range: 13-250 keV; collimator: 1.0 cm; V-shaped BGO crystal; measurement time: 10 min). The results show that the purity was 98.1-98.6%.

### Preparation of Cabozantinib

As a Comparative Example, Cabozantinib (Selleckchem; No. S1119) was dissolved in DMSO at 100 mg/mL, aliquoted, frozen, and stored. This was Cabozantinib DMSO solution. On the day of administration, the Cabozantinib DMSO solution was thawed and water for injection was added to reach a dosage concentration of 12.3 mg/mL. This was Cabozantinib solution.

### Preparation of Experimental Animals (VMRC-RCW Tumor-Bearing Mice)

Five-week-old female BALB/c-nu mice were purchased from Jackson Laboratory Japan (Kanagawa, Japan) and used for the preparation of experimental animals. After acclimatization, human renal cell carcinoma-derived VMRC-RCW cells were suspended at 5 × 10⁷ cells/mL in EMEM (Eagle Minimum Essential Medium, FUJIFILM Wako Pure Chemical Corporation, Osaka, Japan). A 0.1-mL portion of the suspension was subcutaneously inoculated into the right flank of (6-week-old) nude mice by using a disposable syringe with a needle.

Animals evaluated at the time of use weighed 19.3 g to 21.9 g and had tumor sizes of 216 to 445 mm³.

### Efficacy Evaluation

A single dose (80 µL/head) of saline (Saline) and Conjugate No. 62-¹⁷⁷Lu produced as described above was administered via tail vein to mice (8 weeks old at Day 0) in each group as shown in Table 15 below.

### Relationship between Administered Solution and Day of Administration for Each Group

**[Table 15]**

| Relationship between the administered solution and the day of administration for each group | | | | | | |
|---|---|---|---|---|---|---|
| Group | Administered solution | Conjugate No.62-¹⁷⁷Lu | Day of administration | | | |
| | | | Day0 | Day7 | Day14 | Day21 |
| Group 1 | Saline | | ○ | ○ | ○ | - |
| Group 3 | 30 MBq/head Conjugate No.62-¹⁷⁷Lu | 0.2nmol/head | ○ | - | - | - |
| Group 4 | 30 MBq/head Conjugate No.62-¹⁷⁷Lu | 0.2nmol/head | ○ | - | ○ | - |
| Group 5 | 30 MBq/head Conjugate No.62-¹⁷⁷Lu | 0.2nmol/head | ○ | ○ | ○ | - |
| Group 6 | 30 MBq/head Conjugate No.62-¹⁷⁷Lu | 0.2nmol/head | ○ | ○ | ○ | ○ |
| Group 7 | 60 MBq/head Conjugate No.62-¹⁷⁷ Lu | 0.4nmol/head | ○ | - | - | - |
| Group 8 | 60 MBq/head Conjugate No.62-¹⁷⁷Lu | 0.4nmol/head | ○ | - | ○ | - |

| | | | | | | |
|---|---|---|---|---|---|---|
| **Administered** on the day indicated by ○. Not administered on the day indicated by -. | | | | | | |

The cabozantinib administration group was orally administered daily from Day 0 to Day 27 at a dose calculated to be 12.3 mg/kg based on body weight data on the day of administration.

### Tumor Size Results

The time course of tumor size for each group is shown in Fig. 18. Conjugate No. 62-¹⁷⁷Lu exhibited excellent anti-tumor effects.

### Body Weight Results

The mean percent change in body weight for each group is shown in Fig. 19. No significant weight loss was observed in each group treated with Conjugate No. 62-¹⁷⁷Lu.

### Example 9: Efficacy Study on and In Vivo Distribution of ²²⁵Ac-Labeled Peptide

### Peptide ²²⁵ Ac-Labeling

The labeling reaction solution (Table 16) containing Conjugate No. 62 was heated at 95°C for 20 min for labeling to prepare the ²²⁵Ac-labeled compound.

Composition of labeling reaction solution (50 µL)

**[Table 16]**

| Composition of labeling reaction solution (50 µL) | |
|---|---|
| Peptide | 1.5 nmol |
| DMSO | 3.22 µL |
| Actinium chloride (²²⁵Ac) | 227-242 MBq (test at 10:00 on the day of administration) |
| Sodium acetate hydrate | 7.89 µmol |
| Acetic acid | 0.0867 µmol |
| Ascorbic acid | 132 µg |
| Tween (R) 20 | 0.02 mg |

### Radio HPLC

The test substance was mixed with acetonitrile, and the radiochemical purity was measured before administration while using TSK gel (registered trademark) ODS-80Ts QA, 4.6 mm ID × 150 mm, 5 µm (Tosoh Corporation). The results show that the purity was 97.9%.

### Radio TLC

TLC Silica Gel 60 RP-18 F₂₅₄s; Merck, 115389 spotted with the test substance was developed with a mixed solution of 2 mol/L ammonium acetate solution/acetone (1:1). Thereafter, radioactivity was measured using a radiochromatograph (energy range: 180-500 keV; collimator: 1.0 cm; V-shaped BGO crystal; measurement time: 10 min). The results show that the purity was 97.7%.

### Preparation of Experimental Animals (HT-29 Tumor-bearing Mice)

Six-week-old female BALB/cSlc-nu/nu mice were purchased from Japan SLC (Shizuoka, Japan) and used for the preparation of experimental animals. After acclimatization, human colon cancer-derived HT-29 cells were suspended at 5 × 10⁷ cells/mL in a 1:2 mixture of PBS (pH 7.4, 1×; Thermo Fisher Scientific, 10010-023) and VitroGel (The Well Bioscience, VHM01). A 0.1-mL portion of the suspension was subcutaneously inoculated into the right flank of (7-week-old) nude mice by using a disposable syringe with a needle.

The number of animals evaluated was 3 per group, for a total of 3 groups. Animals evaluated at the time of use weighed 17.8 g to 19.63 g and had tumor sizes of 151 to 275 mm³.

### In Vivo Distribution

The ²²⁵Ac-labeled compound was administered as a single intravenous dose to HT-29 tumor-bearing (9-week-old) mice. Autopsies were performed at 4, 24, and 48 hours after administration, and the radioactivity concentration (%ID/g) of each tissue was measured.

### Results

The results of the *in vivo* distribution of the ²²⁵Ac-labeled compound are shown in Fig. 20.

### Example 10: Efficacy Study on ²²⁵ Ac-Labeled Peptide

### Peptide ²²⁵Ac-Labeling

The labeling reaction solution (Table 17) containing Conjugate No. 62 was heated at 95°C for 20 min for labeling to prepare the test substance.

Composition of test substance

**[Table 17]**

| Composition of test substance | |
|---|---|
| Component | Volume |
| Peptide solution (10mg/mL in DMSO) | 19.7 µL |
| ²²⁵Ac dissolving solution (in 0.04 mol/L HCl) | 20 µL (14580kBq) |
| Buffer | 210.3 µL |
| Composition | |
| 0.2 mol/L sodium acetate buffer (pH 6.5) | |
| 3.3 mg/mL Ascorbic acid | |
| 0.05% Tween (R) 20 | |

### Radio HPLC

The test substance was measured for radiochemical purity before administration while using a Jupiter (registered trademark) C18 5 µm 4.6 × 150 mm (Phenomenex). The results show that the purity was 97.25%.

### Radio TLC

The test substance was spotted on TLC-SG Paper (Agilent, SGI0001), which was developed in a solvent containing 25 mM EDTA and 0.1 mol/L ammonium acetate, and the radioactivity was measured by Bioscan AR-2000 (Eckert & Ziegler). The results show that the purity was 99% or higher.

### Preparation of Experimental Animals (HT-29 Tumor-bearing Mice)

Five-week-old female BALB/cSlc nude mice were purchased from Charles River Laboratories (Wilmington, USA) and used for the preparation of experimental animals. After acclimatization, human colon cancer-derived HT-29 cells were suspended at 5 × 10⁷ cells/mL in a 1:2 mixture of PBS (pH 7.4, 1×; Thermo Fisher Scientific, 10010-023) and VitroGel (The Well Bioscience, VHM01). A 0.1-mL portion of the suspension was subcutaneously inoculated into the right flank of (7-week-old) nude mice by using a disposable syringe with a needle.

The number of animals evaluated was 10 per group, for a total of 6 groups. On the day before administration of the ²²⁵Ac-labeled peptide, the evaluated animals weighed 16.95 g to 17.83 g and had tumor sizes of 189.08 to 201.72 mm³.

### Efficacy Evaluation

PBS and ²²⁵Ac-labeled peptide were administered intravenously to HT-29 tumor-bearing mice 10 days after HT-29 cell implantation at 30 kBq/head, 60 MBq/head, 90 MBq/head, 120 MBq/head, or 150 MBq/head. Body weight and tumor volume were measured twice a week until Day 70, and all surviving animals on the last day of measurement were euthanized.

### Tumor Size Results

The time course of tumor size for each group is shown in Fig. 21. ²²⁵Ac-labeled Conjugate No. 62 exhibited excellent anti-tumor effects depending on radioactivity.

### Body Weight Results

The time course of body weight for each group is shown in Fig. 22. No significant weight loss was observed by administration of ²²⁵Ac-labeled Conjugate No. 62.

### Example 11: Synthesis of Cyclic Peptides and Synthesis of Conjugates

### Example 11-1: Synthesis of Conjugate No. 74

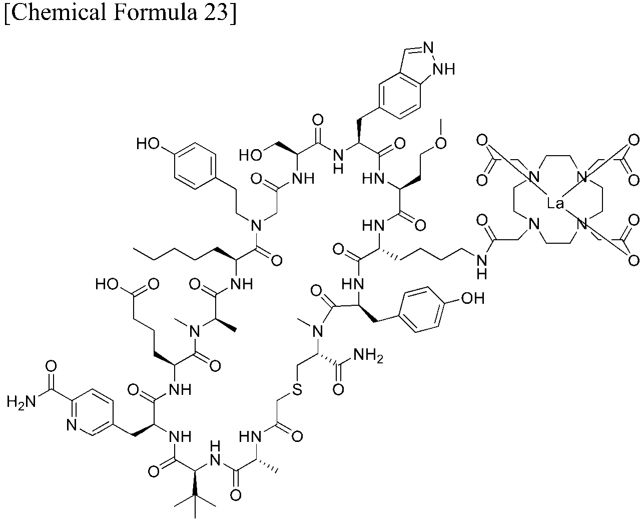

The peptide conjugate Conjugate No. 62 (10 mg) obtained in Example 1-5 was dissolved in 100 mM sodium acetate aqueous solution (0.465 mL), and lanthanum(III) chloride heptahydrate (25.9 mg, CAS: 10025-84-0) was then dissolved. The reaction mixture was stirred at 45°C for 60 min.

The reaction mixture obtained above was purified under the following conditions (column: Waters XSelect (registered trademark) C18 5 µm, 50 × 250 mm; mobile phase: A = 0.1% TFA (in water) and B = 0.1% TFA (in MeCN); temperature: 50°C; gradient (%B): 0-0% over 5 min, 0-4.2% over 2 min, 4.2-24.6% over 3 min, 24.6-29.7% over 15.5 min, 29.7-60% over 1.5 min, and 60-90% over 4 min; flow rate: 18-18 mL/min over 5.0 min, 18-118 mL/min over 2 min, then 118 mL/min). The target product-containing fractions were collected and lyophilized to obtain the peptide of interest.

The purity of the target product was calculated from the area ratio of LC/MS (UV wavelength: 225 nm) chromatogram under the analytical conditions. The purity of the target product was 96.5%.
Analytical condition A: retention time = 3.54 min
ESI-MS(+): observed m/z = 1143.45 (M+2H)²⁺

### Example 11-2: Synthesis of Conjugate No. 75

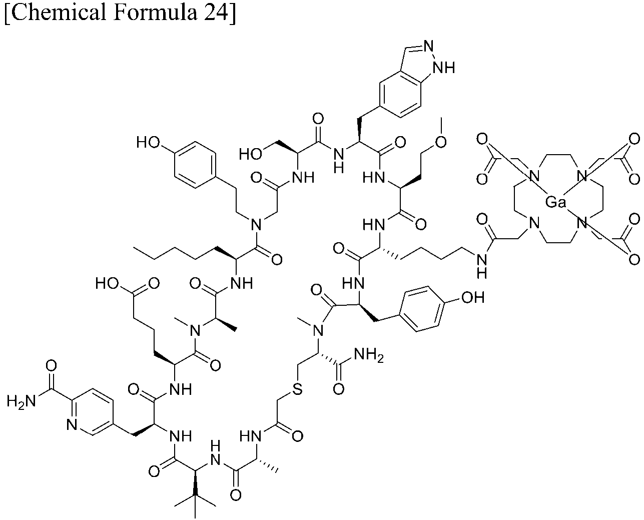

The peptide SEQ ID No. 46 (10 mg) obtained in Example 1-2, DIEA (15.7 µL), and DOTA-NHS ester hexafluorophosphate trifluoroacetate (5.04 mg) were dissolved In DMF (0.50 mL) at 0°C. The reaction mixture was stirred at room temperature for 60 min, and gallium(III) chloride (3.52 mg, CAS:13450-90-3) and 50 mM sodium acetate aqueous solution (0.40 mL) were then added to the reaction mixture. The reaction mixture was stirred at 90°C for 1 hour.

The resulting reaction mixture was purified using the following conditions (column: Waters XBridge (registered trademark) C18 5 µm 30 × 150 mm; mobile phase: A = 0.1% TFA (in water), B = 0.1% TFA (in MeCN); temperature: 50°C; gradient (%B): 5.0-27% over 3 min, 27-32% over 8 min, 32-60% over 1 min; flow rate: 45 ml/min). The target product-containing fractions were collected and lyophilized to obtain the peptide of interest.

The purity of the target product was calculated from the area ratio of LC/MS (UV wavelength: 225 nm) chromatogram under the analytical conditions. The purity of the target product was 99.5%.
Analytical condition A: retention time = 3.24 min
ESI-MS(+): observed m/z = 1108.94 (M+2H)²⁺

### Example 11-3: Synthesis of Conjugate No. 78

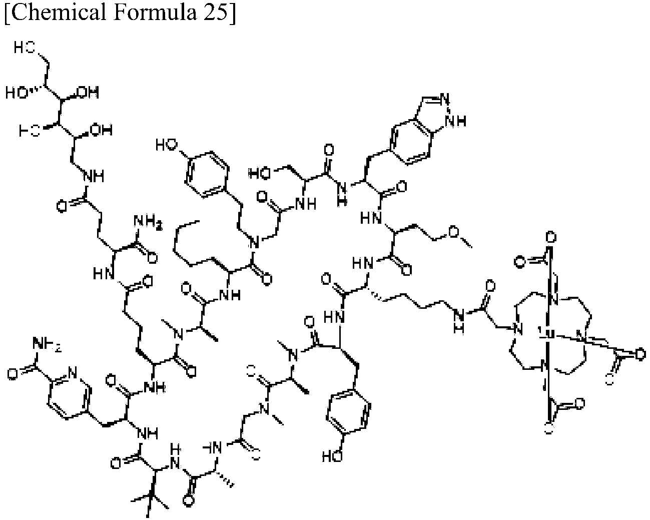

Substantially the same method as for Conjugate No. 67 was used to synthesize a cyclic peptide (sequence: da-Tbg-3Py6CON-Hgl-Meda-Ahp-pHPeG-S-5Inda-HseMe-dk(Alloc)-Y-MeA-MeG) (SEQ ID No. 103). Fmoc-dk(Alloc)-OH was used instead of Fmoc-dk(Boc)-OH.

Purification was performed using the following conditions (column: Waters XBridge (registered trademark) C18 5 µm 50 × 150 mm; mobile phase: A = 0.1% TFA (in water), B = 0.1% TFA (in MeCN); temperature: 40°C; gradient (%B): 8-8% over 2 min, 8-33% over 1 min, 33-38% over 8 min, 38-60% over 1 min; flow rate: 20-20 mL/min over 1 min, 20-120 mL/min over 1 min, then 120 ml/min). The target product-containing fractions were collected and lyophilized.

The cyclic peptide (38 mg) obtained by the above method was dissolved in DMF (0.65 mL), a DMF solution (58.7 µL) containing 2,4,6-trimethylpyridine (25.8 µL, CAS: 108-75-8) and 0.5 M HATU was added, and the mixture was stirred at 25°C for 5 min. Fmoc-Qglucamine-NH₂ (50 mg) was dissolved in DMSO (0.94 mL); TEA (0.20 mL) was added and the mixture was stirred at 60°C for 1 hour; the mixture was then concentrated under reduced pressure by using Biotage's V-10; trituration with methyl *tert-butyl* ether was performed; a DMSO solution (0.50 mL) containing H-Qglucamine-NH₂ (7.9 mg) synthesized by the above method was added to the reaction solution containing the above cyclic peptide, and the mixture was stirred for 2 hours. Water (50 µL) was added, and the mixture was then concentrated under reduced pressure by using Biotage's V-10. The resulting mixture was dissolved in DMF (0.67 mL), phenylsilane (25 µL, CAS: 694-53-1) and Pd(PPh₃)₄ (4.6 mg, CAS: 14221-01-3) were added, and the mixture was stirred at room temperature for 50 min. To the resulting mixture were added DIPEA (68 µL) and DOTA-NHS TFA salt (36 mg, CAS: 2832911-57-4). The mixture was then stirred at 25°C overnight. After water (100 µL) was added, half of the resulting mixture was diluted with DMSO (1 mL) and water (1 mL). The mixture was then stirred with 0.5 M lutetium(III) chloride-containing ammonium acetate aqueous solution (2.4 mL, pH 5.0) at 90°C for 1 hour. The resulting suspension was concentrated under reduced pressure by using Biotage's V-10 and dissolved in DMSO/water (9/1, 4 mL).

The resulting aqueous solution was purified using the following conditions (column: Waters XSelect (registered trademark) C18 5 µm 50 × 250 mm; mobile phase: A = 0.1% TFA (in water), B = 0.1% TFA (in MeCN); temperature: 50°C; gradient (%B): 0-0% over 5 min, 0-4.2% over 2 min, 4.2-19.5% over 3 min, 19.5-24.6% over 15.5 min, 24.6-60% over 1.5 min; flow rate: 18-18 mL/min over 5 min, 18-118 mL/min over 2 min, then 118 ml/min). The target product-containing fractions were collected and lyophilized.

The purity of the target product was calculated from the area ratio of LC/MS (UV wavelength: 225 nm) chromatogram under the analytical conditions. The purity of the target product was 97.7%.
Analytical condition B: retention time = 5.72 min
ESI-MS(+): observed m/z = 865.85 (M+3H)³⁺

### Example 11-4: Synthesis of Conjugate No. 79

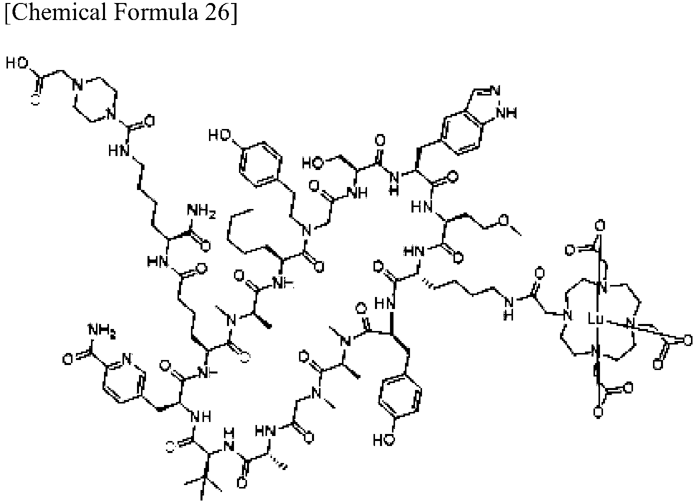

Substantially the same method as for Conjugate No. 67 was used to synthesize a cyclic peptide (sequence: da-Tbg-3Py6CON-Hgl-Meda-Ahp-pHPeG-S-SInda-HseMe-dk(Alloc)-Y-MeA-MeG) (SEQ ID No. 103). Fmoc-dk(Alloc)-OH was used instead of Fmoc-dk(Boc)-OH.

Purification was performed using the following conditions (column: Waters XBridge (registered trademark) C18 5 µm 50 × 150 mm; mobile phase: A = 0.1% TFA (in water), B = 0.1% TFA (in MeCN); temperature: 40°C; gradient (%B): 8-8% over 2 min, 8-33% over 1 min, 33-38% over 8 min, 38-60% over 1 min; flow rate: 20-20 mL/min over 1 min, 20-120 mL/min over 1 min, then 120 ml/min). The target product-containing fractions were collected and lyophilized.

The cyclic peptide (38 mg) obtained by the above method was dissolved in DMF (0.65 mL), a DMF solution (58.7 µL) containing 2,4,6-trimethylpyridine (25.8 µL, CAS: 108-75-8) and 0.5 M HATU was added at 25°C, and the mixture was stirred for 5 min. A DMSO solution (0.50 mL) containing H-KCOpipzaa(Mpe)-NH₂ (10.2 mg) was added to the above cyclic peptide-containing reaction solution, and the mixture was then stirred for 1.5 hours. Water (50 µL) was added, and the mixture was then concentrated under reduced pressure by using Biotage's V-10. The resulting mixture was dissolved in TFA/TIS/H₂O (95/2.5/2.5, 0.5 mL) and stirred for 30 min. The resulting mixture was added to diisopropyl ether/heptane (1/1, 5 mL), and the resulting solid was centrifuged. The supernatant was removed, and the rest was washed with diisopropyl ether/heptane (1/1, 5 mL). The resulting material was dried under reduced pressure and dissolved in DMF (0.67 mL). Phenylsilane (25 µL, CAS: 694-53-1) and Pd(PPh₃)₄ (4.6 mg, CAS: 14221-01-3) were added, and the mixture was stirred at room temperature for 50 min. To the resulting mixture were added DIPEA (68 µL) and DOTA-NHS TFA salt (36 mg, CAS: 2832911-57-4). The mixture was then stirred at 25°C overnight. After water (100 µL) was added, half of the resulting mixture was diluted with DMSO (1 mL) and water (1 mL). The mixture was then stirred with 0.5 M lutetium(III) chloride-containing ammonium acetate aqueous solution (2.4 mL, pH 5.0) at 90°C for 1 hour. The resulting suspension was concentrated under reduced pressure by using Biotage's V-10 and dissolved in DMSO/water (9/1, 4 mL).

The resulting aqueous solution was purified using the following conditions (column: Waters XSelect (registered trademark) C18 5 µm 50 × 250 mm; mobile phase: A = 0.1% TFA (in water), B = 0.1% TFA (in MeCN); temperature: 50°C; gradient (%B): 0-0% over 5 min, 0-4.2% over 2 min, 4.2-19.5% over 3 min, 19.5-24.6% over 15.5 min, 24.6-60% over 1.5 min; flow rate: 18-18 mL/min over 5 min, 18-118 mL/min over 2 min, then 118 ml/min). The target product-containing fractions were collected and lyophilized.

The purity of the target product was calculated from the area ratio of LC/MS (UV wavelength: 225 nm) chromatogram under the analytical conditions. The purity of the target product was 96.6%.
Analytical condition B: retention time = 5.70 min
ESI-MS(+): observed m/z = 867.91 (M+3H)³⁺

### Example 11-5: Synthesis of Conjugate

According to the general method described in Example 1 and Examples 11-1/2, each target peptide and a conjugate containing said peptide and a payload as listed in the following table were synthesized.

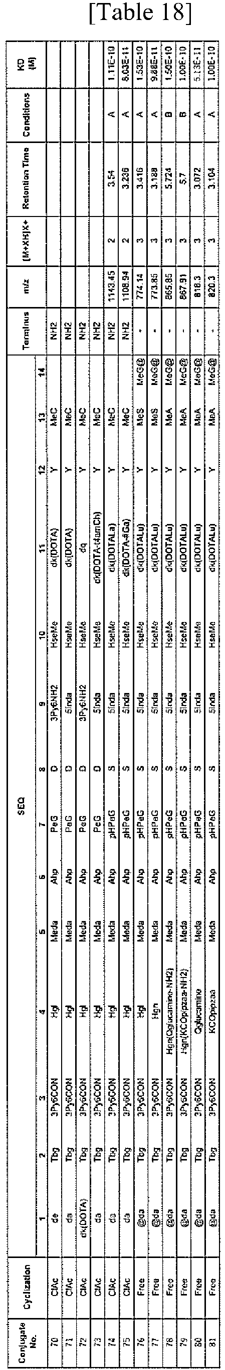

Table 18 lists the target conjugates, analytical conditions and retention times, and ESI-MS(+) observed values.

In Table 18, the "terminus" indicates the C-terminal functional group (in the table, "-OH" indicates COOH and "-NH2" indicates CO(NH₂)), and the blank case indicates that no functional group is present at the C-terminus. With respect to "Cyclization" in the tables, entries with the description "ClAc" represent that cyclization was achieved by linking the amino acid at position 1 into which a chloroacetyl group was introduced with C or MeC present at the C-terminus, whereas the description "Free" indicates that the N-terminal amino group of the amino acid at position 1 is linked to the C-terminal carboxy group of the amino acid at position 13 or 14. In addition, for example, in the conjugate of Conjugate No. 75, the amino acid residues of C at X1 and X13 form a cyclic structure and the side chain of dk at X11 is attached to the payload, namely Ga-coordinated DOTA. Furthermore, in Conjugate No. 76 listed in Table 8, the amino acid residues of MeG at X1 and X14 likewise form a cyclic structure and the side chain of dk at X11 is attached to the payload, namely Lu-coordinated DOTA.

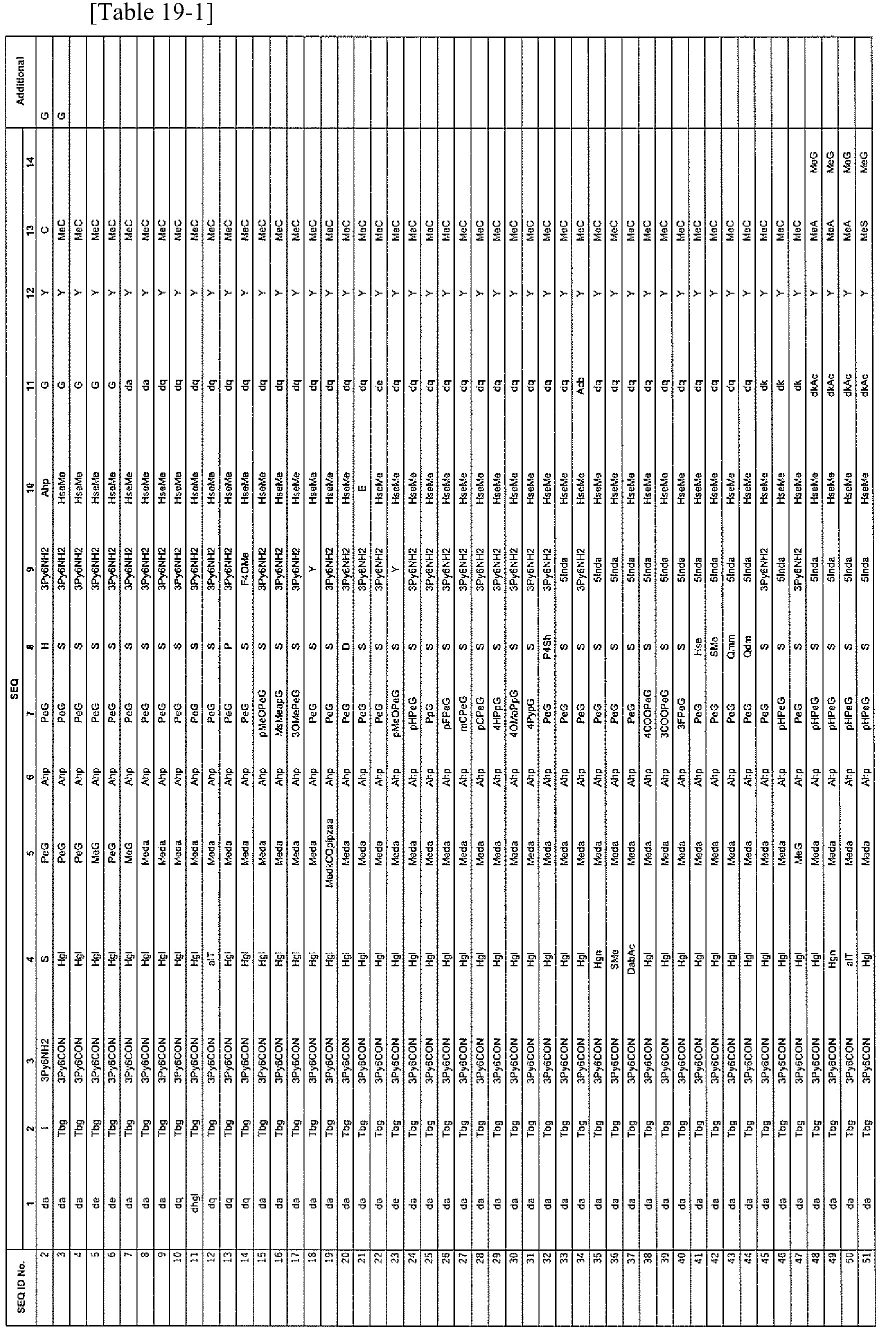

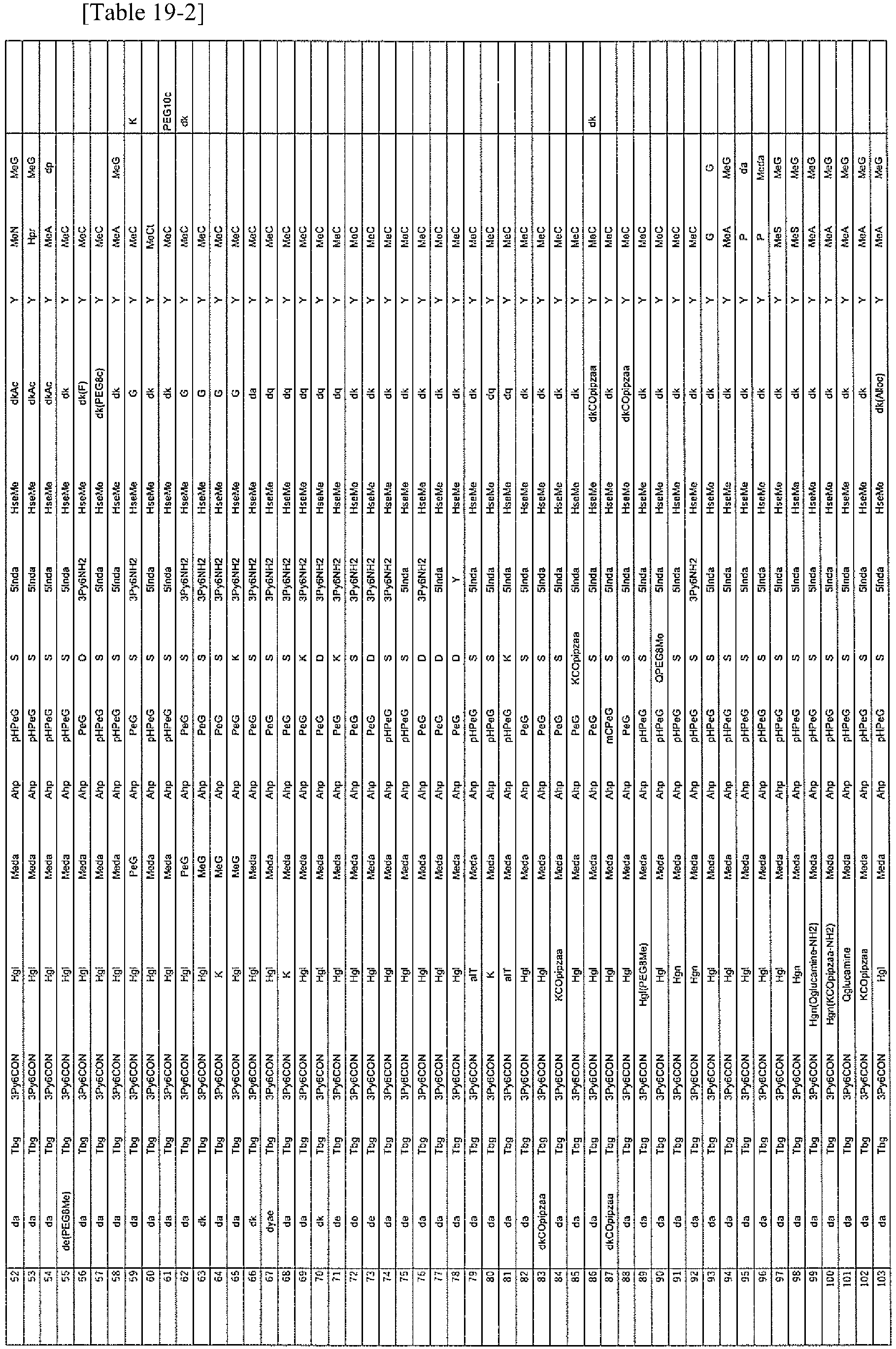

**[Table 20-1]**

| **Conjugate No.** | **SEQ ID No.** |
|---|---|
| 1 | 2 |
| 2 | 4(59) |
| 3 | 4(62) |
| 4 | 4(59) |
| 5 | 4(62) |
| 6 | 63 |
| 7 | 64 |
| 8 | 65 |
| 9 | 47 |
| 10 | 66 |
| 11 | 67 |
| 12 | 68 |
| 13 | 69 |
| 14 | 45 |
| 15 | 70 |
| 16 | 71 |
| 17 | 72 |
| 18 | 73 |
| 19 | 70 |
| 20 | 72 |
| 21 | 4(59) |
| 22 | 4(59) |
| 23 | 74 |
| 24 | 74 |
| 25 | 74 |
| 26 | 46 |
| 27 | 75 |
| 28 | 76 |
| 29 | 77 |
| 30 | 78 |
| 31 | 77 |
| 32 | 76 |
| 33 | 76 |
| 34 | 76 |
| 35 | 76(56) |
| 36 | 76 |
| 37 | 45 |
| 38 | 46(57) |
| 39 | 79 |
| 40 | 80 |

**[Table 20-2]**

| | |
|---|---|
| 41 | 81 |
| 42 | 80 |
| 43 | 82 |
| 44 | 82 |
| 45 | 60 |
| 46 | 83 |
| 47 | 84 |
| 48 | 85 |
| 49 | 88(86) |
| 50 | 87 |
| 51 | 55 |
| 52 | 89 |
| 53 | 90 |
| 54 | 46 |
| 55 | 45 |
| 56 | 46 |
| 57 | 45 |
| 58 | 46 |
| 59 | 91 |
| 60 | 91 |
| 61 | 92 |
| 62 | 46 |
| 63 | 46 |
| 64 | 45 |
| 65 | 47 |
| 66 | 93 |
| 67 | 58 |
| 68 | 95 |
| 69 | 96 |
| 70 | 73 |
| 71 | 77 |
| 72 | 70 |
| 73 | 77 |
| 74 | 46 |
| 75 | 46 |
| 76 | 97 |
| 77 | 98 |
| 78 | 99 |
| 79 | 100 |
| 80 | 101 |
| 81 | 102 |

Table 19 shows only the amino acid sequences of the peptides or peptide portions contained in the conjugates as synthesized in Examples 2 and 11-3. In addition, Table 20 shows a correspondence table between the conjugates synthesized and evaluated in this Example and the SEQ ID Nos. of the amino acid sequences contained in the cyclic structural portions of the corresponding conjugates. Note that Conjugate Nos. 2-5, 21, 22, 35, 38, or 49 correspond to one of 4(59), 4(62), 76(55), 46(57), or 88(86). This indicates, for example, that in the Conjugate No. 2, the amino acid sequence contained in the cyclic structural portion corresponds to the amino acid sequence described in SEQ ID No. 4, and that the peptide-linker complex, in which K (lysine), serving as a linker for attaching the payload DOTA, is attached to the C-terminus, corresponds to the amino acid sequence set forth in SEQ ID No. 59. This indicates, for example, that in the Conjugate No. 35, the amino acid sequence contained in the cyclic structural portion corresponds to the amino acid sequence set forth in SEQ ID No. 76, and that the peptide-linker complex, in which F (phenylalanine), serving as a linker for attaching the payload DOTALu, is attached to the side chain of dk at position 11 of the amino acid sequence contained in this cyclic structural portion, corresponds to the amino acid sequence set forth in SEQ ID No. 56. Likewise, in the Conjugate No. 38, the amino acid sequence contained in the cyclic structural portion corresponds to the amino acid sequence set forth in SEQ ID No. 46, and that the peptide-linker complex, in which PEG8c, serving as a linker for attaching the payload SulfoCy5, is attached to the side chain of dk at position 11 of the amino acid sequence contained in this cyclic structural portion, corresponds to the amino acid sequence set forth in SEQ ID No. 57. That is, the number shown in parentheses in Table 20 indicates the amino acid sequence SEQ ID No. when the linker or additional amino acid is attached.

For example, Conjugate No. 1 has the amino acid sequence set forth in SEQ ID No. 2 as a cyclic structural portion, and is a complex in which the payload, SulfoCy5, is attached via a linker (i.e., G, PEG10c, K) added to the C-terminus. Here, both the cyclic amino acid sequence alone, as shown in Table 7, and the conjugate with some payload, as shown in Table 8, have CA9 binding ability. Furthermore, multiple conjugates having the amino acid sequence set forth in SEQ ID No. 45 as a cyclic structural portion but differing only in the payload exist (Conjugate Nos. 14, 37, 55, 57, and 64). Even SEQ ID No. 45 without any payload possesses CA9 binding ability, and conjugates in which various chelators such as DOTA, structures having metal ions coordinated to such chelators, or low-molecular-weight substances such as SulfoCy5 are attached likewise possess CA9 binding ability. That is, when a certain conjugate possesses CA9 binding ability, it is apparent that a cyclic peptide having the amino acid sequence of the cyclic structural portion contained in the conjugate but lacking a payload, as well as conjugates differing only in the payload, also possess CA9 binding ability.

Thus, the peptides of the present invention have CA9 binding ability regardless of the presence or absence of the payload and the type of payload.

### Reference Example 3: Synthesis of Fmoc-Qglucamine-NH2

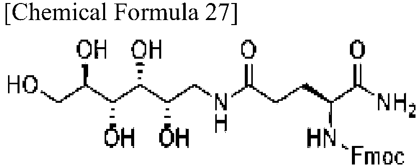

DIPEA (0.14 mL) and HATU (0.19 mg) were added to a DMF solution (3.3 mL) containing N2-(((9H-fluoren-9-yl)methoxy)carbonyl)-N5-((2S)-2-hydroxy-2-((4'R,5S)-2,2,2',2'-tetramethyl-[4,4'-bi(1,3-dioxolan)]-5-yl)ethyl)-L-glutamine (200 mg, CAS: 1227510-37-3) and ammonium chloride (34.9 mg, CAS: 12125-02-9), and the mixture was stirred at 0°C for 1 hour. Purification was performed by silica gel flash column chromatography (DCM/MeOH = 100/0 to 80/20), and the target product-containing fractions were collected. A portion of the resulting compound (111 mg) was dissolved in DCM (2.7 mL) and ethanol (1.8 mL) and stirred with TFA (1 mL) at 25°C for one week. The resulting suspension was diluted in methyl *tert*-butyl ether and centrifuged. After the supernatant was removed, trituration with methyl *tert*-butyl ether was performed to afford the title compound.
ESI-MS(+): observed m/z = 532.2 (M+H)⁺

### Reference Example 4: Synthesis of H-KCOpipzaa(Mpe)-NH2

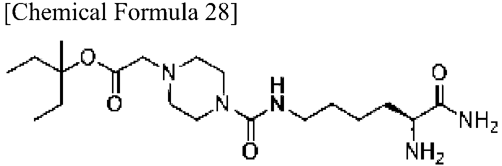

DIPEA (0.14 mL) and HATU (0.18 mg) were added to a DMF solution (3.2 mL) containing N2-(((9H-fluoren-9-yl)methoxy)carbonyl)-N6-(4-(2-((3-methylpentan-3-yl)oxy)-2-oxoethyl)piperazin-1-carbonyl)-L-lysine (200 mg, CAS: 2973753-38-5) and ammonium chloride (34.4 mg, CAS: 12125-02-9), and the mixture was stirred at 25°C for 2 hours. Purification was performed by silica gel flash column chromatography (DCM/MeOH = 100/0 to 90/10), and the target product-containing fractions were collected. A portion (100 mg) of the compound obtained was dissolved in DMSO (1.6 mL); TEA (0.34 mL) was added and the mixture was then stirred at 60°C for 1 hour. The mixture was concentrated under reduced pressure by using Biotage's V-10. Trituration with methyl *tert*-butyl ether was then performed to afford the title compound.
ESI-MS(+): observed m/z = 400.3 (M+H)⁺

### INDUSTRIAL APPLICABILITY

The peptides and conjugates of the present invention have CA9 binding activity and can be used as pharmaceutical compositions, diagnostic compositions, and research compositions for the prevention or treatment of CA9-associated symptoms, etc.

## Claims

1. A peptide comprising the following amino acid sequence:
da-Tbg-3Py6CON-Hgl-Meda-Ahp-PeG-S-3Py6NH2-HseMe-dk-Y (SEQ ID No. 1) or
an amino acid sequence having a substitution, addition, deletion, or insertion in 1 to 10 amino acid residues selected from the group consisting of amino acid residues at positions 1, 2, 3, 4, 5, 7, 8, 9, 10, and 11 in the above amino acid sequence.

2. The peptide according to claim 1, wherein
(1) MeC, C, or MeCt is attached, as an amino acid residue at position 13, to the amino acid sequence set forth in SEQ ID No. 1; or
(2) MeA, MeS, MeN, Hpr, G, or P is attached, as an amino acid residue at position 13, to the amino acid sequence of SEQ ID No. 1 and MeG, G, da, or Meda is further attached as an amino acid residue at position 14.

3. The peptide according to claim 1 or 2, comprising an amino acid sequence having a substitution, addition, deletion, or insertion in 1 to 7 amino acid residues selected from the group consisting of amino acid residues at positions 1, 4, 7, 8, 9, 10, and 11 in the amino acid sequence set forth in SEQ ID No. 1.

4. The peptide according to any one of claims 1 to 3, wherein the following one or two or more requirements are satisfied:
the amino acid residue at position 1 is da, dq, de, dhgl, or dk;
the amino acid residue at position 2 is I or Tbg;
the amino acid residue at position 3 is 3Py6NH2 or 3Py6CON;
the amino acid residue at position 4 is S, Hgl, Hgl(PEG8Me), alT, Hgn, SMe, KCOpipzaa, or DabAc;
the amino acid residue at position 5 is PeG, MeG, Meda, or MedkCOpipzaa;
the amino acid residue at position 7 is PeG, pMeOPeG, MsMeapG, 3OMePeG, pHPeG, PpG, pFPeG, mCPeG, pCPeG, 4HPpG, 4OMePpG, 4PypG, 4COOPeG, 3COOPeG, or 3FPeG;
the amino acid residue at position 8 is H, S, P, D, KCOpipzaa, Q(PEG8Me), P4Sh, Hse, SMe, Qmm, or Qdm;
the amino acid residue at position 9 is 3Py6NH2, 5Inda, Y or F4OMe;
the amino acid residue at position 10 is Ahp, HseMe, or E; and
the amino acid residue at position 11 is dk, G, da, dq, dkCOpipzaa, dk(t4amCh), de, Acb, or dkAc.

5. The peptide according to any one of claims 1 to 4, wherein the following one or two or more requirements are satisfied:
the amino acid residue at position 2 is Tbg;
the amino acid residue at position 3 is 3Py6NH2;
the amino acid residue at position 5 is Meda;
the amino acid residue at position 7 is PeG or pHPeg;
the amino acid residue at position 9 is 3Py6NH2 or 5Inda; and
the amino acid residue at position 10 is HseMe.

6. The peptide according to any one of claims 1 to 4, wherein
the amino acid residue at position 2 is Tbg;
the amino acid residue at position 3 is 3Py6NH2;
the amino acid residue at position 5 is Meda;
the amino acid residue at position 7 is PeG or pHPeg;
the amino acid residue at position 9 is 3Py6NH2 or 5Inda; and
the amino acid residue at position 10 is HseMe.

7. The peptide according to any one of claims 1 to 6, wherein the peptide comprises or consists of an amino acid sequence set forth in any one of SEQ ID Nos. 2-54.

8. The peptide according to any one of claims 1 to 7, further comprising an additional amino acid residue.

9. The peptide according to any one of claims 1 to 8, wherein the peptide is a cyclic peptide.

10. The peptide according to any one of claims 1 to 9, which has a cyclic structure in which a chloroacetylated amino acid residue at position 1 in the amino acid sequence set forth in SEQ ID No. 1 is bonded to a cysteine residue included in the peptide.

11. The peptide according to any one of claims 1 to 9, which has a cyclic structure in which an amino group of an amino acid residue at position 1 in the amino acid sequence set forth in SEQ ID No. 1 included in the peptide is bonded to a carboxy group of an amino acid residue at position 14.

12. The peptide according to any one of claims 1 to 11, wherein
(a) a linker and/or a payload can bind to a C-terminus of the peptide; or
(b) the amino acid residue at position 1, 4, 8, 11, or 13 in the amino acid sequence set forth in SEQ ID No. 1 is an amino acid residue to which a linker and/or a payload can bind.

13. A conjugate comprising the peptide according to any one of claims 1 to 12 and a payload, wherein
(i) the amino acid residue at position 13 in SEQ ID NO: 1 is attached, with or without a linker, to any payload; or
(ii) the amino acid residue at position 1, 4, 8, or 11 in SEQ ID No. 1 is attached to a payload as shown in the following formula (I): 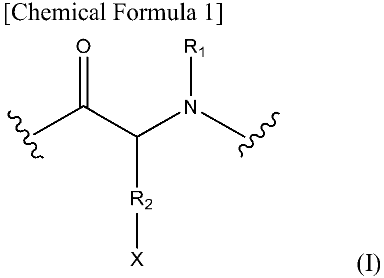
wherein in the formula (I),
R₁ is H or a C₁₋₃ alkyl group;
R₂ is C₁₋₆ alkyl-NH-, C₁₋₆ alkyl-C₆ aryl-O-C₁₋₃ alkyl-NH-, C₁₋₆ alkyl-NH(=O)-CH(C_{1- 6} alkylphenyl)-NH-, C₁₋₆ alkyl-NH(=O)-CH(C₁₋₆ alkyl)-NH-, or C₁₋₆ alkyl-NH(=O)-C_{3- 8} cycloalkyl-C₁₋₃ alkyl-NH-; and
X is any payload.

14. The conjugate according to claim 13, wherein R₂ is C₁₋₆ alkyl-NH-.

15. The conjugate according to claim 13 or 14, wherein X is a chelator.

16. The conjugate according to claim 15, wherein the chelator is attached to a radioactive substance.

17. The conjugate according to claim 15 or 16, wherein the chelator is DOTA, DOTAGA, or NODAGA.

18. A pharmaceutical composition comprising the peptide according to any one of claims 1 to 12.

19. A pharmaceutical composition for preventing or treating a CA9-associated disease, comprising the peptide according to any one of claims 1 to 12.

20. A pharmaceutical composition comprising the conjugate according to any one of claims 13 to 17.

21. A pharmaceutical composition for preventing or treating a CA9-associated disease, comprising the conjugate according to any one of claims 13 to 17.

22. A diagnostic or research composition comprising the peptide according to any one of claims 1 to 12.

23. A diagnostic or research composition for diagnosing a CA9-associated disease, comprising the peptide according to any one of claims 1 to 12.

24. A diagnostic or research composition comprising the conjugate according to any one of claims 13 to 17.

25. A diagnostic or research composition for diagnosing a CA9-associated disease, comprising the conjugate according to any one of claims 13 to 17.

26. An imaging agent comprising the conjugate according to any one of claims 13 to 17.

27. An imaging agent comprising the conjugate according to any one of claims 13 to 17, wherein the imaging agent is used for diagnosis of a tumor.

28. A radioligand imaging agent used for positron emission tomography, comprising the conjugate according to any one of claims 13 to 17.

29. A peptide testing method comprising testing a peptide in terms of at least one of:
a) solubility in a solvent;
b) CA9 binding activity;
c) cell and/or tissue toxicity; or
d) toxicity to an experimental animal, wherein
the peptide is the peptide according to any one of claims 1 to 12.

30. A conjugate testing method comprising testing a conjugate in terms of at least one of:
a) solubility in a solvent;
b) CA9 binding activity;
c) cell and/or tissue toxicity; or
d) toxicity to an experimental animal, wherein
the conjugate is the conjugate according to any one of claims 13 to 17.

31. The peptide according to any one of claims 1 to 3, wherein the following one or two or more requirements are satisfied:
the amino acid residue at position 1 is de(PEG8Me), dk, dyae, or dkCOpipzaa;
the amino acid residue at position 2 is I or Tbg;
the amino acid residue at position 3 is 3Py6NH2 or 3Py6CON;
the amino acid residue at position 4 is K, KCOpipzaa, Hgn(Qglucamine-NH2), Hgn(KCOpipzaa-NH2), or Qglucamine;
the amino acid residue at position 5 is PeG, MeG, Meda, or MedkCOpipzaa;
the amino acid residue at position 7 is PeG, pMeOPeG, MsMeapG, 3OMePeG, pHPeG, PpG, pFPeG, mCPeG, pCPeG, 4HPpG, 4OMePpG, 4PypG, 4COOPeG, 3COOPeG, or 3FPeG;
the amino acid residue at position 8 is K;
the amino acid residue at position 9 is 3Py6NH2, 5Inda, Y, or F4OMe;
the amino acid residue at position 10 is Ahp, HseMe, or E; and
the amino acid residue at position 11 is dk(F) or dk(PEG8c).

32. The peptide according to any one of claims 1 to 6, wherein the peptide comprises or consists of an amino acid sequence set forth in any one of SEQ ID Nos. 55-103.

33. A conjugate comprising the peptide according to claim 32 and a payload, wherein any payload is attached, with or without a linker, to the amino acid residue at position 1, 4, 8, 11, or 13.

34. A conjugate described in any one of Conjugate Nos. 1-81 listed in Table 8 or Table 18.
